# EUROPEAN PATENT SPECIFICATION

(11) **EP 1 939 203 B1**
(45) Date of publication and mention of the grant of the patent: **19.11.2014**
(21) Application number: 08006457.9
(22) Date of filing: 24.04.2001
(51) Int. Cl.: C07D 473/38, C07D 473/34, C07D 473/00, C07D 473/24, C07D 473/16, C07D 473/40, C07D 473/30, C07D 403/12, C07D 487/04, A61K 31/517, A61K 31/52, A61P 7/00, A61P 29/00, A61P 19/10

(54) **Inhibitors of human phosphatidyl-inositol 3-kinase delta isoform**
Hemmer der delta-Isoform der menschlichen Phosphatidyl-Inositol-3-Kinase
Inhibiteurs de delta-isoforme de phosphatidyl-inositol-3-kinase humaine

(30) Priority: 25.04.2000 US 199655 P; 05.10.2000 US 238057 P
(43) Date of publication of application: 02.07.2008
(62) Divisional of application: 01928855.4
(73) Proprietor: ICOS Corporation, Bothell, WA 98201 (US)
(72) Inventor: Sadhu, Chanchal, Bothell WA 98012 (US); Dick, Ken, Bothell WA 98021-8321 (US); Sowell, C. Gregory, Makilteo WA 98275 (US); Kesicki, Edward A., Bothell WA 98021 (US); Oliver, Amy, Bothell WA 98021 (US); Treiberg, Jennifer, Bothell WA 98012 (US)
(74) Representative: Goodfellow, Hugh Robin

(56) References cited:
- EP-A- 0 675 124
- WO-A-94/17090
- WO-A-97/41097
- WO-A-99/08501

## Description

### FIELD OF THE INVENTION

The present invention relates generally to phosphatidylinositol 3-kinase (PI3K) enzymes, and more particularly to selective inhibitors of PI3K activity.

### BACKGROUND OF THE INVENTION

Cell signaling via 3'-phosphorylated phosphoinositides has been implicated in a variety of cellular processes, e.g., malignant transformation, growth factor signaling, inflammation, and immunity (see Rameh et al., J. Biol Chem, 274:8347-8350 (1999) for a review). The enzyme responsible for generating these phosphorylated signaling products, phosphatidylinositol 3-kinase (PI 3-kinase; PI3K), was originally identified as an activity associated with viral oncoproteins and growth factor receptor tyrosine kinases that phosphorylates phosphatidylinositol (PI) and its phosphorylated derivatives at the 3'-hydroxyl of the inositol ring (Panayotou et al., Trends Cell Biol 2:358-60 (1992)).

The levels of phosphatidylinositol-3,4,5-triphosphate (PIP3), the primary product of PI 3-kinase activation, increase upon treatment of cells with a variety of agonists. PI 3-kinase activation, therefore, is believed to be involved in a range of cellular responses including cell growth, differentiation, and apoptosis (Parker et al., Current Biology, 5:577-99 (1995) ; Yao et al., Science, 267:2003-05 (1995)). Though the downstream targets of phosphorylated lipids generated following PI 3-kinase activation have not been well characterized, emerging evidence suggests that pleckstrin-homology domain- and FYVE-finger domain-containing proteins are activated when binding to various phosphatidylinositol lipids (Sternmark et al., J Cell Sci, 122:4175-83 (1999); Lemmon et al., Trends Cell Biol, 7:237-42 (1997)). *In vitro,* some isoforms of protein kinase C (PKC) are directly activated by PIP3, and the PKC-related protein kinase, PKB, has been shown to be activated by PI 3-kinase (Burgering et al., Nature, 376:599-602 (1995)).

Presently, the PI 3-kinase enzyme family has been divided into three classes based on their substrate specificities. Class I PI3Ks can phosphorylate phosphatidylinositol (PI), phosphatidylinositol-4-phosphate, and phosphatidylinositol-4,5-biphosphate (PIP2) to produce phosphatidylinositol-3-phosphate (PIP), phosphatidylinositol-3,4-biphosphate, and phosphatidylinositol-3,4,5-triphosphate, respectively. Class II PI3Ks phosphorylate PI and phosphatidylinositol-4-phosphate, whereas Class III PI3Ks can only phosphorylate PI.

The initial purification and molecular cloning of PI 3-kinase revealed that it was a heterodimer consisting of p85 and p110 subunits (Otsu et al., Cell, 65:91-104 (1991); Hiles et al., Cell, 70:419-29 (1992)). Since then, four distinct Class I PI3Ks have been identified, designated PI3K α, β, δ, and γ, each consisting of a distinct 110 kDa catalytic subunit and a regulatory subunit. More specifically, three of the catalytic subunits, i.e., p110α, p110β and p110δ, each interact with the same regulatory subunit, p85; whereas p110γ interacts with a distinct regulatory subunit, p101. As described below, the patterns of expression of each of these PI3Ks in human cells and tissues are also distinct. Though a wealth of information has been accumulated in recent past on the cellular functions of PI 3-kinases in general, the roles played by the individual isoforms are largely unknown.

Cloning of bovine p110α has been described. This protein was identified as related to the *Saccharomyces cerevisiae* protein: Vps34p, a protein involved in vacuolar protein processing. The recombinant p110α product was also shown to associate with p85α, to yield a PI3K activity in transfected COS-1 cells. See Hiles et al., Cell, 70, 419-29 (1992).

The cloning of a second human p110 isoform, designated p110β, is described in Hu et al., Mol Cell Biol, 13:7677-88 (1993). This isoform is said to associate with p85 in cells, and to be ubiquitously expressed, as p110β mRNA has been found in numerous human and mouse tissues as well as in human umbilical vein endothelial cells, Jurkat human leukemic T cells, 293 human embryonic kidney cells, mouse 3T3 fibroblasts, HeLa cells, and NBT2 rat bladder carcinoma cells. Such wide expression suggests that this isoform is broadly important in signaling pathways.

Identification of the p110δ isoform of PI 3-kinase is described in Chantry et al., J Biol Chem, 272:19236-41 (1997). It was observed that the human p110δ isoform is expressed in a tissue-restricted fashion. It is expressed at high levels in lymphocytes and lymphoid tissues, suggesting that the protein might play a role in PI 3-kinase-mediated signaling in the immune system. Details concerning the P110δ isoform also can be found in U.S. Patent Nos. 5,858,753; 5,822,910; and 5,985,589. See also, Vanhaesebroeck et al., Proc Natl Acad Sci USA, 94:4330-5 (1997), and international publication WO 97/46688.

In each of the PI3Kα, β, and δ subtypes, the p85 subunit acts to localize PI 3-kinase to the plasma membrane by the interaction of its SH2 domain with phosphorylated tyrosine residues (present in an appropriate sequence context) in target proteins (Rameh et al., Cell, 83:821-30 (1995)). Two isoforms of p85 have been identified, p85α, which is ubiquitously expressed, and p85β, which is primarily found in the brain and lymphoid tissues (Volinia et al., Oncogene, 7:789-93 (1992)). Association of the p85 subunit to the PI 3-kinase p110α, β, or δ catalytic subunits appears to be required for the catalytic activity and stability of these enzymes. In addition, the binding of Ras proteins also upregulates PI 3-kinase activity.

The cloning of p110γ revealed still further complexity within the PI3K family of enzymes (Stoyanov et al., Science, 269:690-93 (1995)). The p110γ isoform is closely related to p110α and p110β (45-48% identity in the catalytic domain), but as noted does not make use of p85 as a targeting subunit. Instead, p110γ contains an additional domain termed a "pleckstrin homology domain" near its amino terminus. This domain allows interaction of p110γ with the βγ subunits of heterotrimeric G proteins and.this interaction appears to regulate its activity.

The p101 regulatory subunit for PI3Kgamma was originally cloned in swine, and the human ortholog identified subsequently (Krugmann et al., J Biol Chem, 274:17152-8 (1999)). Interaction between the N-terminal region of p101 with the N-terminal region of p110γ appears to be critical for the PI3Kγ activation through Gβγ mentioned above.

A constitutively active PI3K polypeptide is described in international publication WO 96/25488. This publication discloses preparation of a chimeric fusion protein in which a 102-residue fragment of p85 known as the inter-SH2 (iSH2) region is fused through a linker region to the N-terminus of murine p110. The p85 iSH2 domain apparently is able to activate PI3K activity in a manner comparable to intact p85 (Klippel et al., Mol Cell Biol, 14:2675-85 (1994)).

Thus, PI 3-kinases can be defined by their amino acid identity or by their activity. Additional members of this growing gene family include more distantly related lipid and protein kinases including Vps34 TOR1, and TOR2 of *Saccharomyces cerevisiae* (and their mammalian homologs such as FRAP and mTOR), the ataxia telangiectasia gene product (ATR) and the catalytic subunit of DNA-dependent protein kinase (DNA-PK). See generally, Hunter, Cell, 83:1-4 (1995).

PI 3-kinase also appears to be involved in a number of aspects of leukocyte activation. A p85-associated PI 3-kinase activity has been shown to physically associate with the cytoplasmic domain of CD28, which is an important costimulatory molecule for the activation of T-cells in response to antigen (Pages et al., Nature, 369: 327-29 (1994) ; Rudd, Immunity, 4:527-34 (1996)). Activation of T cells through CD28 lowers the threshold for activation by antigen and increases the magnitude and duration of the proliferative response. These effects are linked to increases in the transcription of a number of genes including interleukin-2 (IL2), an important T cell growth factor (Fraser et al., Science, 251:313-16 (1991)). Mutation of CD28 such that it can no longer interact with PI 3-kinase leads to a failure to initiate IL2 production, suggesting a critical role for PI 3-kinase in T cell activation.

Specific inhibitors against individual members of a family of enzymes provide invaluable tools for deciphering functions of each enzyme. Two compounds, LY294002 and wortmannin, have been widely used as PI 3-kinase inhibitors. These compounds, however, are nonspecific PI3K inhibitors, as they do not distinguish among the four members of Class I PI 3-kinases. For example, the IC₅₀ values of wortmannin against each of the various Class I PI 3-kinases are in the range of 1-10 nM. Similarly, the IC₅₀ values for LY294002 against each of these PI 3-kinases is about 1 µM (Fruman et al., Ann Rev Biochem, 67:481-507 (1998)). Hence, the utility of these compounds in studying the roles of individual Class I PI 3-kinases is limited.

Based on studies using wortmannin, there is evidence that PI 3-kinase function also is required for some aspects of leukocyte signaling through G-protein coupled receptors (Thelen et al., Proc Natl Acad Sci USA, 91:4960-64 (1994)). Moreover, it has been shown that wortmannin and LY294002 block neutrophil migration and superoxide release. However, inasmuch as these compounds do not distinguish among the various isoforms of PI3K, it remains unclear which particular PI3K isoform or isoforms are involved in these phenomena.

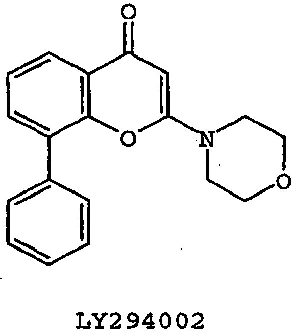

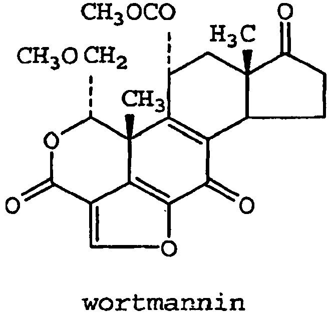

In view of the above considerations, it is clear that existing knowledge is lacking with respect to structural and functional features of the PI 3-kinase enzymes, including subcellular localization, activation states, substrate affinities, and the like. Moreover, the functions that these enzymes perform in both normal and diseased tissues remains to be elucidated. In particular, the function of PI3Kδ in leukocytes has not previously been characterized, and knowledge concerning its function in human physiology remains limited. The coexpression in these tissues of other PI3K isoforms has heretofore confounded efforts to segregate the activities of each enzyme. Furthermore, separation of the activities of the various PI3K isozymes may not be possible without identification of inhibitors that demonstrate selective inhibition characteristics. Indeed, Applicants are not presently aware that such selective, or better, specific, inhibitors of PI3K isozymes have been demonstrated.

Thus, there exists a need in the art for further structural characterization of the PI3Kδ polypeptide. There also exists a need for functional characterization of PI3Kδ. Furthermore, our understanding of PI3Kδ requires further elaboration of the structural interactions of p110δ, both with its regulatory subunit and with other proteins in the cell. There also remains a need for selective or specific inhibitors of PI3K isozymes, in order that the functions of each isozymes can be better characterized. In particular, selective or specific inhibitors of PI3Kδ are desirable for exploring the role of this isozyme and for development of pharmaceuticals to modulate the activity of the isozyme.

One aspect of the present invention is to provide compounds that can inhibit the biological activity of human PI3Kδ. Another aspect of the invention is to provide compounds that inhibit PI3Kδ selectively while having relatively low inhibitory potency against the other PI3K isoforms. Other aspects and advantages of the invention will be readily apparent to the artisan having ordinary skill in the art.

### SUMMARY OF PRIOR ART

WO 94/17090 A discloses 2,6-diaminopurine derivatives for use in medicine as anti-inflammatory agents, particularly in the treatment of patients with inflammatory conditions who are susceptible to leukocyte-induced tissue damage.

EP-A-0 675 124 discloses purine derivatives or pharmaceutically acceptable salts as suppressants for inflammatory diseases.

According to the present invention, there is provided a compound having a general structural formula 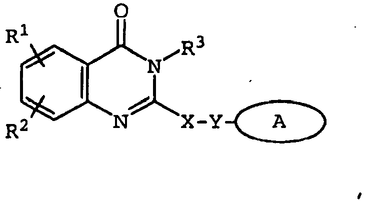
wherein the A ring system is selected from the group consisting of imidazolyl, pyrazolyl, 1,2,3-triazolyl, pyridizinyl, pyrimidinyl, pyrazinyl, 1,3,5-triazinyl, cinnolinyl, phthalazinyl, quinazolinyl, quinoxalinyl, 1,8-naphthyridinyl, pteridinyl, 1H-indazolyl, and benzimidazolyl
each of which which may be unsubstituted or substituted with one to three substituents selected from the group consisting of N(R^{a})₂, halo, C₁₋₃alkyl, S(C₁₋₃alkyl), OR^{a}, halo, and

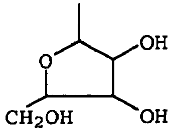

wherein X is selected from the group consisting of CHR^{b}, CH₂CHR^{b}, and CH=C(R^{b});
Y is selected from the group consisting of null, SO, SO₂, NH, O, C(=O), OC(=O), C(=O)O, and NHC(=O)CH₂S;
R¹ and R², independently, are selected from the group consisting of hydrogen, C₁₋₆alkyl, aryl, heteroaryl, halo, NHC(=O)C₁₋₃alkyleneN(R^{a})₂, NO₂, OR^{a}, OCF₃, N(R^{a})₂, CN, OC(=O)R^{a}, C(=O)R^{a}, C(=O)OR^{a}, arylOR^{b}, Het, NR^{a}C (=O)C₁₋₃alkyleneC(=O)OR^{a}, arylOC₁₋₃alkyleneN(R^{a})₂, arylOC (=O) R^{a}, C₁₋₄alkyleneC (=O) OR^{a}, OC₁₋₄alkyleneC(=O)OR^{a}, C₁₋₄alkyleneOC₁₋₄alkyleneC (=O) OR^{a}, C (=O) NR^{a}SO₂R^{a}, C₁₋₄alkyleneN(R^{a})₂, C₂₋₆alkenyleneN(R^{a})₂, C(=O)-NR^{a}C₁₋₄alkyleneOR^{a}, C(=O)NR^{a}C₁₋₄alkyleneHet, OC₂₋₄alkyleneN(R^{a})₂, OC₁₋₄alkyleneCH(OR^{b})CH₂N (R^{a})₂, OC₁₋₄alkyleneHet, OC₂₋₄alkyleneOR^{a}, OC₂₋₄alkyleneNR^{a}C (=O)OR^{a}, NR^{a}C₁₋₄alkyleneN(R^{a})₂, NR^{a}C(=O)R^{a}, NR^{a}C(=O)N(R^{a})₂, N(SO₂C₁₋₄alkyl)₂, NR^{a}(SO₂C₁₋₄alkyl), SO₂N(R^{a})₂, OSO₂CF₃, C₁₋₃alkylenearyl, C₁₋₄alkyleneHet, C₁₋₆alkyleneOR^{b}, C₁₋₃alkyleneN(R^{a})₂, C(=O)N(R^{a})₂, NHC(=O)C₁-C₃alkylenearyl, C₃₋₈cycloalkyl, C₃₋₈heterocycloalkyl, arylOC₁₋₃alkyleneN(R^{a})₂, arylOC(=O)R^{b}, NHC(=O)C₁₋₃alkylene-C₃₋₈heterocycloalkyl, NHC(=O)C₁₋₃alkyleneHet, OC₁₋₄alkyleneOC₁₋₄alkyleneC(=O)OR^{b}, C(=O)C₁₋₄alkyleneHet, and NHC(=O)haloC₁₋₆alkyl ;
or R¹ and R² are taken together to form a 3- or 4-membered alkylene or alkenylene chain component of a 5- or 6-membered ring, optionally containing at least one heteroatom;
R³ is hydrogen or is selected from the group consisting of unsubstituted or substituted C₁₋₆alkyl, C₃₋₈cycloalkyl, C₃₋₈heterocycloalkyl, C₁₋₄alkylenecycloalkyl, C₂₋₆alkenyl, C₁₋₃alkylenearyl, ' arylcᵢ-₃alkyl, C (=O) R^{a}, aryl, heteroaryl, C(=O)OR^{a}, C(=O)N(R^{a})₂, C(=S)N(R^{a})₂, SO₂R^{a}, SO₂N(R^{a})₂, S(=O)R^{a}, S(=O)N(R^{a})₂, C(=O)NR^{a}C₁₋₄alkyleneOR^{a}, C(=O)NR^{a}C₁₋₄alkyleneHet, C(=O)C₁₋₄alkylenearyl, C(=O)C₁₋₄alkyleneheteroaryl, C₁₋₄alkylenearyl optionally substituted with one or more of halo, SO₂N(R^{a})₂, N(R^{a})₂, C(=O)OR^{a}, NR^{a}SO₂CF₃, CN, NO₂, C(=O)R^{a}, OR^{a}, C₁₋₄alkylene-N(R^{a})₂, and OC₁₋₄alkyleneN(R^{a})₂, C₁₋₄alkyleneheteroaryl, C₁₋₄alkyleneHet, C₁₋₄alkyleneC(=O)C₁₋₄alkylenearyl, C₁₋₄alkyleneC(=O)C₁₋₄alkyleneheteroaryl, C₁₋₄alkylene-C (=O)Het, C₁₋₄alkyleneC (=O)N(R^{a})₂, C₁₋₄alkyleneOR^{a}, C₁₋₄alkyleneNR^{a}C(=O)R^{a}, C₁₋₄alkyleneOC₁₋₄alkyleneOR^{a}, C₁₋₄alkyleneN(R^{a})₂, C₁₋₄alkyleneC(=O)OR^{a}, and C₁₋₄alkyleneOC₁₋₄alkyleneC(=O)OR^{a}; wherein the R³ substituent, if present, is selected from the group consisting of halo, OR^{a}, C₁₋₆alkyl, aryl, heteroaryl, NO₂, N(R^{a})₂, NR^{a}SO₂CF₃, NR^{a}C(=O)R^{a}, C(=O)OR^{a}, SO₂N(R^{a})₂, CN, C(=O)R^{a}, C₁₋₄alkyleneN(R^{a})₂, OC₁₋₄alkyleneN(R^{a})₂, and N(R^{a})C₁₋₄alkyleneN(R^{a})₂
R^{a} is selected from the group consisting of hydrogen, C₁₋₆alkyl, C₃₋₈cycloalkyl, C₃₋₈heterocycloalkyl, C₁₋₃alkyleneN(R^{a})₂, aryl, arylC₁₋₃alkyl, C₁₋₃alkylenearyl, heteroaryl, heteroarylC₁₋₃alkyl, and C₁₋₃alkyleneheteroaryl;
or two R^{a} groups are taken together to form a 5- or 6-membered ring, optionally containing at least one heteroatom;
R^{b} is selected from the group consisting of hydrogen, C₁₋₆alkyl, aryl, heteroaryl, arylC₁₋₃alkyl, heteroarylC₁₋₃alkyl, C₁₋₃alkylenearyl, and C₁₋₃alkylene-heteroaryl;
Het is a 5- or 6-membered heterocyclic ring, saturated or partially or fully unsaturated, containing at least one heteroatom selected from the group consisting of oxygen, nitrogen, and sulfur, and optionally substituted with C₁₋₄alkyl or C(=O)OR^{a};
and pharmaceutically acceptable salts and solvates thereof,
wherein "aryl" is defined as a monocyclic or polycyclic aromatic group which may be unsubstituted or substituted with one or
   more halo, alkyl, phenyl, hydroxyalkyl, alkoxy, alkoxyalkyl, haloalkyl, nitro, amino, alkylamino, acylamino, alkylthio, alkylsulfinyl, and alkylsulfonyl and wherein "heteroaryl" is defined as a monocyclic or bicyclic ring system containing one or two aromatic rings and containing at least one nitrogen, oxygen or sulfur atom in an aromatic ring, which may be unsubsitituted or substituted with one or more halo, alkyl, hydroxy, hydroxyalkyl, alkoxy, alkoxyalkyl, haloalkyl, nitro, amino, alkylamino, acylamino, alkylthio, Alkylsulfinyl, and alkylsulfonyl.

Preferably, X is selected from the group consisting of CH₂, CH₂CH₂, CH=CH, CH(CH₃), CH₂CH(CH₃), and C(CH₃)₂.

Advantageously Y is selected from the group consisting of null and NH.

Conviniently, the A ring system is unsubstituted.

Preferably, the ring system is selected from the group consisting of 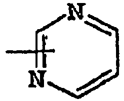 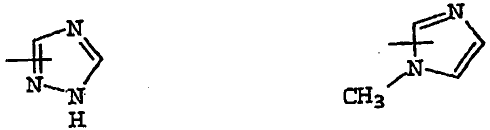 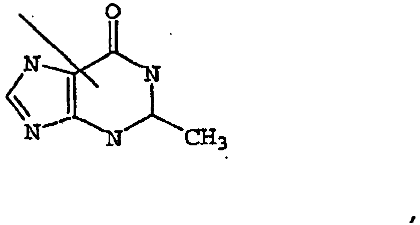 and

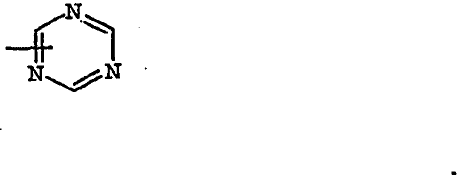

Conveniently the A ring substituent is substituted with one to three substituents selected from the group consisting of N(R^{a})₂, halo, C₁₋₃alkyl, S(C₁₋₃alkyl), OR^{a}, halo, and

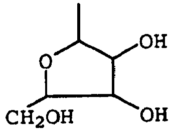

Advantageously the A ring system is substituted with one to three substituents selected from the group consisting of NH₂, NH(CH₃), N(CH₃)₂, NHCH₂C₆H₅, NH(C₂H₅), Cl, F, CH₃, SCH₃, OH, and

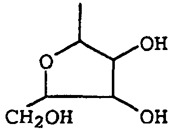

Preferably R¹ and R², are independently, selected from the group consisting of hydrogen, OR^{a}, halo, C₁₋₆alkyl, CF₃, NO₂, N(R^{a})₂, NR^{a}C₁₋₃alkyleneN(R^{a})₂, and OC₁₋₃alkyleneOR^{a}.

Conveniently R¹ and R² are independently selected from H, OCH₃, Cl, Br, F, CH₃, CF₃, NO₂, OH, N(CH3)₂, 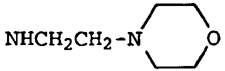 and O (CH₂)₂OCH₂C₆H₅.

Advantageously R¹ and R² are taken together to form a five- or six-membered ring.

Advantageously R³ is selected from the group consisting of C₁₋₆alkyl, aryl, heteroaryl, C₃₋₈cycloalkyl, C₃₋₈heterocycloalkyl, C(=O)OR^{a}, C₁₋₄alkyleneHet, C₁₋₄alkylenecycloalkyl, C₁₋₄alkylenearyl, C₁₋₄alkyleneC(=O)C₁₋₄alkylenearyl, C₁₋₄alkyleneC(=O)OR^{a}, C₁₋₄alkyleneC(=O)N(R^{a})₂, C₁₋₄alkyleneC(=O)Het, C₁₋₄alkyleneN(R^{a})₂, and C₁₋₄alkyleneNR^{a}C (=O)R^{a}.

Conveniently R³ is selected from the group consisting of OR^{a}, C₁₋₆alkyl, aryl, heteroaryl, NO₂, N(R^{a})₂, NR^{a}C(=O)R^{a}, C(=O)OC₂H₅, CH₂CH(CH₃)₂,

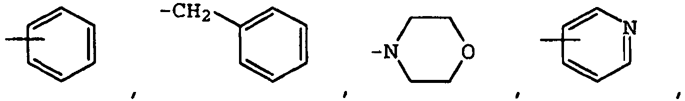

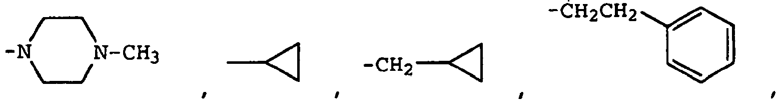

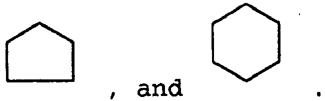

Advantageously R³ is substituted with a substituent selected from the group consisting of halo, OR^{a}, C₁₋₆alkyl, aryl, heteroaryl, NO₂, N(R^{a})₂, NR^{a}SO₂CF₃, NR^{a}C(=O)R^{a}, C (=O)OR^{a}, SO₂N(R^{a})₂, CN, C (=O)R^{a}, C₁₋₄alkyleneN(R^{a})₂, OC₁₋₄alkyleneN(R^{a})₂, and N(R^{a})C₁₋₄alkyleneN(R^{a})₂.

Preferably R³ is substituted with a substituent selected from the group consisting of Cl, F, CH₃, CH(CH₃)₂, OCH₃, C₆H₅, NO₂, NH₂, NHC(=O)CH₃, CO₂H, and N (CH₃)CH₂CH₂N (CH₃)₂.

The invention also includes a pharmaceutical composition comprising a compound according to the invention together with a pharmaceutically acceptable carrier.

There is also provided a compound or a composition according to the invention for use in therapy.

Conveniently the compound or composition is for use in a method of ameliorating a bone-resorption disorder in an animal.

Said bone-resorption disorder may be osteoporosis.

Advantageously the compound or composition is for use in a method of inhibiting the growth or proliferation of chronic myelogenous leukemia cells.

Preferably the compound or composition is for the treatment of a disease selected from
i) ischemia, preferably resulting from cardiac arrest, myocardial infarction, obstruction of a coronary arteries, thromboembolytic occlusion of a cerebral vessel, traumatic head injury, edema and brain tumour,
ii) reperfusion injury, preferably associated with vascular stroke, hemorrhagic shock, myocardial ischemia or infarction, organ transplantation and cerebral vasospasm,
iii) bone diseases, preferably selected from osteoporosis, Paget's diseases and related bone resorption disorders,
iv) hematopoitic cancers, preferably;
   a) lymphomas preferably selected from Burkitt's lymphoma, Hodgkins' lymphoma, non-Hodgkins lymphoma, lymphocytic lymphomas;
   b) multiple myelomas
   c) leukemias preferably selected from lymphocytic leukemias, chronic myeloid (myelogenous) leukemias,
v) diseases related to histamine release, preferably selected from chronic obstructive pulmonary disease (COPD), asthma, ARDS and emphysema,
vi) arthritic diseases, preferably selected from rheumatoid arthritis, monoarticular arthritis, osteoarthritis, gouty arthritis, spondylitis,
vii) Behcet disease,
viii) sepsis, septic shock, endotoxic shock, gram negative sepsis, gram positive sepsis and toxic shock syndrome,
ix) multiple organ injury syndrome secondary to septicemia, trauma or hemorrhage,
x) ophthalmic disorders, preferably selected from allergic conjunctivitis, vernal conjunctivitis, uveitis and thyroid-associated opthalmopathy,
xi) eosinophilic granuloma,
xii) pulmonary or respiratory disorders, preferably selected from asthma, chronic bronchitis, allergic rhinitis, ARDS, chronic pulmonary inflammatory disease, chronic obstructive pulmonary disease, silicosis, pulmonary sarcoidosis, pleurisy, alveolitis, vasculitis, emphysema, pneumonia, bronchiectasis and pulmonary oxygen toxicity,
xiii) fibrosis, preferably cystic fibrosis,
xiv) keloid formation or scar tissue formation,
xv) atherosclerosis
xvi) autoimmune diseases, preferably selected from systemic lupus erythematous (SLE), autoimmune thyroiditis, multiple sclerosis, some forms of diabetes and Reynaud's syndrome,
xvii) transplant rejection disorders, preferably selected from GVHD and allograft rejection,
xviii) chronic glomerulonephritis,
xix) inflammatory bowel diseases, preferably selected from chronic inflammatory bowel disease (CIBD), Crohn's disease, ulcerative colitis and necrotizing enterocolitis,
xx) inflammatory dermatoses, preferably selected from contact dermatitis, atopic dermatitis, psoriasis and urticaria,
xxi) fever and myalgias due to infection,
xxii) central or peripheral nervous system inflammatory disorders, preferably selected from meningitis, encephalitis and brain or spinal cord injury due to minor trauma,
xxiii) Sjögren's disease,
xxiv) diseases involving leukocyte diapedesis,
xxv) alcoholic hepatitis,
xxvi) bacterial pneumonia,
xxvii) antigen-antibody complex mediated diseases
xxviii) hypovolemic shock,
xxix) Type I diabetes mellitus,
xxx) acute and delayed hypersensitivity,
xxxi) disease states due to leukocyte dyscrasia and metastasis,
xxxii) thermal injury,
xxxiii) granulocyte transfusion-associated syndromes, and
xxxiv) cytokine-induced toxicity.

Advantageously the compound or composition is for the treatment of a disease selected from the group consisting of leukemia, lymphoma and multiple myeloma.

Preferably the compound or composition is for the treatment of a disease selected from the group consisting of Burkitt's lymphoma, Hodgkin's lymphoma, non-Hodgkins lymphoma, lymphocytic lymphoma, multiple myeloma, lymphocytic leukemia, and chronic myeloid (myelognous) leukemia.

Conveniently the compound or composition is for the treatment of a disease selected from the group consisting of COPD, asthma, rheumatoid arthritis, diabetes, allergic rhinitis, and multiple sclerosis.

### BRIEF DESCRIPTION OF THE DRAWINGS

Figure 1 shows the effect of a selective PI3Kδ inhibitor Comparative Compound on the activity of three PI3K isoforms.
Figure 2 shows the effect of a selective PI3Kδ inhibitor on superoxide generation by human neutrophils in the presence of TNF or IgG.
Figure 3 shows the effect of a selective PI3Kδ inhibitor on superoxide generation by human neutrophils in the presence of TNF or fMLP.
Figure 4 shows the effect of a selective PI3Kδ inhibitor on elastase exocytosis in the presence of fMLP by human neutrophils.
Figure 5 shows the effect of a selective PI3Kδ inhibitor on fMLP-induced chemotaxis by human neutrophils.
Figure 6 shows that a selective PI3Kδ inhibitor does not affect phagocytosis and killing of S. aureus by neutrophils.
Figure 7 shows the effect of a selective PI3Kδ inhibitor on proliferation and antibody production by human B lymphocytes.
Figure 8 shows the effect of a selective PI3Kδ inhibitor on anti-IgM stimulated mouse splenic B lymphocyte proliferation.

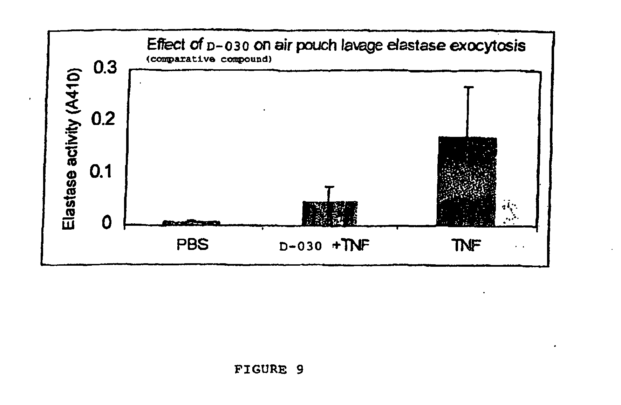

Figure 9 shows the effect of a selective PI3Kδ inhibitor on elastase exocytosis in an animal model.

### DETAILED DESCRIPTION OF THE PREFERRED EMBODIMENTS

The invention provides compounds that selectively inhibit the activity of PI3Kδ. The PI3Kδ enzyme can include a purified and isolated enzyme, wherein the enzyme is isolated from a natural source (e.g., cells or tissues that normally express a PI3Kδ polypeptide absent modification by recombinant technology) or isolated from cells modified by recombinant techniques to express exogenous enzyme.

The term "selective PI3Kδ inhibitor" as used herein refers to a compound that inhibits the PI3Kδ isozyme more effectively than other isozymes of the PI3K family. A "selective PI3Kδ inhibitor" compound is understood to be more selective for PI3Kδ than compounds conventionally and generically designated PI3K inhibitors, e.g., wortmannin or LY294002. Concomitantly, wortmannin and LY294002 are deemed "nonselective PI3K inhibitors." Compounds of any type that selectively negatively regulate PI3Kδ expression or activity can be used as selective PI3Kδ inhibitors in the methods of the invention. Moreover, compounds of any type that selectively negatively regulate PI3Kδ expression or activity and that possess acceptable pharmacological properties can be used as selective PI3Kδ inhibitors in the therapeutic methods of the disclosure.

The relative efficacies of compounds as inhibitors of an enzyme activity (or other biological activity) can be established by determining the concentrations at which each compound inhibits the activity to a predefined extent and then comparing the results. Typically, the preferred determination is the concentration that inhibits 50% of the activity in a biochemical assay, i.e., the 50% inhibitory concentration or "IC₅₀." IC₅₀ determinations can be accomplished using conventional techniques known in the art. In general, an IC₅₀ can be determined by measuring the activity of a given enzyme in the presence of a range of concentrations of the inhibitor under study. The experimentally obtained values of enzyme activity then are plotted against the inhibitor concentrations used. The concentration of the inhibitor that shows 50% enzyme activity (as compared to the activity in the absence of any inhibitor) is taken as the IC₅₀ value. Analogously, other inhibitory concentrations can be defined through appropriate determinations of activity. For example, in some settings it can be desirable to establish a 90% inhibitory concentration, i.e., IC₉₀, etc.

Accordingly, a "selective PI3Kδ inhibitor" alternatively can be understood to refer to a compound that exhibits a 50% inhibitory concentration (IC₅₀) with respect to PI3Kδ that is at least at least 10-fold, preferably at least 20-fold, and more preferably at least 30-fold, lower than the IC₅₀ value with respect to any or all of the other Class I PI3K family members. The term "specific PI3Kδ inhibitor" can be understood to refer to a selective PI3Kδ inhibitor compound that exhibits an IC₅₀ with respect to PI3Kδ that is at least 50-fold, preferably at least 100-fold, more preferably at least 200-fold, and still more preferably at least 500-fold, lower than the IC₅₀ with respect to any or all of the other PI3K Class I family members.

As used herein, the term "alkyl" includes straight chained and branched hydrocarbon groups containing the indicated number of carbon atoms, typically methyl, ethyl, and straight chain and branched propyl and butyl groups. The hydrocarbon group can contain up to 16 carbon atoms, preferably one to eight carbon atoms. The term "alkyl" includes "bridged alkyl," i.e., a C₅-C₁₆ bicyclic or polycyclic hydrocarbon group, for example, norbornyl, adamantyl, bicyclo[2.2.2]octyl, bicyclo[2.2.1]-heptyl, bicyclo[3.2.1]octyl, or decahydronaphthyl. The term "cycloalkyl" is defined as a cyclic C₃-C₈ hydrocarbon group, e.g., cyclopropyl, cyclobutyl, cyclohexyl, and cyclopentyl.

The term "alkenyl" is defined identically as "alkyl," except for containing a carbon-carbon double bond. "Cycloalkenyl" is defined similarly to cycloalkyl, except a carbon-carbon double bond is present in the ring.

The term "alkylene" refers to an alkyl group having a substituent. For example, the term "C₁₋₃alkylenearyl" refers to an alkyl group containing one to three carbon atoms, and substituted with an aryl group.

The term "halo" or "halogen" is defined herein to include fluorine, bromine, chlorine, and iodine.

The term "haloalkyl" is defined herein as an alkyl group substituted with one or more halo substituents, either fluoro, chloro, bromo, iodo, or combinations thereof. Similarly, "halocycloalkyl" is defined as a cycloalkyl group having one or more halo substituents.

The term "aryl," alone or in combination, is defined herein as a monocyclic or polycyclic aromatic group, preferably a monocyclic cr bicyclic aromatic group, e.g., phenyl or naphthyl. Unless otherwise indicated, an "aryl" group can be unsubstituted or substituted, for example, with one or more, and in particular one to three, halo, alkyl, phenyl, hydroxyalkyl, alkoxy, alkoxyalkyl, haloalkyl, nitro, amino, alkylamino, acylamino, alkylthio, alkylsulfinyl, and alkylsulfonyl. Exemplary aryl groups include phenyl, naphthyl, biphenyl, tetrahydronaphthyl, chlorophenyl, fluorophenyl, aminophenyl, methylphenyl, methoxyphenyl, trifluoromethylphenyl, nitrophenyl, carboxyphenyl, and the like. The terms "arylC₁₋₃alkyl" and "heteroaryl-C₁₋₃alkyl" are defined as an aryl or heteroaryl group having a C₁₋₃alkyl substituent.

The term "heteroaryl" is defined herein as a monocyclic or bicyclic ring system containing one or two aromatic rings and containing at least one nitrogen, oxygen, or sulfur atom in an aromatic ring, and which can be unsubstituted or substituted, for example, with one or more, and in particular one to three, substituents, like halo, alkyl, hydroxy, hydroxyalkyl, alkoxy, alkoxyalkyl, haloalkyl, nitro, amino, alkylamino, acylamino, alkylthio, alkylsulfinyl, and alkylsulfonyl. Examples of heteroaryl groups include thienyl, furyl, pyridyl, oxazolyl, quinolyl, isoquinolyl, indolyl, triazolyl, isothiazolyl, isoxazolyl, imidizolyl, benzothiazolyl, pyrazinyl, pyrimidinyl, thiazolyl, and thiadiazolyl.

The term "Het" is defined as monocyclic, bicyclic, and tricyclic groups containing one or more heteroatoms selected from the group consisting of oxygen, nitrogen, and sulfur. A "Het" group also can contain an oxo group (=O) attached to the ring. Nonlimiting examples of Het groups include 1,3-dioxolane, 2-pyrazoline, pyrazolidine, pyrrolidine, piperazine, a pyrroline, 2H-pyran, 4H-pyran, morpholine, thiopholine, piperidine, 1,4-dithiane, and 1,4-dioxane.

The term "hydroxy" is defined as -OH.

The term "alkoxy" is defined as -OR, wherein R is alkyl.

The term "alkoxyalkyl" is defined as an alkyl group wherein a hydrogen has been replaced by an alkoxy group. The term "(alkylthio)alkyl" is defined similarly as alkoxyalkyl, except a sulfur atom, rather than an oxygen atom, is present.

The term "hydroxyalkyl" is defined as a hydroxy group appended to an alkyl group.

The term "amino" is defined as -NH₂, and the term "alkylamino" is defined as -NR₂, wherein at least one R is alkyl and the second R is alkyl or hydrogen.

The term "acylamino" is defined as RC(=O)N, wherein R is alkyl or aryl.

The term "alkylthio" is defined as -SR, wherein R is alkyl.

The term "alkylsulfinyl" is defined as R-SO₂, wherein R is alkyl.

The term "amino" is defined as -NH₂, and the term "alkylamino" is defined as -NR₂, wherein at least one R is alkyl and the second R is alkyl or hydrogen.

The term "acylamino" is defined as RC(=O)N, wherein R is alkyl or aryl.

The term "alkylthio" is defined as -SR, wherein R is alkyl.

The term "alkylsulfinyl" is defined as R-SO₂, wherein R is alkyl.

The term "alkylsulfonyl" is defined as R-SO₃, wherein R is alkyl.

The term "nitro" is defined as -NO₂.

The term "trifluoromethyl" is defined as -CF₃.

The term "trifluoromethoxy" is defined as -OCF₃.

The term "cyano" is defined as -CN.

The term "prodrug" as used herein refers to compounds that are rapidly transformed *in vivo* to a compound having structural formula (I) hereinabove, for example, by hydrolysis. Prodrug design is discussed generally in Hardma et al. (Eds.), Goodman and Gilman's The Pharmacological Basis of Therapeutics, 9th ed., pp. 11-16 (1996). A thorough discussion is provided in Higuchi et al., Prodrugs as Novel Delivery Systems, Vol. 14, ASCD Symposium Series, and in Roche (ed.), Bioreversible Carriers in Drug Design, American Pharmaceutical Association and Pergamon Press (1987). Briefly, administration of a drug is followed by elimination from the body or some biotransformation whereby biological activity of the drug is reduced or eliminated. Alternatively, a biotransformation process can lead to a metabolic by-product, which is itself more active or equally active as compared to the drug initially administered. Increased understanding of these biotransformation processes permits the design of so-called "prodrugs," which, following a biotransformation, become more physiologically active in their altered state. Prodrugs, therefore, encompass pharmacologically inactive compounds that are converted to biologically active metabolites.

To illustrate, prodrugs can be converted into a' pharmacologically active form through hydrolysis of, for example, an ester or amide linkage, thereby introducing or exposing a functional group on the resultant product. The prodrugs can be designed to react with an endogenous compound to form a water-soluble conjugate that further enhances the pharmacological properties of the compound, for example, increased circulatory half-life. Alternatively, prodrugs can be designed to undergo covalent modification on a functional group with, for example, glucuronic acid, sulfate, glutathione, amino acids, or acetate. The resulting conjugate can be inactivated and excreted in the urine, or rendered more potent than the parent compound. High molecular weight conjugates also can be excreted into the bile, subjected to enzymatic cleavage, and released back into the circulation, thereby effectively increasing the biological half-life of the originally administered compound.

### Methods for Identifying Negative Regulators of PI3Kδ Activity

The PI3Kδ protein, as well as fragments thereof possessing biological activity, can be used for screening putative negative regulator compounds in any of a variety of drug screening techniques. A negative regulator of PI3Kδ is a compound that diminishes or abolishes the ability of PI3Kδ to carry out any of its biological functions. An example of such compounds is an agent that decreases the ability of a PI3Kδ polypeptide to phosphorylate phosphatidylinositol or to target appropriate structures within a cell. The selectivity of a compound that negatively regulates PI3Kδ activity can be evaluated by comparing its activity on the PI3Kδ to its activity on other proteins. Selective negative regulators include, for example, antibodies and other proteins or peptides that specifically bind to a PI3Kδ polypeptide, oligonucleotides that specifically bind to PI3Kδ polypeptides, and other nonpeptide compounds (e.g., isolated or synthetic organic molecules) that specifically interact with PI3Kδ polypeptides. Negative regulators also include compounds as described above, but which interact with a specific binding partner of PI3Kδ polypeptides.

Presently preferred targets for the development of selective negative regulators of PI3Kδ include, for example:
(1) cytoplasmic regions of PI3Kδ polypeptides that contact other proteins and/or localize PI3Kδ within a cell;
(2) regions of PI3Kδ polypeptides that bind specific binding partners;
(3) regions of the PI3Kδ polypeptides that bind substrate;
(4) allosteric regulatory sites of the PI3Kδ polypeptides that can or cannot interact directly with the active site upon regulatory signal;
(5) regions of the PI3Kδ polypeptides that mediate multimerization.
For example, one target for development of modulators is the identified regulatory interaction of p85 with p110δ, which can be involved in activation and/or subcellular localization or the p110δ moiety. Still other selective modulators include those that recognize specific regulatory or PI3Kδ-encoding nucleotide sequences. Modulators of PI3Kδ activity can be therapeutically useful in treatment of a wide range of diseases and physiological conditions in which aberrant PI3Kδ activity is involved.

Human PI3K polypeptides are amenable to biochemical or cell-based high throughput screening (HTS) assays according to methods known and practiced in the art, including melanophore assay systems to investigate receptor-ligand interactions, yeast-based assay systems, and mammalian cell expression systems. For a review, see Jayawickreme and Kost, Curr Opin Biotechnol, 8:629-34 (1997). Automated and miniaturized HTS assays also are comprehended as described, for example, in Houston and Banks, Curr Opin Biotechnol, 8:734-40 (1997).

Such HTS assays are used to screen libraries of compounds to identify particular compounds that exhibit a desired property. Any library of compounds can be used, including chemical libraries, natural product libraries, and combinatorial libraries comprising random or designed oligopeptides, oligonucleotides, or other organic compounds.

Chemical libraries can contain known compounds, proprietary structural analogs of known compounds, or compounds that are identified from natural product screening.

Natural product libraries are collections of materials isolated from naturals sources, typically, microorganisms, animals, plants, or marine organisms. Natural products are isolated from their sources by fermentation of microorganisms followed by isolation and extraction of the fermentation broths or by direct extraction from the microorganisms or tissues (plants or animal) themselves. Natural product libraries include polyketides, nonribosomal peptides, and variants (including nonnaturally occurring variants) thereof. For a review, see Cane et al., Science, 282:63-68 (1998).

Combinatorial libraries are composed of large numbers of related compounds, such as peptides, oligonucleotides, or other organic compounds as a mixture. Such compounds are relatively straightforward to design and prepare by traditional automated synthesis protocols, PCR, cloning, or proprietary synthetic methods. Of particular interest are peptide and oligonucleotide combinatorial libraries.

Still other libraries of interest include peptide, protein, peptidomimetic, multiparallel synthetic collection, recombinatorial, and polypeptide libraries. For a review of combinatorial chemistry and libraries created thereby, see Myers, Curr Opin Biotechnol, 8:701-07 (1997).

Once compounds have been identified that show activity as negative regulators of PI3Kδ function, a program of optimization can be undertaken in an effort to improve the potency and or selectivity of the activity. This analysis of structure-activity relationships (SAR) typically involves of iterative series of selective modifications of compound structures and their correlation to biochemical or biological activity. Families of related compounds can be designed that all exhibit the desired activity, with certain members of the family, namely those possessing suitable pharmacological profiles, potentially qualifying as therapeutic candidates.

### Therapeutic Uses of Inhibitors of PI3Kδ Activity (for background information, and not part of the claimed invention)

The disclosure provides a method for selecttively or specifically inhibiting PI3Kδ activity therapeutically or prophylactically. The method comprises administering a selective or specific inhibitor of PI3Kδ activity in an amount effective therefor. This method can be employed in treating humans or animals who are or can be subject to any condition whose symptoms or pathology is mediated by PI3Kδ expression or activity.

"Treating" as used herein refers to preventing a disorder from occurring in an animal that can be predisposed to the disorder, but has not yet been diagnosed as having it; inhibiting the disorder, i.e., arresting its development; relieving the disorder, i.e., causing its regression; or ameliorating the disorder, i.e., reducing the severity of symptoms associated with the disorder. "Disorder" is intended to encompass medical disorders, diseases, conditions, syndromes, and the like, without limitation.

The methods embrace various modes of treating an animal subject, preferably a mammal, more preferably a primate, and still more preferably a human. Among the mammalian animals that can be treated are, for example, companion animals (pets), including dogs and cats; farm animals, including cattle, horses, sheep, pigs, and goats; laboratory animals, including rats, mice, rabbits, guinea pigs, and nonhuman primates, and zoo specimens. Nonmammalian animals include, for example, birds, fish, reptiles, and amphibians.

In one aspect, the method can be employed to treat subjects therapeutically or prophylactically who have or can be subject to an inflammatory disorder. One aspect derives from the involvement of PI3Kδ in mediating aspects of the inflammatory process. Without intending to be bound by any theory, it is theorized that, because inflammation involves processes are typically mediated by leukocyte (e.g., neutrophil, lymphocyte, etc.) activation and chemotactic transmigration, and because PI3Kδ can mediate such phenomena, antagonists of PI3Kδ can be used to suppress injury associated with inflammation.

"Inflammatory disorder" as used herein can refer to any disease, disorder, or syndrome in which an excessive or unregulated inflammatory response leads to excessive inflammatory symptoms, host tissue damage, or loss of tissue function. "Inflammatory disorder" also refers to a pathological state mediated by influx of leukocytes and/or neutrophil chemotaxis.

"Inflammation" as used herein refers to a localized, protective response elicited by injury or destruction of tissues, which serves to destroy, dilute, or wall off (sequester) both the injurious agent and the injured tissue. Inflammation is notably associated with influx of leukocytes and/or neutrophil chemotaxis. Inflammation can result from infection with pathogenic organisms and viruses and from noninfectious means such as trauma or reperfusion following myocardial infarction or stroke, immune response to foreign antigen, and autoimmune responses. Accordingly, inflammatory disorders encompass disorders associated with reactions of the specific defense system as well as with reactions of the nonspecific defense system.

As used herein, the term "specific defense system" refers to the component of the immune system that reacts to the presence of specific antigens. Examples of inflammation resulting from a response of the specific defense system include the classical response to foreign antigens, autoimmune diseases, and delayed type hypersensitivity response mediated by T-cells. Chronic inflammatory diseases, the rejection of solid transplanted tissue and organs, e.g., kidney and bone marrow transplants, and graft versus host disease (GVHD), are further examples of inflammatory reactions of the specific defense system.

The term "nonspecific defense system" as used herein refers to inflammatory disorders that are mediated by leukocytes that are incapable of immunological memory (e.g., granulocytes, and macrophages). Examples of inflammation that result, at least in part, from a reaction of the nonspecific defense system include inflammation associated with conditions such as adult (acute) respiratory distress syndrome (ARDS) or multiple organ injury syndromes; reperfusion injury; acute glomerulonephritis; reactive arthritis; dermatoses with acute inflammatory components; acute purulent meningitis or other central nervous system inflammatory disorders such as stroke; thermal injury; inflammatory bowel disease; granulocyte transfusion associated syndromes; and cytokine-induced toxicity.

"Autoimmune disease" as used herein refers to any group of disorders in which tissue injury is associated with humoral or cell-mediated responses to the body's own constituents. "Allergic disease" as used herein refers to any symptoms, tissue damage, or loss of tissue function resulting from allergy. "Arthritic disease" as used herein refers to any disease that is characterized by inflammatory lesions of the joints attributable to a variety of etiologies. "Dermatitis" as used herein refers to any of a large family of diseases of the skin that are characterized by inflammation of the skin attributable to a variety of etiologies. "Transplant rejection" as used herein refers to any immune reaction directed against grafted tissue, such as organs or cells (e.g., bone marrow), characterized by a loss of function of the grafted and surrounding tissues, pain, swelling, leukocytosis, and thrombocytopenia.

The therapeutic methods of the present disclosure include methods for the treatment of disorders associated with inflammatory cell activation. "Inflammatory cell activation" refers to the induction by a stimulus (including, but not limited to, cytokines, antigens or auto-antibodies) of a proliferative cellular response, the production of soluble mediators (including but not limited to cytokines, oxygen radicals, enzymes, prostanoids, or vasoactive amines), or cell surface expression of new or increased numbers of mediators (including, but not limited to, major histocompatability antigens or cell adhesion molecules) in inflammatory cells (including but not limited to monocytea, macrophages, T lymphocytes, B lymphocytes, granulocytes (i.e., polymorphonuclear leukocytes such as neutrophils, basophils, and eosinophils), mast cells, dendritic cells, Langerhans cells, and endothelial cells). It will be appreciated by persons skilled in the art that the activation of one or a combination of these phenotypes in these cells can contribute to the initiation, perpetuation, or exacerbation of an inflammatory disorder.

The compounds of the invention have been found to inhibit superoxide release by neutrophils. Superoxide is released by neutrophils in response to any of a variety of stimuli, including signals of infection, as a mechanism of cell killing. For example, superoxide release is known to be induced by tumor necrosis factor alpha (TNFα), which is released by macrophages, mast cells, and lymphocytes upon contact with bacterial cell wall components such as lipopolysaccharide (LPS). TNFα is an extraordinarily potent and promiscuous activator of inflammatory processes, being involved in activation of neutrophils and various other cell types, induction of leukocyte/endothelial cell adhesion, pyrexia, enhanced MHC class I production, and stimulation of angiogenesis. Alternatively, superoxide release can be stimulated by formyl-Met-Leu-Phe (fMLP) or other peptides blocked at the N-terminus by formylated methionine. Such peptides are not normally found in eukaryotes, but are fundamentally characteristic of bacteria, and signal the presence of bacteria to the immune system. Leukocytes expressing the fMLP receptor, e.g., neutrophils and macrophages, are stimulated to migrate up gradients of these peptides (i.e., chemotaxis) toward loci of infection. As demonstrated herein, the compounds of the invention inhibit stimulated superoxide release by neutrophils in response to either TNFα or fMLP. Other functions of neutrophils, including stimulated exocytosis and directed chemotactic migration, also have been shown to be inhibited by the PI3Kδ inhibitors of the invention. Accordingly, the compounds of the invention can be expected to be useful in treating disorders, such as inflammatory disorders, that are mediated by any or all of these neutrophil functions.

The present invention provides compounds for treating such diseases as arthritic diseases, such as rheumatoid arthritis, monoarticular arthritis, osteoarthritis, gouty arthritis, spondylitis; Behcet disease; sepsis, septic shock, endotoxic shock, gram negative sepsis, gram positive sepsis', and toxic shock syndrome; multiple organ injury syndrome secondary to septicemia, trauma, or hemorrhage; ophthalmic disorders such as allergic conjunctivitis, vernal conjunctivitis, uveitis, and thyroid-associated ophthalmopathy; ecsinophilic granuloma; pulmonary or respiratory disorders such as asthma, chronic bronchitis, allergic rhinitis, ARDS, chronic pulmonary inflammatory disease (e.g., chronic obstructive pulmonary disease), silicosis, pulmonary sarcoidosis, pleurisy, alveolitis, vasculitis, emphysema, pneumonia, bronchiectasis, and pulmonary oxygen toxicity; reperfusion injury of the myocardium, brain, or extremities; fibrosis such as cystic fibrosis; keloid formation or scar tissue formation; atherosclerosis; autoimmune diseases, such as systemic lupus erythematosus (SLE), autoimmune thyroiditis, multiple sclerosis, some forms of diabetes, and Reynaud's syndrome; and transplant rejection disorders such as GVHD and allograft rejection; chronic glomerulonephritis; inflammatory bowel diseases such as chronic inflammatory bowel disease (CIBD), Crohn's disease, ulcerative colitis, and necrotizing enterocolitis; inflammatory dermatoses such as contact dermatitis, atopic dermatitis, psoriasis, or urticaria; fever and myalgias due to infection; central or peripheral nervous system inflammatory disorders such as meningitis, encephalitis, and brain or spinal cord injury due to minor trauma; Sjögren's syndrome; diseases involving leukocyte diapedesis; alcoholic hepatitis; bacterial pneumonia; antigen-antibody complex mediated diseases; hypovolemic shock; Type I diabetes mellitus; acute and delayed hypersensitivity; disease states due to leukocyte dyscrasia and metastasis; thermal injury; granulocyte transfusion-associated syndromes; and cytokine-induced toxicity.

The compounds can have utility in treating subjects who are or can be subject to reperfusion injury, i.e., injury resulting from situations in which a tissue or organ experiences a period of ischemia followed by reperfusion. The term "ischemia" refers to localized tissue anemia due to obstruction of the inflow of arterial blood. Transient ischemia followed by reperfusion characteristically results in neutrophil activation and transmigration through the endothelium of the blood vessels in the affected area. Accumulation of activated neutrophils in turn results in generation of reactive oxygen metabolites, which damage components of the involved tissue or organ. This phenomenon of "reperfusion injury" is commonly associated with conditions such as vascular stroke (including global and focal ischemia), hemorrhagic shock, myocardial ischemia or infarction, organ transplantation, and cerebral vasospasm. To illustrate, reperfusion injury occurs at the termination of cardiac bypass procedures or during cardiac arrest when the heart, once prevented from receiving blood, begins to reperfuse. It is expected that inhibition of PI3Kδ activity will result in reduced amounts of reperfusion injury in such situations.

With respect to.the nervous system, global ischemia occurs when blood flow to the entire brain ceases for a period. Global ischemia can result from cardiac arrest. Focal ischemia occurs when a portion of the brain is deprived of its normal blood supply. Focal ischemia can result from thromboembolytic occlusion of a cerebral vessel, traumatic head injury, edema, or brain tumor. Even if transient, both global and focal ischemia can cause widespread neuronal damage. Although nerve tissue damage occurs over hours or even days following the onset of ischemia, some permanent nerve tissue damage can develop in the initial minutes following the cessation of blood flow to the brain.

Ischemia also can occur in the heart in myocardial infarction and other cardiovascular disorders in which the coronary arteries have been obstructed as a result of atherosclerosis, thrombus, or spasm. Accordingly, the invention is believed to be useful for treating cardiac tissue damage, particularly damage resulting from cardiac ischemia or caused by reperfusion injury in mammals.

In another aspect, selective inhibitors of PI3Kδ activity, such as the compounds of the invention, can be employed in methods of treating diseases of bone, especially diseases in which osteoclast function is abnormal or undesirable. As shown in Example 6, below, compounds of the invention inhibit osteoclast function in vitro. Accordingly, the use of such compounds and other PI3Kδ selective inhibitors can be of value in treating osteoporosis, Paget's disease, and related bone resorption disorders.

In a further aspect, the invention includes PI3Kδ inhibitory compounds for inhibiting the growth or proliferation of cancer cells of hematopoietic origin, preferably cancer cells of lymphoid origin, and more preferably cancer cells related to or derived from B lymphocytes or B lymphocyte progenitors. Cancers amenable to treatment using the compounds of the invention include, without limitation, lymphomas, e.g., malignant neoplasms of lymphoid and reticuloendothelial tissues, such as Burkitt's lymphoma, Hodgkins' lymphoma, non-Hodgkins lymphomas, lymphocytic lymphomas and the like; multiple myelomas; as well as leukemias such as lymphocytic leukemias, chronic myeloid (myelogenous) leukemias, and the like. In a preferred embodiment, PI3Kδ inhibitory compounds can be used to inhibit or control the growth or proliferation of chronic myeloid (myelogenous) leukemia cells.

In another aspect, the disclosure includes a method for suppressing a function of basophils and/or mast cells, and thereby enabling treatment of diseases or disorders characterized by excessive or undesirable basophil and/or mast cell activity. According to the method, a compound of the invention can be used that selectively inhibits the expression or activity of phosphatidylinositol 3-kinase delta (PI3Kδ) in the basophils and/or mast cells. Preferably, the method employs a PI3Kδ inhibitor in an amount sufficient to inhibit stimulated histamine release by the basophils and/or mast cells. Accordingly, the use of such compounds and other PI3Kδ selective inhibitors can be of value in treating diseases characterized by histamine release, i.e., allergic disorders, including disorders such as chronic obstructive pulmonary disease (COPD), asthma, ARDS, emphysema, and related disorders.

### Pharmaceutical Compositions of Inhibitors of PI3Kδ Activity

A compound of the present invention can be administered as the neat chemical, but it is typically preferable to administer the compound in the form of a pharmaceutical composition or formulation. Accordingly, the present invention also provides pharmaceutical compositions that comprise a chemical or biological compound ("agent") that is active as a modulator of PI3Kδ activity and a biocompatible pharmaceutical carrier, adjuvant, or vehicle. The composition can include the agent as the only active moiety or in combination with other agents, such as oligo- or polynucleotides, oligo- or polypeptides, drugs, or hormones mixed with excipient (s) or other pharmaceutically acceptable carriers. Carriers and other ingredients can be deemed pharmaceutically acceptable insofar as they are compatible with other ingredients of the formulation and not deleterious to the recipient thereof.

Techniques for formulation and administration of pharmaceutical compositions can be found in Remington's Pharmaceutical Sciences, 18th Ed., Mack Publishing Co, Easton, PA, 1990. The pharmaceutical compositions of the present invention can be manufactured using any conventional method, e.g., mixing, dissolving, granulating, dragee-making, levigating, emulsifying, encapsulating, entrapping, melt-spinning, spray-drying, or lyophilizing processes. However, the optimal pharmaceutical formulation will be determined by one of skill in the art depending on the route of administration and the desired dosage. Such formulations can influence the physical state, stability, rate of *in vivo* release, and rate of *in vivo* clearance of the administered agent. Depending on the condition being treated, these pharmaceutical compositions can be formulated and administered systemically or locally.

The pharmaceutical compositions are formulated to contain suitable pharmaceutically acceptable carriers, and can optionally comprise excipients and auxiliaries that facilitate processing of the active compounds into preparations that can be used pharmaceutically. The administration modality will generally determine the nature of the carrier. For example, formulations for parenteral administration can comprise aqueous solutions of the active compounds in water-soluble form. Carriers suitable for parenteral administration can be selected from among saline, buffered saline, dextrose, water, and other physiologically compatible solutions. Preferred carriers for parenteral administration are physiologically compatible buffers such as Hank's solution, Ringer's solution, or physiologically buffered saline. For tissue or cellular administration, penetrants appropriate to the particular barrier to be permeated are used in the formulation. Such penetrants are generally known in the art. For preparations comprising proteins, the formulation can include stabilizing materials, such as polyols (e.g., sucrose) and/or surfactants (e.g., nonionic surfactants), and the like.

Alternatively, formulations for parenteral use can comprise dispersions or suspensions of the active compounds prepared as appropriate oily injection suspensions. Suitable lipophilic solvents or vehicles include fatty oils, such as sesame oil, and synthetic fatty acid esters, such as ethyl oleate or triglycerides, or liposomes. Aqueous injection suspensions can contain substances that increase,the viscosity of the suspension, such as sodium carboxymethylcellulose, sorbitol, or dextran. Optionally, the suspension also can contain suitable stabilizers or agents that increase the solubility of the compounds to allow for the preparation of highly concentrated solutions. Aqueous polymers that provide pH-sensitive solubilization and/or sustained release of the active agent also can be used as coatings or matrix structures, e.g., methacrylic polymers, such as the EUDRAGIT^{®} series available from Röhm America Inc. (Piscataway, NJ). Emulsions, e.g., oil-in-water and water-in-oil dispersions, also can be used, optionally stabilized by an emulsifying agent or dispersant (surface active materials; surfactants). Suspensions can contain suspending agents such as ethoxylated isostearyl alcohols, polyoxy-ethlyene sorbitol and sorbitan esters, micro-crystalline cellulose, aluminum metahydroxide, bentonite, agar-agar, gum tragacanth, and mixtures thereof.

Liposomes containing the active agent also can be employed for parenteral administration. Liposomes generally are derived from phospholipids or other lipid substances. The compositions in liposome form also can contain other ingredients, such as stabilizers, preservatives, excipients, and the like. Preferred lipids include phospholipids and phosphatidyl cholines (lecithins), both natural and synthetic. Methods of forming liposomes are known in the art. See, e.g., Prescott (Ed.), Methods in Cell Biology, Vol. XIV, p. 33, Academic Press, New York (1976).

The pharmaceutical compositions comprising the agent in dosages suitable for oral administration can be formulated using pharmaceutically acceptable carriers well known in the art. The preparations formulated for oral administration can be in the form of tablets, pills, capsules, cachets, dragees, lozenges, liquids, gels, syrups, slurries, elixirs, suspensions, or powders. To illustrate, pharmaceutical preparations for oral use can be obtained by combining the active compounds with a solid excipient, optionally grinding the resulting mixture, and processing the mixture of granules, after adding suitable auxiliaries if desired, to obtain tablets or dragee cores. Oral formulations can employ liquid carriers, similar in type to those described for parenteral use, e.g., buffered aqueous solutions, suspensions, and the like.

Preferred oral formulations include tablets, dragees, and gelatin capsules. These preparations can contain one or excipients, which include, without limitation:
a) diluents, such as sugars, including lactose, dextrose, sucrose, mannitol, or sorbitol;
b) binders, such as magnesium aluminum silicate, starch from corn, wheat, rice, potato, etc.;
c) cellulose materials, such as methyl-cellulose, hydroxypropylmethyl cellulose, and sodium carboxymethylcellulose, polyvinylpyrrolidone, gums, such as gum arabic and gum tragacanth, and proteins, such as gelatin and collagen;
d) disintegrating or solubilizing agents such as cross-linked polyvinyl pyrrolidone, starches, agar, alginic acid or a salt thereof, such as sodium alginate, or effervescent compositions;
e) lubricants, such as silica, talc, stearic acid or its magnesium or calcium salt, and polyethylene glycol;
f) flavorants and sweeteners;
g) colorants or pigments, e.g., to identify the product or to characterize the quantity (dosage) of active compound; and
h) other ingredients, such as preservatives, stabilizers, swelling agents, emulsifying agents, solution promoters, salts for regulating osmotic pressure, and buffers.

Gelatin capsules include push-fit capsules made of gelatin, as well as soft, sealed-capsules made of gelatin and a coating such as glycerol or sorbitol. Push-fit capsules can contain the active ingredient(s) mixed with fillers, binders, lubricants, and/or stabilizers, etc. In soft capsules, the active compounds can be dissolved or suspended in suitable fluids, such as fatty oils, liquid paraffin, or liquid polyethylene glycol with or without stabilizers..

Dragee cores can be provided with suitable coatings such as concentrated sugar solutions, which also can contain gum arabic, talc, polyvinyl pyrrolidone, carbopol gel, polyethylene glycol, and/or titanium dioxide, lacquer solutions, and suitable organic solvents or solvent mixtures.

The pharmaceutical composition can be provided as a salt of the active agent. Salts tend to be more soluble in aqueous or other protonic solvents than the corresponding free acid or base forms. Pharmaceutically acceptable salts are well known in the art. Compounds that contain acidic moieties can form pharmaceutically acceptable salts with suitable cations. Suitable pharmaceutically acceptable cations include, for example, alkali metal (e.g., sodium or potassium) and alkaline earth (e.g., calcium or magnesium) cations.

Compounds of structural formula (I) that contain basic moieties can form pharmaceutically acceptable acid addition salts with suitable acids. For example, Berge et al. describe pharmaceutically acceptable salts in detail in J Pharm Sci, 66:1 (1977). The salts can be prepared *in situ* during the final isolation and purification of the compounds of the invention or separately by reacting a free base function with a suitable acid.

Representative acid addition salts include, acetate, adipate, alginate, citrate, aspartate, benzoate, benzenesulfonate, bisulfate, butyrate, camphorate, camphorol-sulfonate, digluconate, glycerophosphate, hemisulfate, heptanoate, hexanoate, fumarate, hydrochloride, hydrobromide, hydroiodide, 2-hydroxyethane-sulfonate (isothionate), lactate, maleate, methanesulfonate or sulfate, nicotinate, 2-naphthalenesulfonate, oxalate, pamoate, pectinate, persulfate, 3-phenylpropionate, picrate, pivalate, propionate, succinate, tartrate, thiocyanate, phosphate or hydrogen phosphate, glutamate, bicarbonate, p-toluenesulfonate, and undecanoate. Examples of acids that can be employed to form pharmaceutically acceptable acid addition salts include such inorganic acids as hydrochloric acid, hydrobromic acid, sulfuric acid, and phosphoric acid, and such organic acids as oxalic acid, maleic acid, succinic acid, and citric acid.

In light of the foregoing, any reference to compounds of the present invention appearing herein is intended to include compounds of structural formula (I)-(V), as well as pharmaceutically acceptable salts and solvates, thereof.

Basic addition salts can be prepared *in situ* during the final isolation and purification of the compounds of the invention or separately by reacting a carboxylic acid-containing moiety with a suitable base such as the hydroxide, carbonate, or bicarbonate of a pharmaceutically acceptable metal cation, or with ammonia or organic primary, secondary, or tertiary amine. Pharmaceutically acceptable basic addition salts include cations based on alkali metals or alkaline earth metals such as lithium, sodium, potassium, calcium, magnesium, and aluminum salts, and nontoxic quaternary ammonium and amine cations including ammonium, tetramethylammonium, tetraethylammonium, methylammonium, dimethylammonium, trimethylammonium, ethylammonium, diethylammonium, triethylammonium. Other representative organic amines useful for the formation of base addition salts include ethylenediamine, ethanolamine, diethanolamine, piperidine, piperazine.

Basic nitrogen-containing groups can be quaternized with such agents as lower alkyl halides such as methyl, ethyl, propyl, and butyl chlorides, bromides and iodides; dialkyl sulfates like dimethyl, diethyl, dibutyl, and diamyl sulfates; long chain alkyl halides such as decyl, lauryl, myristyl, and stearyl chlorides, bromides, and iodides; arylalkyl halides such as benzyl and phenethyl bromides; and others. Products having modified solubility or dispersibility are thereby obtained.

Compositions comprising a compound of the invention formulated in a pharmaceutical acceptable carrier can be prepared, placed in an appropriate container, and labeled for treatment of an indicated condition. Accordingly, there also is contemplated an article of manufacture, such as a container comprising a dosage form of a compound of the invention and a label containing instructions for use of the compound. Kits are also contemplated under the invention. For example, the kit can comprise a dosage form of a pharmaceutical composition and a package insert containing instructions for use of the composition in treatment of a medical condition. In either case, conditions indicated on the label can include treatment of inflammatory disorders, cancer, etc.

### Methods of Administration of Inhibitors of PI3Kδ Activity

Pharmaceutical compositions comprising an inhibitor of PI3Kδ activity can be administered to the subject by any conventional method, including parenteral and enteral techniques. Parenteral administration modalities include those in which the composition is administered by a route other than through the gastrointestinal tract, for example, intravenous, intraarterial, intraperitoneal, intramedullary, intramuscular, intraarticular, intrathecal, and intraventricular injections. Enteral administration modalities include, for example, oral (including buccal and sublingual) and rectal administration. Transepithelial administration modalities include, for example, transmucosal administration and transdermal administration. Transmucosal administration includes, for example, enteral administration as well as nasal, inhalation, and deep lung administration; vaginal administration; and rectal administration. Transdermal administration includes passive or active transdermal or transcutaneous modalities, including, for example, patches and iontophoresis devices, as well as topical application of pastes, salves, or ointments. Parenteral administration also can be accomplished using a high-pressure technique, e.g., POWDERJECT^{®}.

Surgical techniques include implantation of depot (reservoir) compositions, osmotic pumps, and the like. A preferred route of administration for treatment of inflammation can be local or topical delivery for localized disorders such as arthritis, or systemic delivery for distributed disorders, e.g., intravenous delivery for reperfusion injury or for systemic conditions such as septicemia. For other diseases, including those involving the respiratory tract, e.g., chronic obstructive pulmonary disease, asthma, and emphysema, administration can be accomplished by inhalation or deep lung administration of sprays, aerosols, powders, and the like.

For the treatment of neoplastic diseases, especially leukemias and other distributed cancers, parenteral administration is typically preferred. Formulations of the compounds to optimize them for biodistribution following parenteral administration would be desirable. The PI3Kδ inhibitor compounds can be administered before, during, or after administration of chemotherapy, radiotherapy, and/or surgery,

Moreover, the therapeutic index of the PI3Kδ inhibitor compounds can be enhanced by modifying or derivatizing the compounds for targeted delivery to cancer cells expressing a marker that identifies the cells as such. For example, the compounds can be linked to an antibody that recognizes a marker that is selective or specific for cancer cells, so that the compounds are brought into the vicinity of the cells to exert their effects locally, as previously described '(see for example, Pietersz et al., Immunol Rev, 129:57 (1992); Trail et al., Science, 261:212 (1993); and Rowlinson-Busza et al., Curr Opin Oncol, 4:1142 (1992)). Tumor-directed delivery of these compounds enhances the therapeutic benefit by, *inter alia*, minimizing potential nonspecific toxicities that can result from radiation treatment or chemotherapy. In another aspect, PI3Kδ inhibitor compounds and radioisotopes or chemotherapeutic agents can be conjugated to the same anti-tumor antibody.

For the treatment of bone resorption disorders or osteoclast-mediated disorders, the PI3Kδ inhibitors can be delivered by any suitable method. Focal administration can be desirable, such as by intraarticular injection..In some cases, it can be desirable to couple the compounds to a moiety that can target the compounds to bone. For example, a PI3Kδ inhibitor can be coupled to compounds with high affinity for hydroxyapatite, which is a major constituent of bone. This can be accomplished, for example, by adapting a tetracycline-coupling method developed for targeted delivery of estrogen to bone (Orme et al., Bioorg Med Chem Lett, 4(11):1375-80 (1994)).

To be effective therapeutically in modulating central nervous system targets, the agents used in the methods of the invention should readily penetrate the blood brain barrier when peripherally administered. Compounds that cannot penetrate the blood brain barrier, however, can still be effectively administered by an intravenous route.

As noted above, the characteristics of the agent itself and the formulation of the agent can influence the physical state, stability, rate of *in vivo* release, and rate of *in vivo* clearance of the administered agent Such pharmacokinetic and pharmacodynamic information can be collected through preclinical *in vitro* and *in vivo* studies, later confirmed in humans during the course of clinical trials. Thus, for any compound used in the method of the invention, a therapeutically effective dose can be estimated initially from biochemical and/or cell-based assays. Then, dosage can be formulated in animal models to achieve a desirable circulating concentration range that modulates PI3Kδ expression or activity. As human studies are conducted, further information will emerge regarding the appropriate dosage levels and duration of treatment for various diseases and conditions.

Toxicity and therapeutic efficacy of such compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the "therapeutic index," which typically is expressed as the ratio LD50/ED50. Compounds that exhibit large therapeutic indices, i.e., the toxic dose is substantially higher than the effective dose, are preferred. The data obtained from such cell culture assays and additional animal studies can be used in formulating a range of dosage for human use. The dosage of such compounds lies preferably within a range of circulating concentrations that include the ED₅₀ with little or no toxicity.

For the methods of the invention, any effective administration regimen regulating the timing and sequence of doses can be used. Doses of the agent preferably include pharmaceutical dosage units comprising an effective amount of the agent. As used herein, "effective amount" refers to an amount sufficient to modulate PI3Kδ expression or activity and/or derive a measurable change in a physiological parameter of the subject through administration of one or more of the pharmaceutical dosage units.

Exemplary dosage levels for a human subject are of the order of from about 0.001 milligram of active agent per kilogram body weight (mg/kg) to about 100 mg/kg. Typically, dosage units of the active agent comprise from about 0.01 mg to about 10,000 mg, preferably from about 0.1 mg to about 1,000 mg, depending upon the indication, route of administration, etc. Depending on the route of administration, a suitable dose can be calculated according to body weight, body surface area, or organ size. The final dosage regimen will be determined by the attending physician in view of good medical practice, considering various factors that modify the action of drugs, e.g., the agent's specific activity, the identity and severity of the disease state, the responsiveness of the patient, the age, condition, body weight, sex, and diet of the patient, and the severity of any infection. Additional factors that can be taken intro account include time and frequency of administration, drug combinations, reaction sensitivities, and tolerance/response to therapy. Further refinement of the dosage appropriate for treatment involving any of the formulations mentioned herein is done routinely by the skilled practitioner without undue experimentation, especially in light of the dosage information and assays disclosed, as well as the pharmacokinetic data observed in human clinical trials. Appropriate dosages can be ascertained through use of established assays for determining concentration of the agent in a body fluid or other sample together with dose response data.

The frequency of dosing will depend on the pharmacokinetic parameters of the agent and the route of administration. Dosage and administration are adjusted to provide sufficient levels of the active moiety or to maintain the desired effect. Accordingly, the pharmaceutical compositions can be administered in a single dose, multiple discrete doses, continuous infusion, sustained release depots, or combinations thereof, as required to maintain desired minimum level of the agent. Short-acting pharmaceutical compositions (i.e., short half-life) can be administered once a day or more than once a day (e.g., two, three, or four times a day). Long acting pharmaceutical compositions might be administered every 3 to 4 days, every week, or once every two weeks. Pumps, such as subcutaneous, intraperitoneal, or subdural pumps, can be preferred for continuous infusion.

The following Examples are provided to further aid in understanding the invention, and presuppose an understanding of conventional methods well-known to those persons having ordinary skill in the art to which the examples pertain, e.g., the construction of vectors and plasmids, the insertion of genes encoding polypeptides into such vectors and plasmids, or the introduction of vectors and plasmids into host cells. Such methods are described in detail in numerous publications including, for example, Sambrook et al., Molecular Cloning: A Laboratory Manual, Cold Spring Harbor Laboratory Press (1989), Ausubel et al. (Eds.), Current Protocols in Molecular Biology, John Wiley & Sons, Inc. (1994); and Ausubel et al. (Eds.), Short Protocols in Molecular Biology, 4th ed., John Wiley & Sons, Inc. (1999). The particular materials and conditions described hereunder are intended to exemplify particular aspects of the invention.

### EXAMPLE 1

### Preparation and Purification of Recombinant PI3Kα, β, and δ

Recombinant PI3K heterodimeric complexes consisting of a p110 catalytic subunit and a p85 regulatory subunit were overexpressed using the BAC-TO-BAC^{®} HT baculovirus expression system (GIBCO/- BRL), and then purified for use in biochemical assays. The four Class I PI 3-kinases were cloned into baculovirus vectors as follows:
p110δ: A FLAG^{®}-tagged version of human p110δ (SEQ ID NO:1) (see Chantry et al., J Biol Chem, 272:19236-41 (1997)) was subcloned using standard recombinant DNA techniques into the *Bam*H1-*Xba*1 site of the insect cell expression vector pFastbac HTb (Life Technologies, Gaithersburg, MD), such that the clone was in frame with the His tag of the vector. The FLAG^{®} system is described in U.S. Patent Nos. 4,703,004; 4,782,137; 4,851,341; and 5,011,912, and reagents are available from Eastman Kodak Co.
p110α: Similar to the method used for p110δ, described above, a FLAG^{®}-tagged version of p110α (see Volinia et al., Genomics, 24 (3) :427- 477 (1994)) was subcloned in *Bam*H1-*Hind*III sites of pFastbac HTb (Life Technologies) such that the clone was in frame with the His tag of the vector.
p110β: A p110β (see Hu et al., Mol Cell Biol, 13:7677-88 (1993) ) clone was amplified from the human MARATHON^{®} Ready spleen cDNA library (Clontech, Palo Alto CA) according to the manufacturer's protocol using the following primers:
   **5' Primer** 
5'-
GATCGAATTCGGCGCCACCATGGACTACAAGGACGACGATGACAAGTGCTTC
AGTTTCATAATGCCTCC-3' (SEQ ID NO:3)
   **3' Primer**
   5'-GATCGCGGCCGCTTAAGATCTGTAGTCTTTCCGAACTGTGTG-3' (SEQ ID NO:4)
   The 5' primer was built to contain a FLAG^{®} tag in frame with the p110β sequence. After amplification, the FLAG^{®}-p110β sequence was subcloned using standard recombinant techniques into the *Eco*R1-*Not*1 sites of pFastbac HTa (Life Technologies), such that the clone was in frame with the His tag of the vector.
p110γ: The p110γ cDNA (see Stoyanov et al., Science, 269:690-93 (1995)) was amplified from a human Marathon Ready spleen cDNA library (Clontech) according to the manufacturer's protocol using the following primers:
   **5' Primer**
      5'-AGAATGCGGCCGCATGGAGCTGGAGAACTATAAACAGCCC-3' (SEQ ID NO:5)
   **3' Primer**
      5'-CGCGGATCCTTAGGCTGAATGTTTCTCTCCTTGTTTG-3' (SEQ ID NO:6)
   A FLAG^{®} tag was subsequently attached to the 5' end of the p110γ sequence and was cloned in the *Bam*H1-*Spe*1 sites of pFastbac HTb (Life Technologies) using standard recombinant DNA techniques, with the FLAG^{®}-110γ sequence in-frame with the His tag of the vector.
p85α: A *Bam*H1-*Eco*R1 fragment of FLAG^{®}-tagged p85 cDNA (see Skolnik et al., Cell, 65:83-89 (1991)) was subcloned into the *Bam*H1-*Eco*R1 sites of the vector pFastbac dual (Life Technologies).

Recombinant baculoviruses containing the above clones were generated using manufacturer's recommended protocol (Life Technologies). Baculoviruses expressing His-tagged p110α, p110β, or p110δ catalytic subunit and p85 subunit were coinfected into Sf21 insect cells. To enrich the heterodimeric enzyme complex, an excess amount of baculovirus expressing p85 subunit was infected, and the His-tagged p110 catalytic subunit complexed with p85 was purified on nickel affinity column. Since p110γ does not associate with p85, Sf21 cells were infected with recombinant baculoviruses expressing His-tagged p110γ only. In an alternate approach, p101 can be cloned into baculovirus, to permit coexpression with its preferred binding partner p110γ.

The 72-hour post-infected Sf21 cells (3 liters) were harvested and homogenized in a hypotonic buffer (20 mM HEPES-KOH, pH 7.8, 5 mM KCl, complete protease inhibitor cocktail (Roche Biochemicals, Indianapolis, IN), using a Dounce homogenizer. The homogenates were centrifuged at 1,000 x g for 15 min. The supernatants were further centrifuged at 10,000 x g for 20 min, followed by ultracentrifugation at 100,000 x g for 60 min. The soluble fraction was immediately loaded onto 10 mL of HITRAP^{®} nickel affinity column (Pharmacia, Piscataway, NJ) equilibrated with 50 mL of Buffer A (50 mM HEPES-KOH, pH 7.8, 0.5 M NaCl, 10 mM imidazole). The column was washed extensively with Buffer A, and eluted with a linear gradient of 10-500 mM imidazole. Free p85 subunit was removed from the column during the washing step and only the heterodimeric enzyme complex eluted at 250 mM imidazole. Aliquots of nickel fractions were analyzed by 10% SDS-polyacrylamide gel electrophoresis (SDS-PAGE), stained with SYPRO^{®} Red (Molecular Probes, Inc., Eugene, OR), and quantitated with STORM^{®} PhosphoImager (Molecular Dynamics, Sunnyvale, CA). The active fractions were pooled and directly loaded onto a 5 mL Hi-trap heparin column preequilibrated with Buffer B containing 50 mM HEPES-KOH, pH 7.5, 50 mM NaCl, 2 mM dithiothreitol (DTT). The column was washed with 50 mL of Buffer B and eluted with a linear gradient of 0.05-2 M NaCl. A single peak containing PI3K enzyme complex eluted at 0.8 M NaCl. SDS-polyacrylamide gel analysis showed that the purified PI3K enzyme fractions contained a 1:1 stoichiometric complex of p110 and p85 subunits. The protein profile of the enzyme complex during heparin chromatography corresponded to that of lipid kinase activity. The active fractions were pooled and frozen under liquid nitrogen.

### EXAMPLE 2

### PI3Kδ High Throughput Screen (HTS) and Selectivity Assay

A high throughput screen of a proprietary chemical library was performed to identify candidate inhibitors of PI3Kδ activity. PI3Kδ catalyzes a phosphotransfer from γ-[³²P]ATP to PIP₂/PS liposomes at the D3' position of the PIP₂ lipid inositol ring. This reaction is MgCl₂ dependent and is quenched in high molarity potassium phosphate buffer pH 8.0 containing 30 mM EDTA. In the screen, this reaction is performed in the presence or absence of library compounds. The reaction products (and all unlabelled products) are transferred to a 96-well, prewetted PVDF filter plate, filtered, and washed in high molarity potassium phosphate. Scintillant is added to the dried wells and the incorporated radioactivity is quantitated.

The majority of assay operations were performed using a BIOMEK^{®} 1000 robotics workstations (Beckman) and all plates were read using Wallac liquid scintillation plate counter protocols.

The 3X assay stocks of substrate and enzyme were made and stored in a trough (for robotics assays) or a 96-well, V-bottom, polypropylene plate (for manual assays). Reagents were stable for at least 3 hours at room temperature.

The 3X substrate for the HTS contained 0.6 mM Na₂ATP, 0.10 mCi/mL γ-[³²P]ATP (NEN, Pittsburgh, PA), 6 µM PIP₂/PS liposomes (Avanti Polar Lipids, Inc., Atlanta, GA), in 20 mM HEPES, pH 7.4.

The 3X enzyme stock for the HTS contained 1.8 nM PI3Kδ, 150 µg/mL horse IgG (used only as a stabilizer), 15 mM MgCl₂, 3 mM DTT in 20 mM HEPES, pH 7.4.

The chemical high throughput screen (HTS) library samples (each containing a pool of 22 compounds) in dimethyl sulfoxide (DMSO) were diluted to 18.75 µM or 37.8 µM in double distilled water, and 20 µL of the dilutions were placed in the wells of a 96-well polypropylene plate for assaying. The negative inhibitor control (or positive enzyme control) was DMSO diluted in water, and the positive inhibitor controls employed concentrations of LY294002 sufficient to provide 50% and 100% inhibition.

To the 20 µL pooled chemical library dilutions, 20 µL of 3X substrate was added. The reaction was initiated with 20 µL of 3X enzyme, incubated at room temperature for 10 minutes: This dilution established a final concentration of 200 µM ATP in the reaction volume. The reaction was stopped with 150 µL quench buffer (1.0 M potassium phosphate pH 8.0, 30 mM EDTA). A portion of the quenched solution (180 µL) was then transferred to a PVDF filter plate (Millipore #MAIP NOB prewetted with sequential 200 µL washes of 100% methanol, water, and finally 1.0 M potassium phosphate pH 8.0 wash buffer).

The PVDF filter plate was aspirated under moderate vacuum (2-5 mm Hg), washed with 5 x 200 µL of wash buffer, and then dried by aspiration. The filter was subsequently blotted, allowed to air dry completely, and inserted into a Wallac counting cassette with 50 µL of Ecoscint scintillation cocktail added per well. The incorporated radioactivity was quantitated, and data were analyzed, after normalizing to the enzyme positive control (set at 100%), to identify the curve intersection at the 50% inhibition value to estimate IC₅₀ values for the inhibitors.

A total of 57 pooled master wells were selected for deconvolution, based on combined criteria of <42% residual activity at the tested concentration, and a total accepted hit rate of no more than 0.2%. At 22 compounds per well, a total of 1254 compounds were identified through this deconvolution and individually assayed at the 1X concentration of 27.7 µM to identify which compounds exhibited the desired activity. From these assays, 73 compounds were selected and assayed further to develop IC₅₀ curves. From the IC₅₀ curve results, 34 compounds were selected for selectivity assays against PI3Kα and PI3Kβ (see assay protocol in Example 11).
From the selectivity assays, one comparative compound, 3-(2-chlorophenyl)-2-(9H-purin-6-ylsulfanylmethyl)-3H-quinazolin-4-one (Compound D-000), was selected as being a relatively potent and selective compound. Catalog searches and selectivity assays of many analogous compounds of the potent and/or selective hits yielded only one compound that was both an active and selective analogue of D-000. This compound was purchased from Contract Services Corporation (Catalog #7232154) and differed from D-000 in substituting a phenyl group for the 2-chlorophenyl group of D-000.

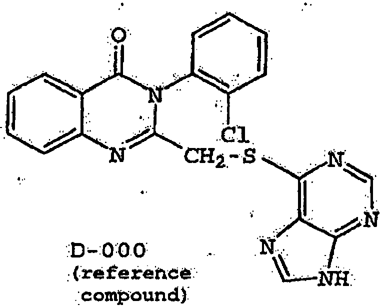

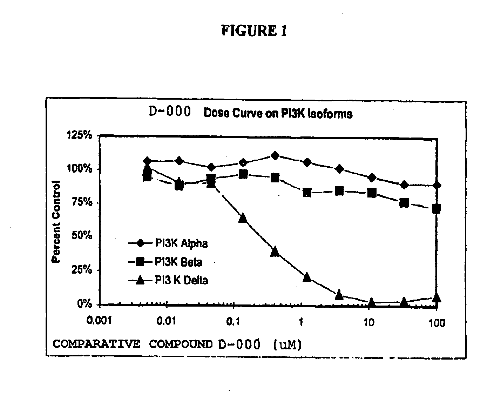

As described above, the PI 3-kinase inhibitor LY294002 (Calbiochem, La Jolla, CA) does not have significant selectivity among the different PI 3-kinase isoforms tested. Under our assay conditions, LY294002 inhibited all three isoforms of PI 3-kinases with an IC₅₀ of 0.3 to 1 µM. However, when the compound D-000 was tested against the same PI 3-kinase isoforms distinct selectivity was observed. Specifically, as shown in Figure 1, D-000 inhibited the activity of the δ isoform of PI3K with an IC₅₀ of approximately 0.3 µM, whereas under similar conditions it did not inhibit activities of the α and β isoforms at a limit of 100 µM compound. These results show that D-000 selectively inhibits PI3Kδ activity.

### EXAMPLES 3-7

Since PI3Kδ is expressed at significant levels only in leukocytes, it is important to study the effects of the PI3Kδ-selective inhibitor on leukocyte functions. Accordingly, the effects of PI3Kδ inhibition in several types of leukocytes were examined. Neutrophils were examined to determine the effects that selective inhibition of PI3Kδ might elicit (Example 3, below). It surprisingly was found that selective inhibition of PI3Kδ activity appears to be significantly associated with inhibition of some but not all functions characteristic of activated neutrophils. In addition, the effects of PI3Kδ inhibition on B cell and T cell function also were tested (Examples 4-5, below). Moreover, as PI3Kδ also is expressed in osteoclasts, the effect of PI3Kδ inhibition on the function of these specialized cells was studied (Example 6, below).

### EXAMPLE 3

### Characterization of Role of PI3Kδ in Neutrophil Function

The effects of a PI3Kδ inhibitor of the disclosure, i.e., D-000, on neutrophil functions such as superoxide generation, elastase exocytosis, chemotaxis, and bacterial killing were tested.

### A. Preparation of neutrophils from human blood

Aliquots (8 mL) of heparinized blood from healthy volunteers were layered on 3 mL cushions of 7.3% FICOLL^{®} (Sigma, St. Louis, MO) and 15.4% HYPAQUE^{®} (Sigma) and centrifuged at 900 rpm for 30 min at room temperature in a table top centrifuge (Beckman). The neutrophil-rich band just above the FICOLL^{®}-HYPAQUE^{®} cushion was collected and washed with Hanks' balanced salt solution (HBSS) containing 0.1% gelatin. Residual erythrocytes were removed by hypotonic lysis with 0.2% NaCl. The neutrophil preparation was washed twice with HBSS containing 0.1% gelatin and used immediately.

### B. Measurement of superoxide production from neutrophils

Superoxide generation is one of the hall-marks of neutrophil activation. A variety of activators potentiate superoxide generation by neutrophils. The effect of the PI3Kδ inhibitor D-000 on superoxide generation by three different agonists: TNF1α, IgG, and fMLP, each representing separate classes of activator, was measured. Superoxide generated by the neutrophils was measured by monitoring the change in absorbance upon reduction of cytochrome C by modification of the method described by Green et al., (pp. 14.5.1-14.5.11 in Supp. 12, Curr Protocols Immunol (Eds., Colligan et al.) (1994)), as follows. Individual wells of a 96-well plate were coated overnight at 4°C with 50 µL of 2 mg/mL solution of human fibrinogen or IgG. The wells were washed with PBS and the following reagents were added to each well: 50 µL of HBSS or Superoxide dismutase (1 mg/mL), 50 µL of HBSS or TNF1α (50 ng/mL), 50 µL cytochrome C (2.7 mg/mL), and 100 µL of purified human neutrophil suspension (2 x 10⁶ cells/mL). The plate was centrifuged for 2 min at 200 rpm and absorbance at 550 nm was monitored for 2 hr. To measure the relative amounts of superoxide generated, values obtained from the superoxide dismutase-containing wells were subtracted from all, and normalized to the values obtained from the wells without any inhibitor.

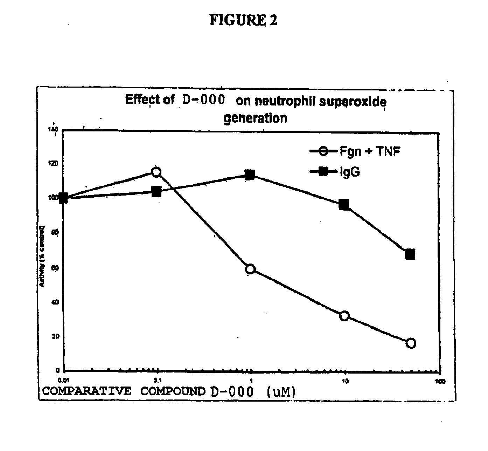

As shown in Figure 2, the PI3Kδ inhibitor D~000 inhibits TNF-induced superoxide generation by neutrophils in a concentration dependent manner. Superoxide generation induced by TNF was reduced to its half-maximal value at about 3 µM D-000. Figure 2 also reveals that Superoxide generation induced by IgG was not significantly inhibited by D-000. In fact, even at 10 µM this PI3Kδ inhibitor did not have any effect on superoxide generation induced by IgG.

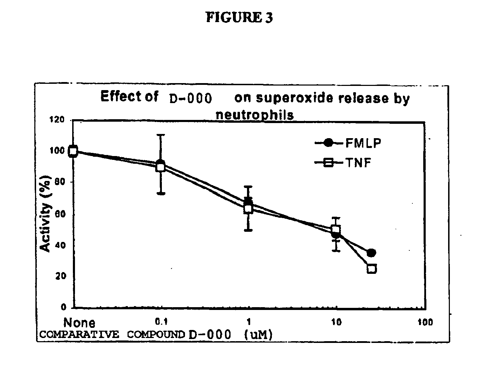

Next, the effect of D-000 on superoxide generation induced by another potent inducer, the bacterial peptide, formylated-Met-Leu-Phe (fMLP) was studied. Like the TNF-induced superoxide generation, fMLP-induced superoxide generation also was inhibited by D-000 (Figure 3). These results show that the PI3Kδ inhibitor D-000 can prevent stimulus specific induction of superoxide generation by neutrophils, indicating that PI3Kδ is involved in this process.

### C. Measurement of elastase exocytosis from neutrophils

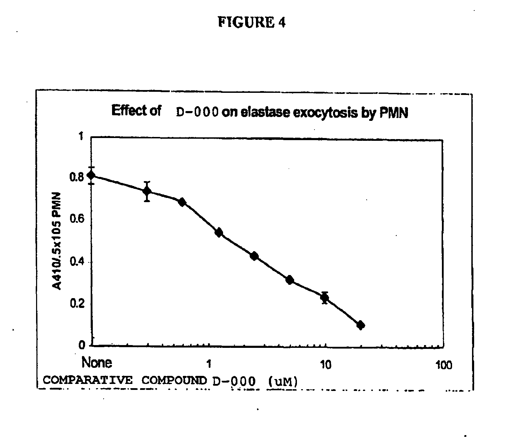

In addition to superoxide generation, activated neutrophils also respond by releasing several proteases that are responsible for the destruction of tissues and cartilage during inflammation. As an indication of protease release, the effect of D-000 on elastase exocytosis was measured. Elastase exocytosis was quantitated by modification of the procedure described by Ossanna et al. (J Clin Invest, 77:1939-1951 (1986)), as follows. Purified human neutrophils (0.2 x 10⁶) (treated with either DMSO or a serial dilution of D-000 in DMSO) were stimulated with fMLP in PBS containing 0.01 mg/mL cytochalasin B, 1.0 µM sodium azide (NaN₃), 5 µg/mL L-methionine and 1 µM fMLP for 90 min at 37°C in a 96-well plate. At the end of the incubation period, the plate was centrifuged for 5 min at 1000 rpm, and 90 µL of the supernatant was transferred to 10 µL of 10 mM solution of an elastase substrate peptide, MeO-suc-Ala-Ala-Pro-Val-pNA, wherein MeO-suc = methoxy-succinyl; pNA = p-nitroanilide (Calbiochem, San Diego, CA). Absorbance at 410 nm was monitored for 2 hr in a 96-well plate reader. To measure the relative amounts of elastase excytosed, all adsorbance values were normalized to the values without any inhibitor. As shown in Figure 4, the PI3Kδ inhibitor D-000 inhibits fMLP-induced elastase exocytosis significantly, and does so in a dose-dependent fashion. Inhibition was half-maximal at a concentration of about 2-3 µM D-000.

### D. Measurement of fMLP-induced human neutrophil migration

Neutrophils have the intrinsic capacity to migrate through tissues, and are one of the first cell types to arrive at the sites of inflammation or tissue injury. The effect of D-000 on neutrophil migration towards a concentration gradient of fMLP was measured. The day before the migration assays were performed, 6-well plates were coated with recombinant ICAM-1/Fc fusion protein (Van der Vieren et al., Immunity, 3:683-690 (1995)) (25 µg/mL in bicarbonate buffer, pH 9.3) and left overnight at 4°C. After washing, 1% agarose solution, in RPMI-1640 with 0.5% bovine serum albumin (BSA), was added to wells with or without an inhibitor, and plates were placed into a refrigerator before punching holes in the gelled agarose to create plaques (1 central hole surrounded by'6 peripheral ones per well).

Human neutrophils were obtained as described above, and resuspended in RPMI medium supplemented with 0.5% BSA at 5 x 10⁶ cells/mL. After combining equal volumes of neutrophil suspension and medium (either with DMSO or a serial dilution of the test compound in DMSO), neutrophils were aliquoted into the peripheral holes, while the central hole received fMLP (5 µM). Plates were incubated at 37°C in the presence of 5% CO₂ for 4 hr, followed by termination of migration by the addition of 1% glutaraldehyde solution in D-PBS. After removing the agarose layer, wells were washed with distilled water and dried.

Analysis of neutrophil migration was conducted on a Nikon DIAPHOT^{®} inverted microscope (1x objective) video workstation using the NIH 1.61 program. Using Microsoft Excel and Table Curve 4 (SSPS Inc., Chicago IL) programs, a migration index was obtained for each of the studied conditions. Migration index was defined as the area under a curve representing number of migrated neutrophils versus the net distance of migration per cell.

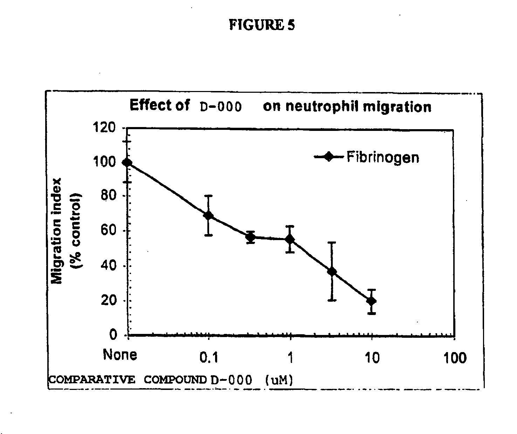

As shown in Figure 5, the PI3Kδ inhibitor D-000 had a profound effect on neutrophil migration, inhibiting this activity in a dose-dependent manner. The EC₅₀ of this compound for inhibition of neutrophil migration in this assay is about 1 µM. Based on a visual inspection of the recorded paths of the cells in this assay, it appears that the total path length for the neutrophils was not significantly affected by the test compound. Rather, the compound affected neutrophil orientation or sense of direction, such that instead of migrating along the axis of the chemoattractant gradient, the cells migrated in an undirected or less directed manner.

### E. Measurement of bactericidal capacity of neutrophils

Given that the PI3Kδ inhibitor D-000 affects certain neutrophil functions detailed above, it was of interest to see whether the compound affects neutrophil-mediated bacterial killing. The effect of D-000 on neutrophil-mediated *Staphylococcus aureus* killing was studied according to the method described by Clark and Nauseef (pp. 7.23.4-7.23.6 in Vol. 2, Supp. 6, Curr Protocols Immunol (Eds., Colligan et al.) (1994)). Purified human neutrophils (5 x 10⁶ cells/mL) (treated with either DMSO or a serial dilution of D-000 in DMSO) were mixed with autologous serum. Overnight-grown *S. aureus* cells were washed, resuspended in HBSS, and added to the serum-opsonized neutrophils at a 10:1 ratio. Neutrophils were allowed to internalize the bacteria by phagocytosis by incubation at 37°C for 20 min. The noninternalized bacteria were killed by 10 units/mL lysostaphin at 37°C for 5 min and the total mixture was rotated at 37°C. Samples were withdrawn at various times for up to 90 min and the neutrophils were lysed by dilution in water. Viable bacteria were counted by plating appropriate dilutions on trypticase-soy-agar plate and counting the *S. aureus* colonies after overnight growth.

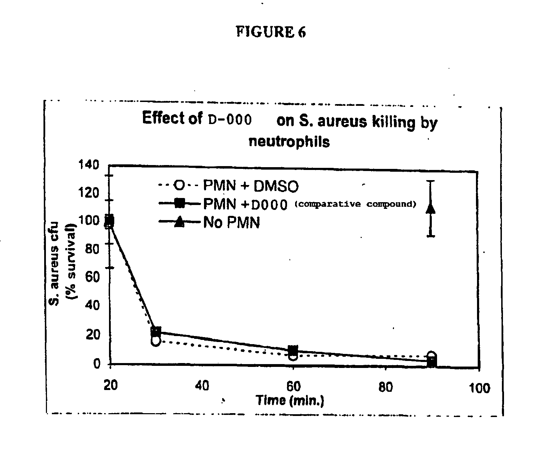

As shown in Figure 6, neutrophil-mediated killing of *S. aureus* was similar in samples treated with DMSO (control) and with D-000. These results indicate that the PI3Kδ inhibitor does not significantly affect the ability of neutrophils to kill *S. aureus,* suggesting that PI3Kδ is not involved in this pathway of neutrophil function.

### EXAMPLE 4

### Characterization of Role of PI3Kδ in B Lymphocyte Function

The effects of the PI 3-kinase inhibitor on B cell functions including classical indices such as antibody production and specific stimulus-induced proliferation also were studied.

### A. Preparation and stimulation of B cells from peripheral human blood

Heparinized blood (200 mL) from healthy volunteers was mixed with an equal volume of D-PBS, layered on 10 x 10 mL FICOLL-PAQUE^{®} (Pharmacia), and centrifuged at 1600 rpm for 30 min at room temperature. Peripheral blood mononuclear cells (PBMC) were collected from the FICOLL^{®}/serum interface, overlayed on 10 mL fetal bovine serum (FBS) and centrifuged at 800 rpm for 10 min to remove platelets. After washing, cells were incubated with DYNAL^{®} Antibody Mix (B cell kit) (Dynal Corp., Lake Success, NY) for 20 min at 4-8°C. Following the removal of unbound antibody, PBL were mixed with anti-mouse IgG coated magnetic beads (Dynal) for 20 min at 4-8°C with gentle shaking followed by elimination of labeled non-B cells on the magnetic bead separator. This procedure was repeated once more. The B cells were resuspended in RPMI-1640 with 10% FBS, and kept on ice until further use.

### B. Measurement of antibody production by human B cells

To study antibody production, B cells were aliquoted at 50-75 x 10³ cells/well into 96-well plate with or without inhibitor, to which IL-2 (100 U/mL) and PANSORBIN^{®} (Calbiochem) *Staphylococcus* aureus cells (1:90,000) were added. Part of the media was removed after 24-36 hr, and fresh media (with or without inhibitor) and IL-2 were added. Cultures were incubated at 37°C, in the presence of a CO₂ incubator for additional 7 days. Samples from each condition (in triplicate) were removed, and analyzed for IgG and IgM, as measured by ELISA. Briefly, IMMULON^{®} 4 96-well plates were coated (50 µL/well) with either 150 ng/mL donkey antihuman IgG (H+L) (Jackson ImmunoResearch, West Grove PA), or 2 µg/mL donkey antihuman IgG+IgM (H+L) (Jackson ImmunoResearch) in bicarbonate buffer, and left overnight at 4°C. After 3x washing with phosphate buffered saline containing 0.1% TWEEN^{®}-80 (PBST) (350 µL/well), and blocking with 3% goat serum in PBST (100 µL/well) for 1 hr at room temperature, samples (100 µL/well) of B cell spent media diluted in PBST were added. For IgG plates the dilution range was 1:500 to 1:10000, and for IgM 7.:50 to 1:1000. After 1 hr, plates were exposed to biotin-conjugated antihuman IgG (100 ng/mL) or antihuman IgM (200 ng/mL) (Jackson ImmunoResearch) for 30 min, following by streptavidin-HRP (1:20000) for 30 min, and finally, to TMB solution (1:100) with H₂O₂ (1:10000) for 5 min, with 3 x PBST washing between steps. Color development was stopped by H₂SO₄ solution, and plates were read on an ELISA plate reader.

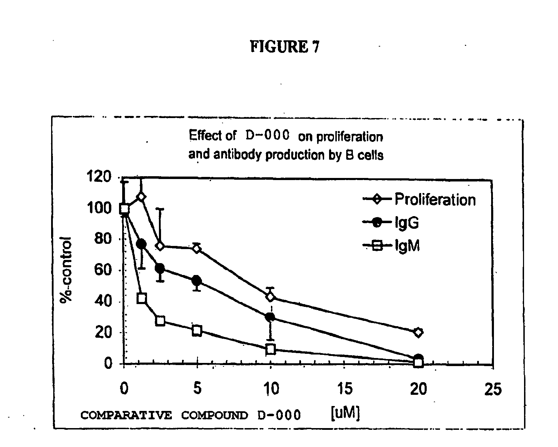

As shown in Figure 7, D-000 significantly inhibited antibody production. IgM production was affected more than IgG production: half-maximal inhibition of IgM production was observed at about 1 µM, versus about 7µM for comparable inhibition of IgG production.

### C. Measurement of B Cell Proliferation in response to cell surface IgM stimulation

In the above experiment, the B cells were stimulated using PANSORBIN^{®}. The effect of D-000 on B cell proliferation response when they were stimulated through their cell surface IgM using anti-IgM antibody also was measured. Murine splenocytes (Balb/c) were plated into 96-well microtiter plates at 2 x 10⁵ cells per well in 10% FBS/RPMI. Appropriate dilutions of test inhibitor in complete medium were added to the cells and the plates were incubated for 30-60 minutes prior to the addition of stimulus. Following the preincubation with test inhibitor an F(ab')₂ preparation of goat antibody specific for the µ-chain of mouse IgM was added to the wells at a final concentration of 25 µg/mL. The plates were incubated at 37°C for 3 days and 1 µCi of [³H]-thymidine was added to each well for the final four hours of culture. The plates were harvested onto fiber filters washed and the incorporation of radiolabel was determined using a beta counter (Matrix 96, Packard Instrument Co., Downers Grove, IL) and expressed as counts per minute (CPM).

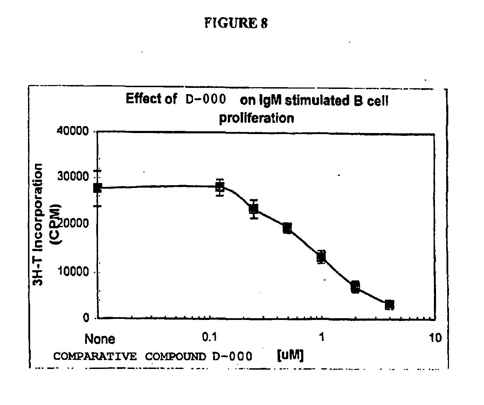

Figure 8 shows the effect of D-000 on anti-IgM stimulated proliferation of B cells. The compound inhibited anti-IgM-stimulated B cell proliferation in a dose-dependent manner. At about 1 µM, proliferation was reduced to its half-maximal value.

Because the compound D-000 inhibits B cell proliferation, it is envisioned that this compound and other PI3Kδ inhibitors could be used to suppress undesirable proliferation of B cells in clinical' settings. For example, in B cell malignancy, B cells of various stages of differentiation show unregulated proliferation. Based on the results shown above, one can infer that PI3Kδ selective inhibitors could be used to control, limit, or inhibit growth of such cells.

### EXAMPLE 5

### Characterization of Role of PI3Kδ in T Lymphocyte Function

T cell proliferation in response to costimulation of CD3+CD28 was measured. T cells were purified from healthy human blood by negative selection using antibody coated magnetic beads according to the manufacturer's protocol (Dynal) and resuspended in RPMI. The cells were treated with either DMSO or a serial dilution of D-000 in DMSO and plated at 1 x 10⁵ cells/well on a 96-well plate precoated with goat antimouse IgG. Mouse monoclonal anti-CD3 and anti-CD28 antibodies were then added to each well at 0.2 ng/mL and 0.2 µg/mL, respectively. The plate was incubated at 37°C for 24 hr and [³H]-thymidine (1 µCi/well) was added. After another 18 hr incubation the cells were harvested with an automatic cell harvester, washed and the incorporated radioactivity was quantified.

Although the PI3Kδ inhibitor D-000 inhibited anti-CD3- and anti-CD28-induced proliferation of T cells, its effect is not as strong as its effect on B cells or on some of the functions of neutrophils. Half-maximal.inhibition of thymidine incorporation was not achieved at the highest tested concentration, i.e., 10 µM D-000.

### EXAMPLE 6

### Characterization of Role of PI3Kδ in Osteoclast Function

To analyze the effect of the PI3Kδ inhibitor D-000 on osteoclasts, mouse bone marrow cells were isolated and differentiated them to osteoclasts by treating the cells with Macrophage Colony Stimulating Factor⁻¹ (mCSF⁻¹) and Osteoprotegerin Ligand (OPGL) in serum-containing medium (αMEM with 10% heat-inactivated FBS; Sigma) for 3 days. On day four, when the osteoclasts had developed, the medium was removed and cells were harvested. The osteoclasts were plated on dentine slices at 10⁵ cells/-well in growth medium, i.e., αMEM containing 1% serum and 2% BSA with 55 µg/mL OPGL and 10 ng/mL mCSF-1. After 3 hr, the medium was changed to 1% serum and 1% BSA, with or without osteopontin (25 µg/mL) and the PI3K inhibitors (100 nM). The medium was changed every 24 hours with fresh osteopontin and the inhibitors. At 72 hr, the medium was removed, and the dentine surfaces were washed with water to remove cell debris and stained with acid hematoxylin. Excess stain was washed and the pit depths were quantitated using confocal microscopy.

As shown in Table 1, in two experiments, the PI 3-kinase inhibitors had an inhibitory effect on osteoclast function. Both the nonspecific inhibitors LY294002 and wortmannin inhibited osteoclast activity. However, the PI3Kδ inhibitor D-000 had the most profound effect, as at 100 nM this compound almost completely inhibited the osteoclast activity.

| **Table 1** | | | |
|---|---|---|---|
| **Osteopontin (OPN)** | **D-000 + OPN** | **LY294002 + OPN** | **Wortmannin + OPN** |
| 10±0.5 | 1 | 4.6±0.22 | 5.7±0.6 |
| 9±0.4 | 1 | 5.8±0.5 | 5±0.5 |

### EXAMPLE 7

### Characterization of Role of PI3Kδ in Basophil Function

Assessment of the effect of a compound of the invention on basophil function was tested using a conventional histamine release assay, generally in accordance with the method described in Miura et al., J Immunol, 162:4198-206 (1999). Briefly, enriched basophils were preincubated with test compounds at several concentrations from 0.1 nM to 1,000 nM, for 10 min at 37°C. Then, polyclonal goat antihuman IgE (0.1 µg/mL) or fMLP was added, and allowed to incubate for an additional 30 min. Histamine released into the supernatant was measured using an automated fluorometric technique. Two reference compounds were tested, shown below. 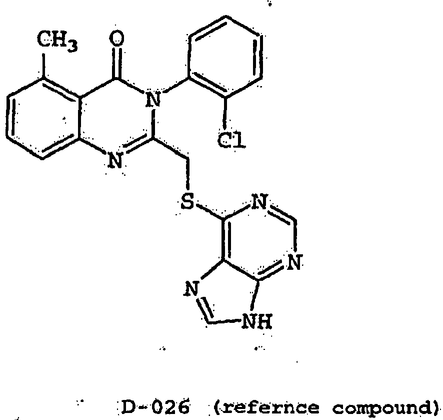 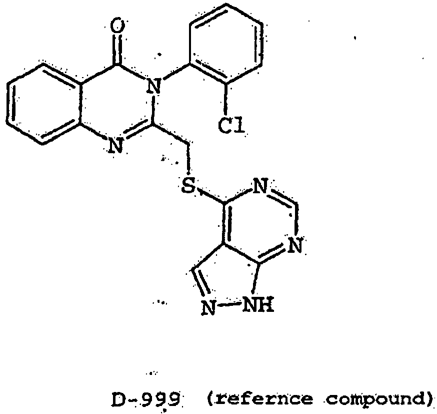 A dose-dependent decrease in histamine release was observed for 3-(2-chlorophenyl)-5-methyl-2-(9H-purin-6-ylsulfanylmethyl)-3H-quinazolin-4-one (D-026) when the basophils were stimulated with anti-IgE. This suppression of histamine release was essentially 100% at 1,000 nM, with an EC₅₀ of about 25 nM. Another reference compound, 3-(2-chlorophenyl)-2-(1H-pyrazolo[3,4-d]pyrimidin-4-ylsulfanylmethyl)-3H-quinazolin-4-one (D-999), in which the purine ring structure is rearranged, was less efficacious in the inhibition of histamine release. Neither compound elicited any effect when the basophils were stimulated with fMLP. For comparison, the nonselective PI3K inhibitor LY294002 was tested at 0.1 nM and 10,000 nM, showing close to 100% inhibition of histamine release at the highest concentration.

These data indicate that inhibitors of PI 3-kinase delta activity can be used to suppress release of histamine, which is one of the mediators of allergy. Since the activity of various PI 3-kinases are required for protein trafficking, secretion, and exocytosis in many cell types, the above data suggest that histamine release by other cells, such as mast cells, also can be disrupted by PI 3-kinase delta-selective inhibitors.

### CHEMICAL SYNTHESIS EXAMPLES

Specific nonlimiting examples of compounds are provided below. It is under-stood in the art that protecting groups can be employed where necessary in accordance with general principles of synthetic chemistry. These protecting groups are removed in the final steps of the synthesis under basic, acidic, or hydrogenolytic conditions readily apparent to those persons skilled in the art. By employing appropriate manipulation and protection of any chemical functionalities, synthesis of compounds of structural formula (I) not specifically set forth herein can be accomplished by methods analogous to the schemes set forth below.

Unless otherwise noted, all starting materials were obtained from commercial suppliers and used without further purification. All reactions and chromatography fractions were analyzed by thin-layer chromatography (TLC) on 250 mm silica gel plates, visualized with ultraviolet (UV) light or iodine (I₂) stain. Products and intermediates were purified by flash chromatography or reverse-phase high performance liquid chromatography.

The following abbreviations are used in the synthetic examples: aq (aqueous), H₂O (water), CHCl₃ (chloroform), HCl (hydrochloric acid), MeOH (methanol), NaOH (sodium hydroxide), NaOMe (sodium methoxide), TFA (trifluoroacetic acid), K₂CO₃ (potassium carbonate), SOCl₂ (thionyl chloride), CH₂Cl₂ (methylene chloride), EtOAC (ethyl acetate), DMF (dimethylformamide), EtOH (ethanol), DMSO (dimethyl sulfoxide), NaHCO₃ (sodium bicarbonate), TLC (thin layer chromatography), HPLC (high performance liquid chromatography), HOBT (hydroxybenzotriazole), EDC (ethyldiethylaminopropylcarbodiimide), DIEA (diisopropylethylamine), and HOAc (acetic acid).

### I. General Procedures

### Procedure A

Thionyl chloride was added to a rapidly stirring solution of anthranilic acid or benzoic acid in benzene, and the mixture was stirred at reflux for 5 to 18 hours. The reaction was concentrated *in vacuo,* and stripped down twice with benzene. The resulting oil was dissolved in CHCl₃ and to that solution was added the appropriate aniline. The reaction mixture was heated to reflux and stirred until complete, as determined by TLC, at which point the reaction mixture was cooled to ambient temperature. The precipitate was removed by filtration, and the filtrate concentrated *in vacuo.* The crude product was purified by chromatography and/or recrystallization from MeOH to provide amides 1a-1r.

### Procedure B

To a rapidly stirring suspension of an amide in glacial acetic acid was added chloroacetyl chloride. The reaction mixture was heated to 120°C, and allowed to stir at that temperature until complete, as determined by TLC. After brief cooling, the reaction mixture was concentrated *in vacuo.* The crude residue was purified by extraction, chromatography, and/or recrystallization to provide chlorides 2a-2r.

### Procedure C

A mixture of a chloride, either a nitrogen or a sulfur nucleophile, for example, mercaptopurine monohydrate or adenine, and K₂CO₃ in DMF was stirred at room temperature for 15-72 hours. The resulting suspension was poured into water, and kept at 4°C for several hours. The crude solid.was filtered, washed with water, and purified by chromatography or recrystallization to provide.the final products:

### EXAMPLE 8

### Preparation of Intermediate Compounds: Amides

### 2-Amino-N-(2-chlorophenyl)-4,5-dimethoxybenzamide (1a)

Prepared according to Procedure A using 4,5-dimethoxyanthranilic acid (5.0 g, 25.4 mmol) and SOCl₂ (5.5 mL, 76.1 mmol) in benzene (100 mL), followed by 2-chloroaniline (6.7 mL, 63.5 mmol) and CHCl₃ (75 mL). The product was washed with aqueous NaHCO₃ (2 x 25 mL) and HCl (0.5 M, 75 mL) and purified by chromatography in CH₂Cl₂ to provide 4.3 g of a brown foam (55%). ¹H NMR (CDCl₃) δ: 8.42 (dd, J=1.5, 8.3 Hz, 1H); 8.32 (br s, 1H); 7.40 (dd, J=1.4, 8.0 Hz, 1H); 7.31 (dt, J=1.4, 7.9 Hz, 1H); 7.05 (dt, J=1.5, 7.7 Hz, 1H); 7.03 (s, 1H); 6.24 (s, 1H) ; 3.88 (s, 3H) ; 3.87 (s, 3H). MS (ES): m/z 307.0 (M+).

### 2-Amino-5-bromo-N-(2-chlorophenyl)benzamide (1b)

Prepared according to Procedure A using 2-amino-5-bromobenzoic acid (5.0 g, 23.1 mmol) and SOCl₂ (7.0 mL, 95.9 mmol) in benzene (50 mL), followed by 2-chloroaniline (7.3 mL, 69.3 mmol) and CHCl₃ (50 mL). The product was purified by two chromatographies in CH₂Cl₂ to provide 1.48 g of a yellow orange solid (20%). ¹H NMR (CDCl₃) δ: 8.36 (dd, J=1.2, 8.2 Hz, 1H); 8.20 (br s, 1H); 7.62 (d, J=2.1 Hz, 1H); 7.42 (dd, J=1.3, 8.0 Hz, 1H); 7.34 (dd, J=2.2, 8.8 Hz, 1H); 7.28-7.33 (m, 1H); 7.09 (dt, J=1.4, 7.7 Hz, 1H); 6.62 (d, J=8.7 Hz, 1H); 5.57 (br s, 2H).

### 2-Amino-N-(2-chlorophenyl)-4-fluorobenzamide (1c)

Prepared according to Procedure A using 2-amino-4-fluorobenzoic acid (1.15 g, 7.41 mmol) and SOCl₂ (1.4 mL, 18.5 mmol) in benzene (25 mL), followed by 2-chloroaniline (1.6 mL, 14.8 mmol) and CHCl₃ (25 mL). The product was chromatographed in CH₂Cl₂, then triturated from hexanes to provide 1.02 g of an off-white solid (52%). ¹H NMR (CDCl₃) δ: 12.91 (br s, 1H); 8.72 (dd, J=2.7, 12 Hz, 1H); 8.34 (dd, J=6.4, 9.2 Hz, 1H); 8.29 (dd, J=5.9, 8.8 Hz, 1H); 7.81 (dd, J=6.2, 8.8 Hz, 1H); 7.28 (dt, J=2.4, 8.4 Hz, 1H); 7.21 (dd, J=2.4, 9.0 Hz, 1H); 6.92 (ddd, J=2.4, 7.3, 9.1 Hz, 1H); 6.54 (ddd, J=2.4, 7.8, 8.8 Hz, 1H); 6.45 (dd, J=2.4, 11 Hz, 1H); 5.93 (br s, 2H). MS (ES) : m/z 265.0 (M+).

### 2-Amino-5-chloro-N-(2-chlorophenyl)benzamide (1d)

Prepared according to Procedure A using 2-amino-5-chlorobenzoic acid (2.0 g, 11.7 mmol) and SOCl₂ (2.2 mL, 29.2 mmol) in benzene (50 mL), followed by 2-chloroaniline (2.5 mL, 23.3 mmol) and CHCl₃ (50 mL). The product was purified by recrystallization from MeOH to provide 1.72 g of a dark yellow solid (52%). ¹H NMR (CDCl₃) δ: 8.37 (dd, J=1.5, 8.3 Hz, 1H) ; 8.22 (br s, 1H); 7.48 (d, J=2.3 Hz, 1H); 7.42 (dd, J=1.5, 8.1 Hz, 1H); 7.31 (dt, J=1.4, 7.8 Hz, 1H); 7.22 (dd, J=2.4, 8.8 Hz, 1H); 7.09 (dt, J=1.5, 7.7 Hz, 1H); 6.67 (d, J=8.8 Hz, 1H); 5.56 (br s, 2H),

### 2-Amino-N-(2-chlorophenyl)-6-fluorobenzamide (1e)

Prepared according to Procedure A using 2-amino-6-fluorobenzoic acid (2.0 g, 12.9 mmol) and SOCl₂ (2.3 mL, 32.2 mmol) in benzene (50 mL), followed by 2-chloroaniline (2.7 mL, 25.8 mmol) and CHCl₃ (50 mL). The product was purified by chromatography in EtOAc/hexanes to provide 2.06 g of a pale orange solid (60%). ¹H NMR (CDCl₃) δ: 9.00 (d, J=17 Hz, 1H); 8.47 (d, J=8.3 Hz, 1H); 7.41 (d, J=8.0 Hz, 1H) ; 7.30 (t, J=7.9 Hz, 1H); 7.10-7.20 (m, 1H); 7.07 (t, J=7.7 Hz, 1H); 6.49 (d, J=8.3 Hz, 1H); 6.03 (br s, 2H). MS (ES) : m/z 265.0 (M+).

### 2-Amino-6-chloro-N-(2-chlorophenyl)benzamide (1f)

Prepared according to Procedure A using 2-amino-6-chlorobenzoic acid (2.5 g, 14.6 mmol) and SOCl₂ (2.7 mL, 36.4 mmol) in benzene (75 mL), followed by 2-chloroaniline (3.1 mL, 29.1 mmol) and CHCl₃ (75 mL). The product chromatographed in CH₂Cl₂ to provide 1.05 g of a yellow orange solid (26%). ¹H NMR (CDCl₃) δ: 8.54 (d, J=8.1 Hz, 1H) ; 8.30 (br s, 1H); 7.41 (dd, J=1.5, 8.0 Hz, 1H); 7.33 (t, J=7.8 Hz, 1H); 7.10 (t, J=8.1 Hz, 1H); 7.09 (dt, J=1.6, 7.8 Hz, 1H); 6.78 (dd, J=0.4, 7.9 Hz, 1H); 6.63 (dd, J=0.9, 8.2 Hz, 1H); 4.69 (br s, 2H). MS (ES) : m/z 303.0 (M+22), 281.0 (M+).

### 2-Amino-N-(2-chlorophenyl)-6-methylbenzamide (1g)

Prepared according to Procedure A using 2-amino-6-methylbenzoic acid (2.5 g, 16.5 mmol) and SOCl₂ (3.0 mL, 41.3 mmol) in benzene (75 mL), followed by 2-chloroaniline (3.5 mL, 33.0 mmol) and CHCl₃ (75 mL). The product was chromatographed in CH₂Cl₂ to provide 2.19 g of a brown oil (51%). ¹H NMR (CDCl₃) δ: 8.58 (d, J=8.1 Hz, 1H); 7.99 (br.s,. 1H); 7.40 (dd, J=1.4, 8.0 Hz, 1H); 7.34 (t, J=7.7 Hz, 1H); 7.11 (t, J=7.8 Hz, 1H); 7.09 (dt, J=1.5, 7.7 Hz, 1H); 6.64 (d, J=7.5 Hz, 1H); 6.59 (d, J=8.1 Hz, 1H); 4.29 (br s, 2H) ; 2.45 (s, 3H). MS (ES): m/z 283.0 (M+22).

### 2-Amino-3-chloro-N-(2-chlorophenyl)benzamide (1h)

Prepared according to Procedure A using 2-amino-3-chlorobenzoic acid (1.0 g, 5.82 mmol) and SOCl₂ (1.1 mL, 14.6 mmol) in benzene (25 mL), followed by 2-chloroaniline (1.2 mL, 11.7 mmol) and CHCl₃ (25 mL). The product was recrystallized from MeOH to provide 1.29 g of a yellow solid (78%). ¹H NMR (CDCl₃) δ: 8.43 (dd, J=1.4, 8.3 Hz, 1H); 8.30 (br s, 1H); 7.47 (dd, J=1.1, 8.0 Hz, 1H) ; 7.42 (d, J=8.0 Hz, 2H); 7.33 (dt, J=1.4, 7.9 Hz, 1H); 7.09 (dt, J=1.5, 7.7 Hz, 1H); 6.68 (t, J=7.9 Hz, 1H); 6.13 (br s, 2H). MS (ES) : m/z 281.0 (M+).

### 2-Amino-N-biphenyl-2-yl-6-chlorobenzamide (1i)

Prepared according to Procedure A using 2-amino-6-chlorobenzoic acid (2.0 g, 11.7 mmol) and SOCl₂ (2.1 mL, 29.3 mmol) in benzene (60 mL), followed by 2-aminobiphenylamine (4.15 g, 24.5 mmol) and CHCl₃ (60 mL). The product was chromatographed in CH₂Cl₂ to provide 2.16 g of a foamy dark-amber residue (57%). ¹H NMR (CDCl₃) δ: 8.48 (d, J=8.2 Hz, 1H); 7.79 (br s, 1H); 7.34-7.46 (m, 6H); 7.20-7.30 (m, 2H); 7.00 (t, J=8.1 Hz, 1H); 6.63 (dd, J=0.6, 7.9 Hz, 1H); 6.54 (d, J=8.3 Hz, 1H); 4.58 (br s, 2H). MS (ES) : m/z 323.1 (M+).

### 2-Amino-6-chloro-N-o-tolylbenzamide (1j)

Prepared according to Procedure A using 2-amino-6-chlorobenzoic acid (1.0 g, 5.83 mmol) and SOCl₂ (1.1 mL, 14.6 mmol) in benzene (30 mL), followed by o-toluidine (1.4 mL, 12.8 mmol) and CHCl₃ (30 mL). The product was chromatographed in CH₂Cl₂ to provide 840 mg of an oily yellow solid (55%). ¹H NMR (CDCl₃) δ: 7.96 (d, J=7.9 Hz, 1H); 7.60 (br s, 1H); 7.23-7.30 (m, 2H); 7.14 (t, J=7.5 Hz, 1H); 7.11 (t, J=8.3 Hz, 1H); 6.78 (d, J=7.9 Hz, 1H); 6.64 (d, J=8.2 Hz, 1H); 4.73 (br s, 2H); 2.35 (s, 3H). MS (ES) : m/z 261.0 (M+).

### 2-Amino-6-chloro-N-(2-fluorophenyl)benzamide (1k)

Prepared according to Procedure A using 2-amino-6-chlorobenzoic acid (2.0 g, 11.7 mmol) and SOCl₂ (2.1 mL, 29.1 mmol) in benzene (60 mL), followed by 2-fluoroaniline (2.3 mL, 23.4 mmol) and CHCl₃ (60 mL). The product was chromatographed in CH₂Cl₂ to provide 1.05 g of a yellow solid (34%). ¹H NMR (CDCl₃) δ: 8.45 (t, J=8.0 Hz, 1H); 8.01 (br s, 1H); 7.02-7.22 (m, 4H); 6.78 (dd, J=0.5, 7.9 Hz, 1H); 6.64 (dd, J=0.8, 8.2 Hz, 1H); 4.73 (br s, 2H). MS (ES): m/z 265.0 (M+).

### 2-Amino-6-chloro-N-(2-methoxyphenyl)benzamide (11)

Prepared according to Procedure A using 2-amino-6-chlorobenzoic acid (2.0 g, 11.7 mmol) and SOCl₂ (2.1 mL, 29.1 mmol) in benzene (60 mL), followed by o-anisidine (2.6 mL, 23.4 mmol) and CHCl₃ (60 mL). The product was chromatographed in CH₂Cl₂ to provide 2.61 g of a dark yellow oil (81%). ¹H NMR (CDCl₃) δ: 8.53 (dd, J=1.7, 7.9 Hz, 1H); 8.39 (br s, 1H); 7.11 (dt, J=1.6, 7.8 Hz, 1H); 7.09 (t, J=8.1 Hz, 1H) ; 7.02 (dt, J=1.4, 7.8 Hz, 1H); 6.92 (dd, J=1.4, 8.0 Hz, 1H); 6.62 (dd, J=0.9, 8.2 Hz, 1H); 4.66 (br s, 2H); 3.87 (s, 3H). MS (ES): m/z 277.0 (M+).

### 2-Amino-N-(2-chlorophenyl)-3-trifluoromethylbenz-amide (1m)

Prepared according to Procedure A using 3-trifluoromethylanthranilic acid (2.0 g, 9.75 mmol) and SOCl₂ (1.8 mL, 24.4 mmol) in benzene (50 mL), followed by 2-chloroaniline (2.1 mL, 19.5 mmol) and CHCl₃ (50 mL). The product was purified by recrystallization from MeOH to provide 2.38 g yellow crystals (78%). ¹H NMR (CDCl₃) δ: 8.40 (dd, J=1.4, 8.3 Hz, 1H); 8.25 (br s, 1H); 7.71 (d, J=7.8 Hz, 1H); 7.60 (d, J=7.8 Hz, 1H); 7.43 (dd, J=1.4, 8.0 Hz, 1H); 7.34 (dt, J=1.3, 7.9 Hz, 1H); 7.11 (dt, J=1.5, 7.7 Hz, 1H); 6.77 (t, J=7.8 Hz, 1H); 6.24 (br s, 2H). MS (ES) : m/z 315.0 (M+).

### 3-Aminonaphthalene-2-carboxylic acid (2-chlorophenyl) amide (1n)

Prepared according to Procedure A using 3-amino-2-napthoic acid (2.0 g, 10.7 mmol) and SOCl₂ (1.9 mL, 26.7 mmol) in benzene (50 mL), followed by 2-chloroaniline (2.3 mL, 21.4 mmol) and CHCl₃ (50 mL). The product was recrystallized from MeOH to provide 1.71 g of a brown solid (54%). ¹H NMR (CDCl₃) δ: 10.88 (br s, 1H); 9.21 (s, 1H); 8.91 (s, 1H); 8.70 (dd, J=1.0, 8.3 Hz, 1H); 7.95-8.01 (m, 1H); 7.87-7.94 (m, 1H); 7.60-7.68 (m, 2H); 7.41 (dd, J=1.3, 8.0 Hz, 1H); 7.34 (dt, J=1.2, 7.8 Hz, 1H); 7.07 (dt, J=1.4, 7.7 Hz, 1H). MS (ES): m/z 297.1 (M+).

### 2-Amino-N-(2-chlorophenyl)-4-nitrobenzamide (1o)

Prepared according to Procedure A using 4-nitroanthranilic acid (5.0 g, 27.5 mmol) and SOCl₂ (5.0 mL, 68.6 mmol) in benzene (150 mL), followed by 2-chloroaniline (5.8 mL, 55.0 mmol) and CHCl₃ (150 mL). The product was purified by chromatography in CH₂Cl₂ followed by recrystallization from MeOH to provide 2.20 g of an orange-brown solid (31%). ¹H NMR (CDCl₃) δ: 8.41 (dd, J=1.3, 8.3 Hz, 1H); 8.31 (br s, 1H); 7.67 (d, J=8.6 Hz, 1H); 7.57 (d, J=2.1 Hz, 1H); 7.52 (dd, J=2.2, 8.5 Hz, 1H); 7.44 (dd, J=1.3, 8.1 Hz, 1H); 7.35 (dt, J=1.3, 7.9 Hz, 1H); 7.13 (dt, J=1.4, 7.8 Hz, 1H); 5.88 (br s, 2H). MS (ES): m/z 292.0 (M+).

### 2-Amino-N-(2-chlorophenyl)-5-hydroxybenzamide (1p)

Prepared according to Procedure A using 2-amino-5-hydroxybenzoic acid (5.0 g, 32.7 mmol) and SOCl₂ (6.0 mL, 81.6 mmol) in benzene (150 mL), followed by 2-chloroaniline (6.9 mL, 65.4 mmol) and CHCl₃ (150 mL). The product was purified by two chromatographies in MeOH/CH₂Cl₂ to provide 990 mg of a brown solid (12%). ¹H NMR (MeOH-d₄) δ: 7.92 (dd, J=1.6, 8.1 Hz, 1H); 7.48 (dd, J=1.5, 7.7 Hz, 1H); 7.34 (dt, J=1.5, 7.7 Hz, 1H); 7.20 (dt, J=1.7, 7.7 Hz, 1H); 7.16 (d, J=2.7 Hz, 1H); 6.83 (dd, J=2.7, 8.7 Hz, 1H); 6.76 (d, J=8.7 Hz, 1H); [6.24 (br s, 2H)). MS (ES) : m/z 263.0 (M+).

### 2-Amino-N-(2-chlorophenyl)-4,5-difluorobenzamide (1q)

Prepared according to Procedure A using 4,5-difluoroanthranilic acid (2.0 g, 11.6 mmol) and SOCl₂ (2.1 mL; 28.9 mmol) in benzene (60 mL), followed by 2-chloroaniline (2.4 mL, 23.2 mmol) and CHCl₃ (60 mL). The product was purified by two chromatographies in CH₂Cl₂ and EtOAc/hexanes to provide 769 mg of a yellow solid (23%). ¹H NMR (CDCl₃) δ: 8.69-8.82 (m, 1H); 8.00 (dd, J=8.4, 9.0 Hz, 1H); 7.90 (dd, J=8.9, 12 Hz, 1H); 7.39 (dd, J=6.8, 10 Hz, 1H); 6.53 (dd, J=6.6, 12 Hz, 1H); 6.41 (br s, 2H); 5.79 (br s, 1H). MS (ES) : m/z 283.1 (M+).

### 2-Amino-N-(2-chlorophenyl)-5-fluorobenzamide (1r)

Prepared according to Procedure A using 2-amino-5-fluorobenzoic acid (1.0 g, 6.45 mmol) and SOCl₂ (1.2 mL, 16.1 mmol) in benzene (30 mL), followed by 2-chloroaniline (1.4 mL, 12.9 mmol) and CHCl₃ (30 mL). The product was triturated from CH₂Cl₂ to provide 985 mg of a mustard-yellow solid (58%). ¹H NMR (CDCl₃) δ: 7.66 (dd, J=2.9, .8.7 Hz, 1H); 7.52-7.55 (m, 1H); 7.32-7.37 (m, 3H); 7.09 (dt, J=3.0, 8.5 Hz, 1H); 6.71 (dd, J=4.3, 8.7 Hz, 1H). MS (ES) : m/z 305.0 (M+40).

### EXAMPLE 9

### Preparation of Intermediate Compounds: Chlorides

### 2-Chloromethyl-3-(2-chlorophenyl)-6,7-dimethoxy-3H-quinazolin-4-one (2a)

Prepared according to Procedure B with 1a (2.95 g, 9.63 mmol) and chloroacetyl chloride (2.3 mL, 28.9 mmol) in acetic acid (30 mL). Purified by extraction from aq. K₂CO₃ and recrystallization from isopropanol to afford 1.61 g of a brown crystalline solid (46%). ¹H NMR (CDCl₃) δ: 7.59-7.66 (m, 2H); 7.45-7.56 (m, 3H); 7.20 (s, 1H); 4.37 (d, J=12 Hz, 1H), 4.08 (d, J=12 Hz, 1H); 4.04 (s, 3H); 4.00 (s, 3H). MS (ES) : m/z 365.0 (M+).

### 6-Bromo-2-chloromethyl-3-(2-chlorophenyl)-3H-quin-azolin-4-one (2b)

Prepared according to Procedure B with 1b (500 mg, 1.54 mmol) and chloroacetyl chloride (0.37 mL, 4.61 mmol) in acetic acid (10 mL). Purified by recrystallization from isopropanol to afford 490 mg of an off-white solid (83%). ¹H NMR (CDCl₃) δ: 8.43 (d, J=2.3 Hz, 1H); 7.91 (dd, J=2.3, 8.7 Hz, 1H); 7.67 (d, J=8.7 Hz, 1H); 7.60-7.65 (m, 1H); 7.47-7.56 (m, 2H) ; 7.52 (t, J=5.3 Hz, 1H); 7.47-7.56 (m, 1H); 4.37 (d, J=12 Hz, 1H), 4.06 (d, J=12 Hz, 1H). MS (ES) : m/z 385:0 (M+).

### 2-Chloromethyl-3-(2-chlorophenyl)-7-fluoro-3H-quinazolin-4-one (2c)

Prepared according to Procedure B with 1c (500 mg, 1.89 mmol) and chloroacetyl chloride (0.45 mL, 5.67 mmol) in acetic acid (10 mL). Purified by extraction from aqueous K₂CO₃, followed by recrystallization from isopropanol to afford 501 mg of a yellow crystalline solid (82%) . ¹H NMR (CDCl₃) δ: 8.32 (dd, J=6.0, 8.9 Hz, 1H); 7.59-7.66 (m, 1H); 7.50-7.55 (m, 3H); 7.44 (dd, J=2.4, 9.4 Hz, 1H); 7.27 (dt, J=2.5, 8.5 Hz, 1H); 4.37 (d, J=12 Hz, 1H), 4.07 (d, J=12 Hz, 1H). MS (ES): m/z 323.0 (M+).

### 6-Chloro-2-chloromethyl-3-(2-chlorophenyl)-3H-quinazolin-4-one (2d)

Prepared according to Procedure B with 1d (500 mg, 1.78 mmol) and chloroacetyl chloride (0.42 mL, 5.33 mmol) in acetic acid (10 mL). Purified by recrystallization from isopropanol to afford 555 mg of a yellow solid (92%). ¹H NMR (CDCl₃) δ: 8.27 (d, J=1.9 Hz, 1H); 7.74-7.78 (m, 2H); 7.60-7.66 (m, 1H); 7.48-7.57 (m, 3H); 4.37 (d, J=12 Hz, 1H), 4.07 (d, J=12 Hz, 1H). MS (ES): m/z 339.0 (M+).

### 2-Chloromethyl-3-(2-chlorophenyl)-5-fluoro-3H-quinazolin-4-one (2e)

Prepared according to Procedure B with 1e (500 mg, 1.89 mmol) and chloroacetyl chloride (0.45 mL, 5.67 mmol) in acetic acid (10 mL). Purified by extraction from aq. K₂CO₃ and recrystallization from isopropanol to afford 430 mg of an off-white crystalline solid (70%) . ¹H NMR (CDCl₃) δ: 7.76 (dt, J=5.3, 8.2 Hz, 1H); 7.56-7.65 (m, 2H); 7.47-7.56 (m, 3H); 7.16-7.25 (m, 1H); 4.35 (d, J=12 Hz, 1H), 4.07 (d, J=12 Hz, 1H). MS (ES): m/z 323.0 (M+).

### 5-Chloro-2-chloromethyl-3-(2-chlorophenyl)-3H-quinazolin-4-one (2f)

Prepared according to Procedure B with 1f (1.00 g, 3.56 mmol) and chloroacetyl chloride (0.85 mL, 10.7 mmol) in acetic acid (15 mL). Purified by recrystallization from isopropanol to afford 791 mg of an off-white crystalline solid (65%). ¹H NMR (CDCl₃) δ: 7.70 (s, 1H) ; 7.68 (d, J=3.8 Hz, 1H); 7.61-7.65 (m, 1H); 7.55 (dd, J=2.7, 6.4 Hz, 1H); 7.51 (d, J=3.1 Hz, 1H); 7.50 (s, 2H); 4.35 (d, J=12 Hz, 1H), 4.05 (d, J=12 Hz, 1H). MS (ES): m/z 339.0 (M+).

### 2-Chloromethyl-3-(2-chlorophenyl)-5-methyl-3H-quinazolin-4-one (2g)

Prepared according to Procedure B with 1g (2.18 g, 8.36 mmol) and chloroacetyl chloride (2.0 mL, 25.1 mmol) in acetic acid (40 mL). Purified by two chromatographies in CH₂Cl₂ and EtOAc/hexanes, followed by recrystallization from isopropanol to afford 638 mg of an off-white crystalline solid (24%) . ¹H NMR (DMSO-d₆) δ: 7.73-7.80 (m, 3H); 7.58-7.64 (m, 3H); 7.41 (d, J=7.4 Hz, 1H); 4.40 (d, J=12 Hz, 1H), 4.26 (d, J=12 Hz, 1H); 2.74 (s, 3H). MS (ES): m/z 319.0 (M+).

### B-Chloro-2-chloromethyl-1-3-(2-'chlorophenyl)-3H-quinazolin-4-one (2h)

Prepared according to Procedure B with 1h (500 mg, 1.78 mmol) and chloroacetyl chloride (0.49 mL, 6.13 mmol) in acetic acid (10 mL). Purified by extraction from aqueous K₂CO₃, followed by recrystallization from isopropanol to afford 448 mg of a yellow solid (74%). ¹H NMR (CDCl₃) δ: 8.23 (dd, J=1.4, 8.0 Hz, 1H); 7.90 (dd, J=1.4, 7.8 Hz, 1H); 7.61-7.66 (m, 1H); 7.51-7.55 (m, 3H); 7.47 (t, J=8.0 Hz, 1H); 4.48 (d, J=12 Hz, 1H), 4.12 (d, J=12 Hz, 1H). MS (ES) : m/z 339.0 (M+).

### 3-Biphenyl-2-yl-5-chloro-2-chloromethyl-3H-quinazolin-4-one (2i)

Prepared according to Procedure B with 1i (2.0 g, 6.20 mmol) and chloroacetyl chloride (1.5 mL, 18.6 mmol) in acetic acid (30 mL). Purified by chromatography in CH₂Cl₂, followed by recrystallization from isopropanol to afford 1.44 g of an off-white solid (61%). ¹H NMR (CDCl₃) δ: 7.61-7.64 (m, 1H); 7.58-7.59 (m, 1H); 7.54-7.57 (m, 2H); 7.52-7.53 (m, 1H); 7.45-7.52 (m, 2H); 7.24 (s, 5H); 3.92-4.03 (m, 2H). MS (ES) : m/z 381.0 (M+).

### 5-Chloro-2-chloromethyl-3-o-tolyl-3H-quinazolin-4-one (2j)

Prepared according to Procedure B with 1j (750 mg, 2.88 mmol) and chloroacetyl chloride (0.69 mL, 8.63 mmol) in acetic acid (15 mL). Purified by chromatography in CH₂Cl₂, followed by recrystallization from isopropanol to afford 340 mg of a white solid (37%). ¹H NMR (CDCl₃) δ: 7.69 (d, J=2.1 Hz, 1H); 7.68 (q, J=7.4 Hz, 1H); 7.54 (dd, J=2.2, 7.0 Hz, 1H); 7.35-7.47 (m, 3H); 7.21-7.25 (m, 1H); 4.27 (d, J=12 Hz, 1H); 4.11 (d, J=12 Hz, 1H); 2.18 (s, 3H). MS (ES): m/z 319.0 (M+).

### 5-Chloro-2-chloromethyl-3-(2-fluorophenyl)-3H-quinazolin-4-one (2k)

Prepared according to Procedure B with 1k (1.0 g, 3.78 mmol) and chloroacetyl chloride (0.90 mL, 11.3 mmol) in acetic acid (20 mL). Purified by chromatography in CH₂Cl₂ to afford 484 mg of a pale pink solid (40%). ¹H NMR (CDCl₃) δ: 7.69 (s, 1H); 7.68 (d, J=3.2 Hz, 1H); 7.56 (d, J=3.0 Hz, 1H); 7.54 (d, J=3.0 Hz, 1H); 7.40-7.47 (m, 1H); 7.35-7.38 (m, 1H); 7.27-7.32 (m, 1H); 4.35 (d, J=12.Hz, 1H); 4.18 (d, J=12 Hz, 1H). MS (ES): m/z 323.0 (M+).

### 5-Chloro-2-chloromethyl-3-(2-methoxyphenyl)-3H-quinazolin-4-one (21)

Prepared according to Procedure B with 11 (2.6 g, 9.41 mmol) and chloroacetyl chloride (2.2 mL, 28.2 mmol) in acetic acid (40 mL). Purified by chromatography in CH₂Cl₂, followed by recrystallization from isopropanol to afford 874 mg of a pale yellow solid (28%). ¹H NMR (CDCl₃) δ: 7.55-7.74 (m, 2H); 7.47-7.54 (m, 2H); 7.34 (dd, J=1.7, 7.8 Hz, 1H); 7.13 (dt, J=1.2, 7.7 Hz, 1H, 7.08 (dd, J=1.0, 8.4 Hz, 1H); 4.29 (d, J=12 Hz, 1H); 4.11 (d, J=12 Hz, 1H); 3.80 (s, 3H). MS (ES): m/z 335.0 (M+).

### 2-Chloromethyl-3-(2-chlorophenyl)-8-trifluoromethyl-3H-quinazolin-4-one (2m)

Prepared according co Procedure B with 1m (500 mg, 1.59 mmol) and chloroacetyl chloride (0.38 mL, 4.77 mmol) in acetic acid (10 mL). Purified by recrystallization from isopropanol to afford 359 mg of a white crystalline solid (61%). ¹H NMR (CDCl₃) δ: 8.51 (dd, J=1.0, 8.0 Hz, 1H); 8.14 (d, J=7.3 Hz, 1H); 7.65 (dd, J=2.5, 5.6 Hz, 1H); 7.62 (d, J=3.9 Hz, 1H); 7.48-7.60 (m, 3H); 4.44 (d, J=12 Hz, 1H), 4.12 (d, J=12 Hz, 1H). MS (ES): m/z 373.0 (M+).

### 2-Chloromethyl-3-(2-chlorophenyl)-3H-benzo[g]quin-azolin-4-one (2n)

Prepared according to Procedure B with 1n (500 mg, 1.68 mmol) and chloroacetyl chloride (0.40 mL, 5.05 mmol) in acetic acid (10 mL). Purified by chromatography in CH₂Cl₂ followed by recrystallization from isopropanol to afford 232 mg of a light-brown solid (39%). ¹HNMR (CDCl₃) 5: 8.92 (s, 1H); 8.29 (s, 1H); 8.81 (d, J=8.3, 1H); 8.32 (d, J=8.3 Hz, 1H); 7.51-7.69 (m, 4H); 7.55 (d, J=5.2 Hz, 1H); 7.53 (d, J=3.8 Hz, 1H); 4.43 (d, J=12 Hz, 1H), 4.12 (d, J=12 Hz, 1H). MS (ES): m/z 355.0 (M+).

### 2-Chloromethyl-3-(2-chlorophenyl)-7-nitro-3H-quinazolin-4-one (2o)

Prepared according to Procedure B with 1o (500 mg, 1.71 mmol) and chloroacetyl chloride (0.41 mL, 5.14 mmol) in acetic acid (10 mL). Purified by extraction from aqueous K₂CO₃, followed by two chromatographies in CH₂Cl, to afford 338 mg of a yellow oil (56%). ¹H NMR (CDCl₃) δ: 8.64 (d, J=2.2 Hz, 1H); 8.48 (d, J=8.8 Hz, 1H); 8.32 (dd, J=2.2, 8.7 Hz, 1H); 7.66 (dd, J=2.5, 6.0.Hz, 1H); 7.52-7.59 (m, 3H); 4.41 (d, J=12 Hz, 1H), 4.10 (d, J=12 Hz, 1H). MS (ES): m/z 350.0 (M+).

### Acetic acid 2-chloromethyl-3-(2-chlorophenyl)-4-oxo-3,4-dihydro-quinazolin-6-yl ester (2p)

Prepared according to Procedure B with 1p (670 mg, 2.55 mmol) and chloroacetyl chloride (0.61 mL, 7.65 mmol) in acetic acid (10 mL). Purified by chromatography in 0-3% MeOH/CH₂Cl₂, followed by recrystallization from isopropanol to afford 523 mg of the acetate as pale-peach crystals (57%). ¹H NMR (CDCl₃) δ: 8.00 (d, J=2.7 Hz, 1H); 7.82 (d, J=8.8 Hz, 1H); 7.60-7.66 (m, 1H); 7.56 (dd, J=2.7, 8.8 Hz, 1H); 7.51 (t, J=4.7 Hz, 2H); 7.50 (s, 1H); 4.38 (d, J=12 Hz, 1H), 4.08 (d, J=12 Hz, 1H); 2.36 (s, 3H). MS (ES): m/z 363.0 (M+).

### 2-Chloromethyl-3-(2-chlorophenyl)-6,7-difluoro-3H-quinazolin-4-one (2q)

Prepared according to Procedure B with 1q (700 mg, 2.48 mmol) and chloroacetyl chloride (0.60 mL, 7.43 mmol) in acetic acid (12 mL). Purified by chromatography in CH₂Cl₂, followed by recrystallization from isopropanol to afford 219 mg of a yellow crystalline solid (26%). ¹H NMR (CDCl₃) δ: 8.07 (dd, J=8.5, 9.7 Hz, 1H); 7.64 (dd, J=2.5, 5.6 Hz, 1H); 7.60 (dd, J=3.5, 11 Hz, 1H); 7.55 (q, J=2.9 Hz, 3H); 7.52 (d, J=1.9 Hz, 1H); 7.49-7.51 (m, 1H); 4.36 (d, J=12 Hz, 1H), 4.06 (d, J=12 Hz, 1H). MS (ES): m/z 341.0 (M+).

### 2-Chloromethyl-3-(2-chlorophenyl)-6-fluoro-3H-quinazolin-4-one (2r)

Prepared according to Procedure B with 1r (850 mg, 3.21 mmol) and chloroacetyl chloride (0.77 mL, 9.63 mmol) in acetic acid (15 mL). Purified by extraction from aqueous K₂CO₃, followed by chromatography in EtOAc/hexanes. A second chromatography in acetone/hexanes afforded 125 mg of a white solid (12%). ¹H NMR (CDCl₃) δ: 7.95 (dd, J=2.9, 8.2 Hz, 1H); 7.81 (dd, J=4.8, 9.0 Hz, 1H) ; 7.61-7.66 (m, 1H); 7.57 (dd, J=2.7, 8.6 Hz, 1H); 7.57 (dd, J=2.7, 8.6 Hz, 1H); 7.52 (dd, J=3.2, 6.9 Hz, 1H); 7.52 (br s; 2H); 4.38 (d, J=12 Hz, 1H), 4.08 (d, J=12 Hz, 1H). MS (ES): m/z 323.0 (M+).

### EXAMPLE 10

### Preparation of PI3K5 Inhibitor Compounds

The following compounds of the present invention were prepared as outlined in Procedure C, using 2-chloromethyl-5-methyl-3-o-tolyl-3H-quinazolin-4-one (10 mg), the appropriate nucleophile XH (20 mg, excess), and potassium carbonate (10 mg) in DMF (0.25 mL). The reaction mixture was stirred 16 h at room temperature, quenched with water, and the crude solid product was collected by filtration and air dried. The crude material was dissolved in 0.5 mL of DMSO and purified by reversed-phase HPLC (C18 Luna column, 4.6 x 250 mm, 4.7 mL/min, 10-75% acetonitrile/water over 15 min, 100% acetonitrile at 18 min, detector at 220λ). Appropriate fractions were concentrated *in vacuo* to yield the final products.

### 2-(4-Amino-1,3,5-triazin-2-ylsulfanylmethyl)-5-methyl-3-o-tolyl-3H-quinazolin-4-one (D-087)

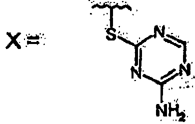

Yield: 5.8 mg.
1H NMR (300 MHz, *d₆*-DMSO) δ: 8.10 (s, 1H), 7.70 (t, J=7.8 Hz, 1H), 7.58 (s, 1H), 7.52 (d, J=8.0 Hz, 1H), 7.48-7.26 (m, 6H), 4.08 (s, 2H), 2.73 (s, 3H), 2.09 (s, 3H).
LRMS (ES pos.) m/z = 391 (M+1).

### 5-Methyl-2-(2-oxo-1,2-dihydro-pyrimidin-4-ylsulfanylmethyl)-3-o-tolyl-3H-quinazolin-4-one (D-089)

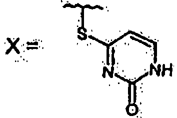

Yield: 2.4 mg.
¹H NMR (300 MHz, d₆-DMSO) δ: 11.49 (s, 1H), 7.70 (t, J=7.8 Hz, 1H), 7.60 (brt, J=6.0 Hz, 1H), 7.53-7.48 (m, 2H), 7.46-7.28 (m, 4H), 6.31 (d, J=6.7 HZ, 1H), 4.05 (s, 2H), 2.73 (s, 3H), 2.12 (s, 3H).
LRMS (ES pos.) m/z = 391 (M+1).

### 2-(2,6-Diamino-pyrimidin-4-ylsulfanylmethyl)-5-methyl-3-o-tolyl-3H-quinazolin-4-one (D-093)

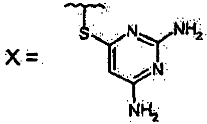

¹H NMR (300 MHz, *d₆*-DMSO) δ: 7.70 (t, J=7.7 Hz, 1H), 7.54 (d, J=8.0 Hz, 1H), 7.45-7.27 (m, 5H), 6.22 (br s, 1H), 5.80 (br s, 1H), 3.99 (AB quartet, *J_{AB}*=14.6 Hz, Δν=26.9 Hz, 2H), 2.73 (s, 3H), 2.08 (s, 3H).
LRMS (ES pos.) m/z = 405 (M+1).

### 5-Methyl-2-(1-methyl-1H-imidazol-2-ylsulfanylmethyl)-3-o-tolyl-3H-quinazolin-4-one (D-097)

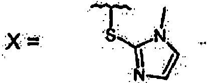

¹H NMR (300 MHz, *d₆*-DMSO) δ: 7.69 t, J=7.8 Hz, 1H), 7.46-7.37 (m, 5H), 7.32 (d, J=7.3 Hz, 1H), 7.20 (d, J=1.0 Hz, 1H), 6.48 (d, J=1.0 Hz), 3.83 (AB quartet, *J_{AB}*=15.0 Hz, Δν=18.8 Hz, 1H), 3.55 (s, 3H), 2.73 (s, 3H), 2.09 (s, 3H).
LRMS (ES pos.) m/z = 364 (M+1).

### 5-Methyl-3-o-tolyl-2-(1H-[1,2,4]triazol-3-ylsulfanylmethyl)-3H-quinazolin-4-one (D-098)

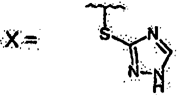

¹H NMR (300 MHz, *d*₆-DMSO) δ: 13.98 (s, 1H), 8.47 (s, 1H), 7.70 (t, J=7,8 Hz, 1H), 7.49 (d, J=7.9 Hz, 1H), 7.44-7.31 (m, 5H), 4.04 (AB quartet, *J_{AB}*=15.5 Hz, Δν=19.1 Hz, 1H), 2.74 (s, 3H), 2.10 (s, 3H).
LRMS (ES pos.) m/z = 364 (M+1).

## Claims

1. A compound having a general structural formula: 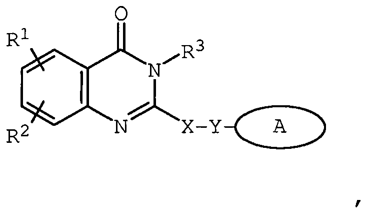
wherein the A ring system is selected from the group consisting of imidazolyl, pyrazolyl, 1,2,3-triazolyl, pyridizinyl, pyrimidinyl, pyrazinyl, 1,3,5-triazinyl, cinnolinyl, phthalazinyl, quinazolinyl, quinoxalinyl, 1,8-naphthyridinyl, pteridinyl, 1H-indazolyl, and benzimidazolyl,
each of which which may be unsubstituted or substituted with one to three substituents selected from the group consisting of N(R^{a})_{2,} halo, C₁₋₃alkyl, S(C₁₋₃alkyl), OR^{a}, halo, and 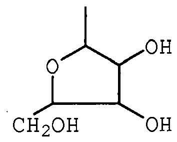
wherein X is selected from the group consisting of CHR^{b}, CH₂CHR^{b}, and CH=C(R^{b});
Y is selected from the group consisting of null, SO, SO₂, NH, 0, C(=O), OC(=O), C(=O)O, and NHC(=O)CH₂S;
R¹ and R², independently, are selected from the group consisting of hydrogen, C₁₋₆alkyl, aryl, heteroaryl, halo, NHC(=O)C₁₋₃alkyleneN(R^{a})₂, NO₂, OR^{a}, OCF₃, N(R^{a})₂, CN, OC(=O)R^{a}, C(=O)R^{a}, C(=O)OR^{a}, arylOR^{b}, Het, NR^{a}C(=O)C₁₋₃alkyleneC(=O)OR^{a}, arylOC₁₋₃alkyleneN(R^{a})₂, arylOC(=O)R^{a}, C₁₋₄alkyleneC(=O)OR^{a}, OC₁₋₄alkyleneC(=O)OR^{a}, C₁₋₄alkyleneOC₁₋₄alkyleneC(=O)OR^{a}, C(=O)NR^{a}SO₂R^{a}, C₁₋₄alkyleneN(R^{a})₂, C₂₋₆alkenyleneN(R^{a})₂, C(=O) - NR^{a}C₁₋₄alkyleneOR^{a}, C(=O)NR^{a}C₁₋₄alkyleneHet, OC₂₋₄alkyl-eneN(R^{a})₂, OC₁₋₄alkyleneCH(OR^{b})CH₂N(R^{a})₂, OC₁₋₄alkyleneHet, OC₂₋₄alkyleneOR^{a}, OC₂₋₄alkyleneNR^{a}C(=O)OR^{a}, NR^{a}C₁₋₄alkyleneN(R^{a})₂, NR^{a}C(=O)R^{a}, NR^{a}C(=O)N(R^{a})₂, N(SO₂C₁₋₄alkyl)₂, NR^{a}(SO₂C₁₋₄alkyl), SO₂N(R^{a})₂, OSO₂CF₃, C₁₋₃alkylenearyl, C₁₋₄alkyleneHet, C₁₋₆alkyleneOR^{b}, C₁₋₃alkyleneN(R^{a})₂, C(=O)N(R^{a})₂, NHC(=O)C₁-C₃alkylenearyl, C₃₋₈cycloalkyl, C₃₋₈heterocycloalkyl, arylOC(=O)R^{b}, NHC(=O)C₁₋₃alkyleneC₃₋₈heterocycloalkyl, NHC(=O)C₁₋₃alkyleneHet, OC₁₋₄lkyleneOC₁₋₄lkyleneC(=O)OR^{b}, C(=O)C₁₋₄alkyleneHet, and NHC(=O)haloC₁₋₆alkyl;
or R¹ and R² are taken together to form a 3- or 4-membered alkylene or alkenylene chain component of a 5- or 6-membered ring, optionally containing at least one heteroatom;
R³ is hydrogen or is selected from the group consisting of C₁₋₆alkyl, C₃₋₈cycloalkyl, C₃₋₈heterocycloalkyl, C₁₋₄alkylenecycloalkyl, C₂₋₆alkenyl, C₁₋₃alkylenearyl, arylC₁₋₃alkyl, C(=O)R^{a}, aryl, heteroaryl, C(=O)OR^{a}, C(=O)N(R^{a})₂, C(=S)N(R^{a})₂, SO₂R^{a}, SO₂N(R^{a})₂, S(=O)R^{a}, S(=O)N(R^{a})₂, C(=O)NR^{a}C₁₋₄alkyleneOR^{a}, C(=O)NR^{a}C₁₋₄alkyleneHet, C(=O)C₁₋₄alkylenearyl, C(=O)C₁₋₄alkyleneheteroaryl, C₁₋₄alkyleneheteroaryl, C₁₋₄alkyleneHet, C₁₋₄alkyleneC(=O)C₁₋₄alkylenearyl, C₁₋₄alkyleneC(=O)C₁₋₄alkyleneheteroaryl, C₁₋₄alkylene-C(=O)Het, C₁₋₄alkyleneC(=O)N(R^{a})₂, C₁₋₄alkyleneOR^{a}, C₁₋₄alkyleneNR^{a}C(=O)R^{a}, C₁₋₄alkyleneOC₁₋₄alkyleneOR^{a}, C₁₋₄alkyleneN(R^{a})₂, C₁₋₄alkyleneC(=O)OR^{a}, and C₁₋₄alkyleneOC₁₋₄alkyleneC(=O)OR^{a}, each of which is optionally substituted with a substituent selected from the group consisting of halo, OR^{a}, C₁₋₆alkyl, aryl, heteroaryl, NO₂, N(R^{a})₂, NR^{a}SO₂CF₃, NR^{a}C(=O)R^{a}, C(=O)OR^{a}, SO₂N(R^{a})_{2,} CN, C(=O)R^{a}, C₁₋₄alkyleneN(R^{a})_{2,} OC₁₋₄alkyleneN(R^{a})₂, and N(R^{a})C₁₋₄alkyleneN(R^{a})₂; or
R³ is C₁₋₄alkylenearyl optionally substituted with one or more of halo, SO₂N(R^{a})₂, N(R^{a})₂, C(=O)OR^{a}, NR^{a}SO₂CF₃, CN, NO₂, C(=O)R^{a}, OR^{a}, C₁₋₄alkyleneN(R^{a})₂, and OC₁₋₄alkyleneN(R^{a})₂;
R^{a} is selected from the group consisting of hydrogen, C₁₋₆alkyl, C₃₋₈cycloalkyl, C₃₋₈heterocycloalkyl, C₁₋₃alkyleneN(R^{a})₂, aryl, arylC₁₋₃alkyl, C₁₋₃alkylenearyl, heteroaryl, heteroarylC₁₋₃alkyl, and C₁₋₃alkyleneheteroaryl;
or two R^{a} groups are taken together to form a 5-or 6-membered ring, optionally containing at least one heteroatom;
R^{b} is selected from the group consisting of hydrogen, C₁₋₆alkyl, aryl, heteroaryl, arylC₁₋₃alkyl, heteroarylC₁₋₃alkyl, C₁₋₃alkylenearyl, and C₁₋₃alkyleneheteroaryl;
Het is a 5- or 6-membered heterocyclic ring, saturated or partially or fully unsaturated, containing at least one heteroatom selected from the group consisting of oxygen, nitrogen, and sulfur, and optionally substituted with C₁₋₄alkyl or C(=O)OR^{a};
and pharmaceutically acceptable salts and solvates thereof,
wherein "aryl" is defined as a monocyclic or polycyclic aromatic group which may be unsubstituted or substituted with one or more, and in particular one to three, halo, alkyl, phenyl, hydroxyalkyl, alkoxy, alkoxyalkyl, haloalkyl, nitro, amino, alkylamino, acylamino, alkylthio, alkylsulfinyl, and alkylsulfonyl and wherein "heteroaryl" is defined as a monocyclic or bicyclic ring system containing one or two aromatic rings and containing at least one nitrogen, oxygen or sulfur atom in an aromatic ring, which may be unsubsitituted or substituted with one or more, and in particular one to three
halo, alkyl, hydroxy, hydroxyalkyl, alkoxy, alkoxyalkyl, haloalkyl, nitro, amino, alkylamino, acylamino, alkylthio, alkylsulfinyl, and alkylsulfonyl.

2. The compound of claim 1 wherein X is selected from the group consisting of CH₂, CH₂CH₂, CH=CH, CH(CH₃), CH₂CH(CH₃, and C(CH₃)₂;or a pharmaceutically acceptable salt or solvate thereof.

3. The compound of claim 1 wherein Y is selected from the group consisting of null and NH; or a pharmaceutically acceptable salt or solvate thereof.

4. The compound of claim 1 wherein the A ring system is unsubstituted;or a pharmaceutically acceptable salt or solvate thereof.

5. The compound of claim 1 wherein the A ring system is selected from the group consisting of 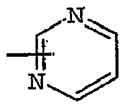 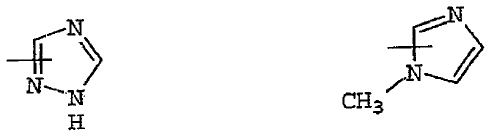 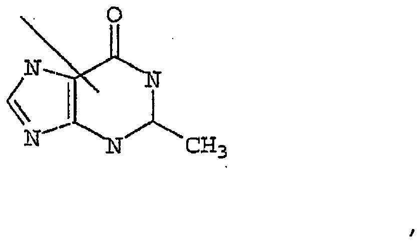 and 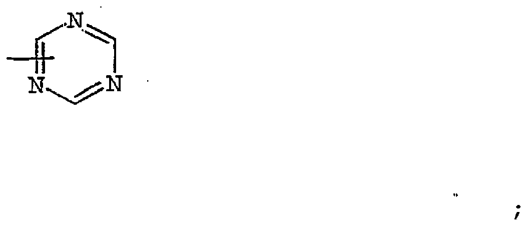 or a pharmaceutically acceptable salt or solvate thereof.

6. The compound of claim 1 wherein the A ring substituent is substituted with one to three substituents selected from the group consisting of N(R^{a})₂, halo, C₁₋₃alkyl, , S(C₁₋₃alkyl), OR^{a}, halo, and 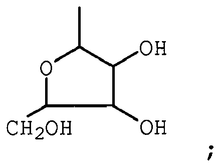 or a pharmaceutically acceptable salt or solvate thereof.

7. The compound of claim 1 wherein the A ring system is substituted with one to three substituents selected from the group consisting of NH₂, NH(CH₃), N(CH₃)₂, NHCH₂C₆H₅, NH(C₂H₅), Cl, F, CH₃, SCH₃, OH, and 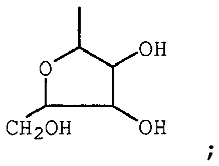 or a pharmaceutically acceptable salt or solvate thereof.

8. The compound of claim 1 wherein R¹ and R², are independently, selected from the group consisting of hydrogen, OR^{a}, halo, C₁₋₆alkyl, CF₃, NO₂, N(R^{a})₂, NR^{a}C₁₋₃alkyleneN(R^{a})₂, and OC₁₋₃alkyleneOR^{a}; or a pharmaceutically acceptable salt or solvate thereof.

9. The compound of claim 1 wherein R¹ and R² are independently selected from H, OCH₃, Cl, Br, F, CH₃, CF₃, NO₂, OH, N(CH3)₂, 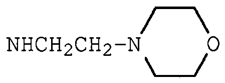 and O(CH₂)₂OCH₂C₆H₅; or a pharmaceutically acceptable salt or solvate thereof.

10. The compound of claim 1 wherein R¹ and R² are taken together to form a five- or six-membered ring; or a pharmaceutically acceptable salt or solvate thereof.

11. The compound of claim 1 wherein R³ is selected from the group consisting of C₁₋₆alkyl, aryl, heteroaryl, C₃₋₈cycloalkyl, C₃₋₈heterocycloalkyl, C(=O)OR^{a}, C₁₋₄alkyleneHet, C₁₋₄alkylenecycloalkyl, C₁₋₄alkylenearyl, C₁₋₄alkyleneC(=O)C₁₋₄alkylenearyl, C₁₋₄alkyleneC(=O)OR^{a}, C₁₋₄alkyleneC(=O)N(R^{a})₂, C₁₋₄alkyleneC(=O)Het, C₁₋₄alkyleneN(R^{a})₂, and C₁₋₄alkyleneNR^{a}C(=O)R^{a}; or a pharmaceutically acceptable salt or solvate thereof.

12. The compound of claim 1 wherein R³ is selected from the group consisting of OR^{a}, C₁₋₆alkyl, aryl, heteroaryl, NO₂, N(R^{a})₂, NR^{a}C(=O)R^{a}, C(=O)OC₂H₅, CH₂CH(CH₃)₂, 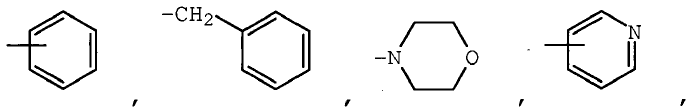 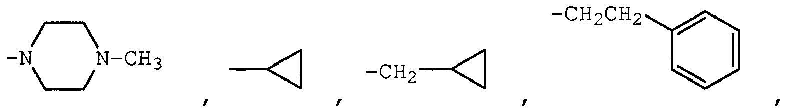 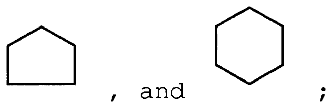 or a pharmaceutically acceptable salt or solvate thereof.

13. The compound of claim 1 wherein R³ is substituted with a substituent selected from the group consisting of halo, OR^{a}, C₁₋₆alkyl, aryl, heteroaryl, NO₂, N(R^{a})₂, NR^{a}SO₂CF₃, NR^{a}C(=O)R^{a}, C(=O)OR^{a}, SO₂N(R^{a})₂, CN, C(=O)R^{a}, C₁₋₄alkyleneN(R^{a})₂, OC₁₋₄alkyleneN(R^{a})₂, and N(R^{a})C₁₋₄alkyleneN(R^{a})₂; or a pharmaceutically acceptable salt or solvate thereof.

14. The compound of claim 5 wherein R³ is substituted with a substituent selected from the group consisting of Cl, F, CH₃, CH(CH₃)₂, OCH₃, C₆H₅, NO₂, NH₂, NHC(=O)CH₃, CO₂H, and N(CH₃)CH₂CH₂N(CH₃)_{2;} or a pharmaceutically acceptable salt or solvate thereof.

15. A pharmaceutical composition comprising a compound according to any preceding claim together with a pharmaceutically acceptable carrier.

16. A compound according to any one of claims 1 to 14 or a composition according to claim 15 for use in therapy.

17. A compound or composition according to claim 16 for use in a method of ameliorating a bone-resorption disorder in an animal.

18. A compound or composition according to claim 17 wherein said bone-resorption disorder is osteoporosis.

19. A compound or composition according to claim 16 for use in a method of inhibiting the growth or proliferation of chronic myelogenous leukemia cells.

20. A compound or composition according to claim 16 for the treatment of a disease selected from
i) ischemia, preferably resulting from cardiac arrest, myocardial infarction, obstruction of a coronary arteries, thromboembolytic occlusion of a cerebral vessel, traumatic head injury, edema and brain tumour,
ii) reperfusion injury, preferably associated with vascular stroke, hemorrhagic shock, myocardial ischemia or infarction, organ transplantation and cerebral vasospasm,
iii) bone diseases, preferably selected from osteoporosis, Paget's diseases and related bone resorption disorders,
iv) hematopoitic cancers, preferably;
a) lymphomas preferably selected from Burkitt's lymphoma, Hodgkins' lymphoma, non-Hodgkins lymphoma, lymphocytic lymphomas;
b) multiple myelomas
c) leukemias preferably selected from lymphocytic leukemias, chronic myeloid (myelogenous) leukemias,
v) diseases related to histamine release, preferably selected from chronic obstructive pulmonary disease (COPD), asthma, ARDS and emphysema,
vi) arthritic diseases, preferably selected from rheumatoid arthritis, monoarticular arthritis, osteoarthritis, gouty arthritis, spondylitis,
vii) Behcet disease,
viii) sepsis, septic shock, endotoxic shock, gram negative sepsis, gram positive sepsis and toxic shock syndrome,
ix) multiple organ injury syndrome secondary to septicemia, trauma or hemorrhage,
x) ophthalmic disorders, preferably selected from allergic conjunctivitis, vernal conjunctivitis, uveitis and thyroid-associated opthalmopathy,
xi) eosinophilic granuloma,
xii) pulmonary or respiratory disorders, preferably selected from asthma, chronic bronchitis, allergic rhinitis, ARDS, chronic pulmonary inflammatory disease, chronic obstructive pulmonary disease, silicosis, pulmonary sarcoidosis, pleurisy, alveolitis, vasculitis, emphysema, pneumonia, bronchiectasis and pulmonary oxygen toxicity,
xiii) fibrosis, preferably cystic fibrosis,
xiv) keloid formation or scar tissue formation,
xv) atherosclerosis
xvi) autoimmune diseases, preferably selected from systemic lupus erythematous (SLE), autoimmune thyroiditis, multiple sclerosis, some forms of diabetes and Reynaud's syndrome,
xvii) transplant rejection disorders, preferably selected from GVHD and allograft rejection,
xviii) chronic glomerulonephritis,
xix) inflammatory bowel diseases, preferably selected from chronic inflammatory bowel disease (CIBD), Crohn's disease, ulcerative colitis and necrotizing enterocolitis,
xx) inflammatory dermatoses, preferably selected from contact dermatitis, atopic dermatitis, psoriasis and urticaria,
xxi) fever and myalgias due to infection,
xxii) central or peripheral nervous system inflammatory disorders, preferably selected from meningitis, encephalitis and brain or spinal cord injury due to minor trauma,
xxiii) Sjögren's disease,
xxiv) diseases involving leukocyte diapedesis,
xxv) alcoholic hepatitis,
xxvi) bacterial pneumonia,
xxvii) antigen-antibody complex mediated diseases
xxviii) hypovolemic shock,
xxix) Type I diabetes mellitus,
xxx) acute and delayed hypersensitivity,
xxxi) disease states due to leukocyte dyscrasia and metastasis,
xxxii) thermal injury,
xxxiii) granulocyte transfusion-associated syndromes, and
xxxiv) cytokine-induced toxicity.

21. A compound or composition according to claim 16 for the treatment of a disease selected from the group consisting of leukemia, lymphoma and multiple myeloma.

22. A compound or composition according to claim 16 for the treatment of a disease selected from the group consisting of Burkitt's lymphoma, Hodgkin's lymphoma, non-Hodgkins lymphoma, lymphocytic lymphoma, multiple myeloma, lymphocytic leukemia, and chronic myeloid (myelognous) leukemia.

23. A compound or composition according to claim 16 for the treatment of a disease selected from the group consisting of COPD, asthma, rheumatoid arthritis, diabetes, allergic rhinitis, and multiple sclerosis.

## Patentansprüche

1. Verbindung mit der allgemeinen Strukturformel: 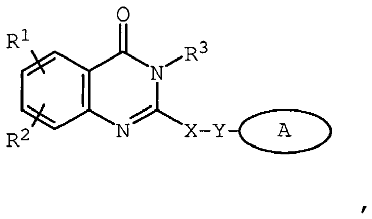
wobei das A-Ringsystem aus der aus Imidazolyl, Pyrazolyl, 1,2,3-Triazolyl, Pyridizinyl, Pyrimidinyl, Pyrazinyl, 1,3,5-Triazinyl, Cinnolinyl, Phtalazinyl, Chinazolinyl, Chinoxalinyl, 1,8-Naphthyridinyl, Pteridinyl, 1H-Indazolyl und Benzimidazolyl bestehenden Gruppe ausgewählt ist,
wobei diese Reste jeweils unsubstituiert oder durch einen bis drei aus der N(R^{a})₂, Halogen, C₁₋₃-Alkyl, S(C₁₋₃-Alkyl), OR^{a}, Halogen und 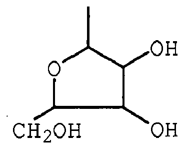
bestehenden Gruppe ausgewählte Substituenten substituiert sein können,
wobei X aus der aus CHR^{b}, CH₂CHR^{b} und CH=C(R^{b}) bestehenden Gruppe ausgewählt ist;
Y aus der aus null, SO, SO₂, NH, O, C(=O), OC(=O), C(=O)O und NHC(=O)CH₂S bestehenden Gruppe ausgewählt ist;
R¹ und R² unabhängig voneinander aus der aus Wasserstoff, C₁₋₆-Alkyl, Aryl, Heteroaryl, Halogen, NHC(=O)-C₁₋₃-Alkylen-N(R^{a})₂, NO₂, OR^{a}, OCF₃, N(R^{a})₂, CN, OC(=O)R^{a}, C(=O)R^{a}, C(=O)OR^{a}, Aryl-OR^{b}, Het, NR^{a}C(=O)-C₁₋₃-Alkylen-C(=O)OR^{a}, Aryl-O-C₁₋₃-alkylen-N(R^{a})₂, Aryl-OC(=O)R^{a}, C₁₋₄-Alkylen-C(=O)OR^{a}, O-C₁₋₄-Alkylen-C(=O)OR^{a}, C₁₋₄-Alkylen-O-C₁₋₄-alkylen-C(=O)OR^{a}, C(=O)NR^{a}SO₂R^{a}, C₁₋₄-Alkylen-N(R^{a})₂, C₂₋₆-Alkenylen-N(R^{a})₂, C(=O)-NR^{a}-C₁₋₄-Alkylen-OR^{a}, C(=O)NR^{a}-C₁₋₄-Alkylen-Het, O-C₂₋₄-Alkylen-N(R^{a})₂, O-C₁₋₄-Alkylen-CH(OR^{b})CH₂N(R^{a})₂, O-C₁₋₄-Alkylen-Het, O-C₂₋₄-Alkylen-OR^{a}, O-C₂₋₄-Alkylen-NR^{a}C(=O)OR^{a}, NR^{a}-C₁₋₄-Alkylen-N(R^{a})₂, NR^{a}C(=O)R^{a}, NR^{a}C(=O)N(R^{a})₂, N(SO₂-C₁₋₄-Alkyl)₂, NR^{a}(SO₂-C₁₋₄-Alkyl), SO₂N(R^{a})₂, OSO₂CF₃, C₁₋₃-Alkylenaryl, C₁₋₄-Alkylen-Het, C₁₋₆-Alkylen-OR^{b}, C₁₋₃-Alkylen-N(R^{a})₂, C(=O)N(R^{a})₂, NHC(=O)-C₁₋₃-Alkylenaryl, C₃₋₈-Cycloalkyl, C₃₋₈-Heterocycloalkyl, Aryl-OC(=O)R^{b}, NHC(=O)-C₁₋₃-Alkylen-C₃₋₈-heterocycloalkyl, NHC(=O)-C₁₋₃-Alkylen-Het, O-C₁₋₄-Alkylen-O-C₁₋₄-alkylen-C(=O)OR^{b}, C(=O)-C₁₋₄-Alkylen-Het und NHC(=O)-Halogen-C₁₋₆-alkyl bestehenden Gruppe ausgewählt sind;
oder R¹ und R² zusammen eine 3- oder 4-gliedrige Alkylen- oder Alkenylen-Kettenkomponente eines 5- oder 6-gliedrigen Rings, welcher gegebenenfalls mindestens ein Heteroatom enthält, bilden;
R³ für Wasserstoff steht oder aus der aus C₁₋₆-Alkyl, C₃₋₈-Cycloalkyl, C₃₋₈-Heterocycloalkyl, C₁₋₄-Alkylencycloalkyl, C₂₋₆-Alkenyl, C₁₋₃-Alkylenaryl, Aryl-C₁₋₃-alkyl, C(=O)R^{a}, Aryl, Heteroaryl, C(=O)OR^{a}, C(=O)N(R^{a})₂, C(=S)N(R^{a})₂, SO₂R^{a}, SO₂N(R^{a})₂, S(=O)R^{a}, S(=O)N(R^{a})₂, C(=O)NR^{a}-C₁₋₄-Alkylen-OR^{a}, C(=O)NR^{a}-C₁₋₄-Alkylen-Het, C(=O)-C₁₋₄-Alkylenaryl, C(=O)-C₁₋₄-Alkylenheteroaryl, C₁₋₄-Alkylenheteroaryl, C₁₋₄-Alkylen-Het, C₁₋₄-Alkylen-C(=O)-C₁₋₄-alkylenaryl, C₁₋₄-Alkylen-C(=O)-C₁₋₄-Alkylenheteroaryl, C₁₋₄-Alkylen-C(=O)-Het, C₁₋₄-Alkylen-C(=O)N(R^{a})₂, C₁₋₄-Alkylen-OR^{a}, C₁₋₄-Alkylen-NR^{a}C(=O)R^{a}, C₁₋₄-Alkylen-O-C₁₋₄-alkylen-OR^{a}, C₁₋₄-Alkylen-N(R^{a})₂, C₁₋₄-Alkylen-C(=O)OR^{a} und C₁₋₄-Alkylen-O-C₁₋₄-alkylen-C(=O)OR^{a} bestehenden Gruppe ausgewählt ist, wobei diese Reste jeweils gegebenenfalls durch einen aus der aus Halogen, OR^{a}, C₁₋₆-Alkyl, Aryl, Heteroaryl, NO₂, N(R^{a})₂, NR^{a}SO₂CF₃, NR^{a}C(=O)R^{a}, C(=O)OR^{a}, SO₂N(R^{a})₂, CN, C(=O)R^{a}, C₁₋₄-Alkylen-N(R^{a})₂, O-C₁₋₄-Alkylen-N(R^{a})₂ und N(R^{a})-C₁₋₄-Alkylen-N(R^{a})₂ bestehenden Gruppe ausgewählten Substituenten substituiert sind; oder
R³ für C₁₋₄-Alkylenaryl, gegebenenfalls substituiert durch einen oder mehrere Halogene, SO₂N(R^{a})₂, N(R^{a})₂, C(=O)OR^{a}, NR^{a}SO₂CF₃, CN, NO₂, C(=O)R^{a}, OR^{a}, C₁₋₄-Alkylen-N(R^{a})₂ und O-C₁₋₄-Alkylen-N(R^{a})₂ steht;
R^{a} aus der aus Wasserstoff, C₁₋₆-Alkyl, C₃₋₈-Cycloalkyl, C₃₋₈-Heterocycloalkyl, C₁₋₃-Alkylen-N(R^{a})₂, Aryl, Aryl-C₁₋₃-alkyl, C₁₋₃-Alkylenaryl, Heteroaryl, Heteroaryl-C₁₋₃-alkyl und C₁₋₃Alkylenheteroaryl bestehenden Gruppe ausgewählt ist;
oder zwei R^{a}-Gruppen zusammen einen 5- oder 6-gliedrigen Ring bilden, der gegebenenfalls mindestens ein Heteroatom enthält;
R^{b} aus der aus Wasserstoff, C₁₋₆-Alkyl, Aryl, Heteroaryl, Aryl-C₁₋₃-alkyl, Heteroaryl-C₁₋₃-alkyl, C₁₋₃-Alkylenaryl und C₁₋₃-Alkylenheteroaryl bestehenden Gruppe ausgewählt ist;
Het für einen 5- oder 6-gliedrigen, gesättigten oder teilweise oder vollständig ungesättigten heterocyclischen Ring mit mindestens einem aus der aus Sauerstoff, Stickstoff und Schwefel bestehenden Gruppe ausgewählten Heteroatom steht, welcher gegebenenfalls durch C₁₋₄-Alkyl oder C(=O)OR^{a} substituiert ist;
und pharmazeutisch unbedenkliche Salze und Solvate davon,
wobei "Aryl" als eine monocyclische oder polycyclische aromatische Gruppe definiert ist, die unsubstituiert oder durch einen oder mehrere und insbesondere ein bis drei Halogen-, Alkyl-, Phenyl-, Hydroxyalkyl-, Alkoxy-, Alkoxyalkyl-, Halogenalkyl-, Nitro-, Amino-, Alkylamino-, Acylamino-, Alkylthio-, Alkylsulfinyl- und Alkylsulfonylreste substituiert sein kann, und wobei "Heteroaryl" als ein monocyclisches oder bicyclisches Ringsystem mit einem oder zwei aromatischen Ringen und mit mindestens einem Stickstoff-, Sauerstoff- oder Schwefelatom in einem aromatischen Ring definiert ist, welches unsubstituiert oder durch einen oder mehrere, insbesondere ein bis drei, Halogen-, Alkyl-, Hydroxy-, Hydroxyalkyl-, Alkoxy-, Alkoxyalkyl-, Halogenalkyl-, Nitro-, Amino-, Alkylamino-, Acylamino-, Alkylthio-, Alkylsulfinyl- und Alkylsulfonylreste substituiert sein kann.

2. Verbindung nach Anspruch 1, wobei X aus der aus CH₂, CH₂CH₂, CH=CH, CH(CH₃), CH₂CH(CH₃) und C(CH₃)₂ bestehenden Gruppe ausgewählt ist; oder ein pharmazeutisch unbedenkliches Salz oder Solvat davon.

3. Verbindung nach Anspruch 1, wobei Y aus der aus null und NH bestehenden Gruppe ausgewählt ist; oder ein pharmazeutisch unbedenkliches Salz oder Solvat davon.

4. Verbindung nach Anspruch 1, wobei das A-Ringsystem unsubstituiert ist; oder ein pharmazeutisch unbedenkliches Salz oder Solvat davon.

5. Verbindung nach Anspruch 1, wobei das A-Ringsystem aus der aus 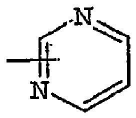 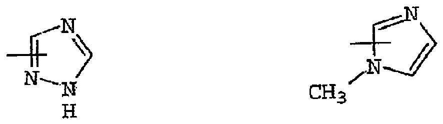 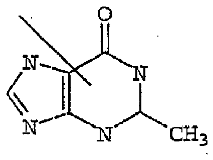 und 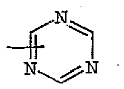 bestehenden Gruppe ausgewählt ist; oder ein pharmazeutisch unbedenkliches Salz oder Solvat davon.

6. Verbindung nach Anspruch 1, wobei der A-Ringsubstituent durch einen bis drei aus der aus N(R^{a})₂, Halogen, C₁₋₃-Alkyl, S-C₁₋₃-Alkyl, OR^{a}, Halogen und 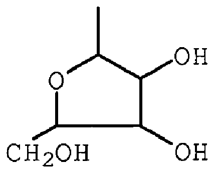 bestehenden Gruppe ausgewählte Substituenten substituiert ist; oder ein pharmazeutisch unbedenkliches Salz oder Solvat davon.

7. Verbindung nach Anspruch 1, wobei das A-Ringsystem durch einen bis drei aus der aus NH₂, NH(CH₃), N(CH₃)₂, NHCH₂C₆H₅, NH(C₂H₅), Cl, F, CH₃, SCH₃, OH und 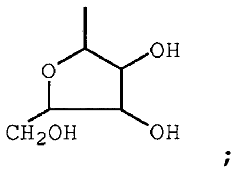 bestehenden Gruppe ausgewählte Substituenten substituiert ist; oder ein pharmazeutisch unbedenkliches Salz oder Solvat davon.

8. Verbindung nach Anspruch 1, wobei R¹ und R² unabhängig voneinander aus der aus Wasserstoff, OR^{a}, Halogen, C₁₋₆-Alkyl, CF₃, NO₂, N(R^{a})₂, NR^{a}-C₁₋₃-Alkylen-N(R^{a})₂ und O-C₁₋₃-Alkylen-OR^{a} bestehenden Gruppe ausgewählt sind; oder ein pharmazeutisch unbedenkliches Salz oder Solvat davon.

9. Verbindung nach Anspruch 1, wobei R¹ und R² unabhängig voneinander aus H, OCH₃, Cl, Br, F, CH₃, CF₃, NO₂, OH, N(CH₃)₂, 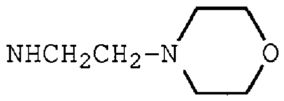 und O(CH₂)₂OCH₂C₆H₅ ausgewählt sind; oder ein pharmazeutisch unbedenkliches Salz oder Solvat davon.

10. Verbindung nach Anspruch 1, wobei R¹ und R² zusammen einen 5- oder 6-gliedrigen Ring bilden; oder ein pharmazeutisch unbedenkliches Salz oder Solvat davon.

11. Verbindung nach Anspruch 1, wobei R³ aus der aus C₁₋₆-Alkyl, Aryl, Heteroaryl, C₃₋₈-Cycloalkyl, C₃₋₈-Heterocycloalkyl, C(=O)OR^{a}, C₁₋₄-Alkylen-Het, C₁₋₄-Alkylencycloalkyl, C₁₋₄-Alkylenaryl, C₁₋₄-Alkylen-C(=O)-C₁₋₄-alkylenaryl, C₁₋₄-Alkylen-C(=O)OR^{a}, C₁₋₄-Alkylen-C(=O)N(R^{a})₂, C₁₋₄-Alkylen-C(=O)-Het, C₁₋₄-Alkylen-N(R^{a})₂ und C₁₋₄-Alkylen-NR^{a}C(=O)R^{a} bestehenden Gruppe ausgewählt ist; oder ein pharmazeutisch unbedenkliches Salz oder Solvat davon.

12. Verbindung nach Anspruch 1, wobei R³ aus der aus OR^{a}, C₁₋₆-Alkyl, Aryl, Heteroaryl, NO₂, N(R^{a})₂, NR^{a}C(=O)R^{a}, C=(O)OC₂H₅, CH₂CH(CH₃)₂ 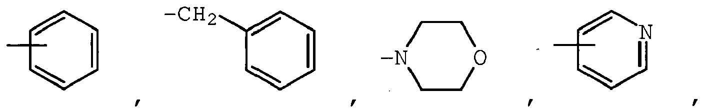 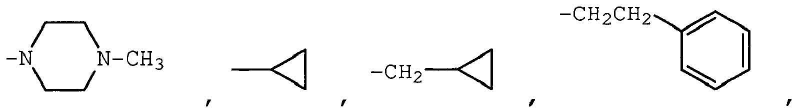 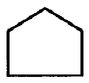 und 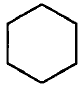 bestehenden Gruppe ausgewählt ist; oder ein pharmazeutisch unbedenkliches Salz oder Solvat davon.

13. Verbindung nach Anspruch 1, wobei R³ durch einen aus der aus Halogen, OR^{a}, C₁₋₆-Alkyl, Aryl, Heteroaryl, NO₂, N(R^{a})₂, NR^{a}SO₂CF₃, NR^{a}C(=O)R^{a}, C(=O)OR^{a}, SO₂N(R^{a})₂, CN, C(=O)R^{a}, C₁₋₄-Alkylen-N(R^{a})₂, O-C₁₋₄-Alkylen-N(R^{a})₂ und N(R^{a})-C₁₋₄-Alkylen-N(R^{a})₂ bestehenden Gruppe ausgewählten Substituenten substituiert ist; oder ein pharmazeutisch unbedenkliches Salz oder Solvat davon.

14. Verbindung nach Anspruch 5, wobei R³ durch einen aus der aus Cl, F, CH₃, CH(CH₃)₂, OCH₃, C₆H₅, NO₂, NH₂, NHC(=O)CH₃, CO₂H und N(CH₃)CH₂CH₂N(CH₃)₂ bestehenden Gruppe ausgewählten Substituenten substituiert ist; oder ein pharmazeutisch unbedenkliches Salz oder Solvat davon.

15. Pharmazeutische Zusammensetzung, umfassend eine Verbindung nach einem der vorhergehenden Ansprüche zusammen mit einem pharmazeutisch unbedenklichen Träger.

16. Verbindung nach einem der Ansprüche 1 bis 14 oder Zusammensetzung nach Anspruch 15 zur Verwendung in der Therapie.

17. Verbindung oder Zusammensetzung nach Anspruch 16 zur Verwendung bei einem Verfahren zur Linderung einer Knochenresorptionsstörung in einem Tier.

18. Verbindung oder Zusammensetzung nach Anspruch 17, wobei es sich bei der Knochenresorptionsstörung um Osteoporose handelt.

19. Verbindung oder Zusammensetzung nach Anspruch 16 zur Verwendung bei einem Verfahren zum Inhibieren des Wachstums bzw. der Proliferation von chronischen myeloischen Leukämiezellen.

20. Verbindung oder Zusammensetzung nach Anspruch 16 zur Behandlung einer Krankheit ausgewählt aus:
i) Ischämie, vorzugsweise als Folge von Herzstillstand, Herzinfarkt, einer Obstruktion von Koronararterien, thromboembolytischer Okklusion eines zerebralen Gefäßes, traumatischer Kopfverletzung, Ödem und Gehirntumor,
ii) Reperfusionsverletzung, vorzugsweise assoziiert mit vaskulärem Schlaganfall, hämorrhagischem Schock, myokardialer Ischämie oder Herzinfarkt, Organtransplantation und zerebralem Vasospasmus,
iii) Knochenkrankheiten, vorzugsweise ausgewählt aus Osteoporose, Morbus Paget und verwandten Knochenresorptionsstörungen,
iv) hämatopoetischen Krebserkrankungen, vorzugsweise:
a) Lymphomen, vorzugsweise ausgewählt aus Burkitt-Lymphom, Hodgkin-Lymphom, Non-Hodgkin-Lymphom, lymphozytischen Lymphomen,
b) multiplen Myelomen,
c) Leukämien, vorzugsweise ausgewählt aus lymphozytischen Leukämien, chronischen myeloischen (myelogenen) Leukämien,
v) mit einer Histaminfreisetzung in Zusammenhang stehenden Krankheiten, vorzugsweise ausgewählt aus chronischer obstruktiver Atemwegserkrankung (Chronic Obstructive Pulmonary Disease, COPD), Asthma, ARDS und Emphysem,
vi) arthritischen Krankheiten, vorzugsweise ausgewählt aus rheumatoider Arthritis, monoartikulärer Arthritis, Osteoarthritis, Gichtarthritis, Spondilitis,
vii) Morbus Becet,
viii) Sepsis, septischem Schock, endotoxischem Schock, gramnegativer Sepsis, grampositiver Sepsis und toxischem Schocksyndrom,
ix) Multiorganversagen als Folge von Septikämie, Trauma oder Blutungen,
x) ophthalmischen Erkrankungen, vorzugsweise ausgewählt aus allergischer Konjunktivitis, vernaler Konjunktivitis, Uveitis und schilddrüsenassoziierter Ophthalmopathie,
xi) eosinophilem Granulom,
xii) Lungen- oder Atemwegserkrankungen, vorzugsweise ausgewählt aus Asthma, chronischer Bronchitis, allergischer Rhinitis, ARDS, chronischer entzündlicher Atemwegserkrankung, chronischer obstruktiver Atemwegserkrankung, Silikose, pulmonaler Sarkoidose, Pleuritis, Alveolitis, Vaskulitis, Emphysem, Pneumonie, Bronchiektasie und pulmonaler Sauerstofftoxizität,
xiii) Fibrose, vorzugsweise zystische Fibrose,
xiv) Keloidbildung oder Narbengewebebildung,
xv) Atherosklerose,
xvi) Autoimmunkrankheiten, vorzugsweise ausgewählt aus systemischem Lupus erythematodes (SLE), Autoimmunthyreoiditis, multipler Sklerose, einigen Formen von Diabetes und Reynaud-Syndrom,
xvii) Transplantatabstoßungserkrankungen, vorzugsweise ausgewählt aus GVHD und Allograftabstoßung,
xviii) chronischer Glomerulonephritis,
xix) entzündlichen Darmkrankheiten, vorzugsweise ausgewählt aus chronischer entzündlicher Darmkrankheit (Chronic Inflammatory Bowle Disease, CIBD), Morbus Chron, Colitis ulcerosa und nekrotisierender Enterokolitis,
xx) entzündlichen Dermatosen, vorzugsweise ausgewählt aus Kontaktdermatitis, atopischer Dermatitis, Psoriasis und Urtikaria,
xxi) Fieber und Myalgien aufgrund von Infektionen,
xxii) entzündlichen Erkrankungen des zentralen oder peripheren Nervensystems, vorzugsweise ausgewählt aus Meningitis, Enzephalitis und Hirn- oder Rückenmarksverletzungen aufgrund kleinerer Traumata,
xxiii) Sjögren-Krankheit,
xxiv) Krankheiten mit Leukodiapedese,
xxv) alkoholischer Hepatitis,
xxvi) bakterieller Pneumonie,
xxvii) Antigen-Antikörper-Komplex-vermittelter Krankheiten,
xxviii) hypovolämischen Schock,
xxix) Diabetes mellitus vom Typ I,
xxx) akuter und retardierter Hypersensibilität,
xxxi) Krankheitszustände aufgrund von Leukozytendyskrasie und Metastasenbildung,
xxxii) thermalen Verletzungen,
xxxiii) mit Granulozytentransfusion assoziierten Syndromen und
xxxiv) zytokininduzierter Toxizität.

21. Verbindung oder Zusammensetzung nach Anspruch 16 zur Behandlung einer Krankheit ausgewählt aus der Gruppe bestehend aus Leukämie, Lymphom und multiplem Myelom.

22. Verbindung oder Zusammensetzung nach Anspruch 16 zur Behandlung einer Krankheit ausgewählt aus der Gruppe bestehend aus Burkitt-Lymphom, Hodgkin-Lymphom, Non-Hodgkin-Lymphom, lymphozytischem Lymphom, multiplem Myelom, lymphozytischer Leukämie und chronischer myeloischer (myelogener) Leukämie.

23. Verbindung oder Zusammensetzung nach Anspruch 16 zur Behandlung einer Krankheit ausgewählt aus der Gruppe bestehend aus COPD, Asthma, rheumatoider Arthritis, Diabetes, allergischer Rhinitis und multipler Sklerose.

## Revendications

1. Composé ayant la formule développée générale : 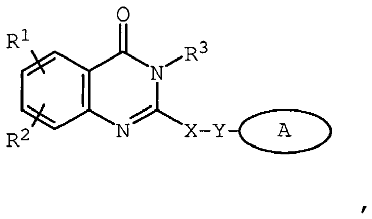
dans laquelle le système cyclique A est choisi dans le groupe consistant en les groupes imidazolyle, pyrazolye, 1,2,3-triazoyle, pyridizinyle, pyrimidinyle, pyrazinyle, 1,3,5-triazinyle, cinnolinyle, phtalazinyle, quinazolinyle, quinoxalinyle, 1,8-naphtyridinyle, ptéridinyle, 1H-indazolyle et benzimidazolyle,
dont chacun peut être non substitué, ou substitué par un à trois substituants choisis dans le groupe consistant en les substituants N(R^{a})₂, halogéno, alkyle en C₁₋₃, S (alkyle en C₁₋₃), OR^{a}, halogéno, et 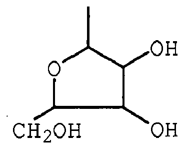
où X est choisi dans le groupe consistant en CHR^{b}, CH₂CHR^{b} et CH=C(R^{b});
Y est choisi dans le groupe consistant en rien, SO, SO₂, NH, 0, C(=O), OC(=O), C(=O)O, et NHC(=O)CH₂S ;
R¹ et R² sont chacun indépendamment de l'autre choisis dans le groupe consistant en l'atome d'hydrogène, les groupes alkyle en C₁₋₆, aryle, hétéroaryle, halogéno, NHC(=O)(alkylène en C₁₋₃)N(R^{a})₂, NO₂, OR^{a}, OCF₃, N(R^{a})₂, CN, OC(=O)R^{a}, C(=O)R^{a}, C(=O)OR^{a}, aryl-OR^{b}, Het, NR^{a}C(=O) (alkylène en C₁₋₃)C(=O)OR^{a}, aryl-O(alkylène en C₁₋₃)N(R^{a})₂, aryl-OC(=O)R^{a}, (alkylène en C₁₋₄)C(=O)OR^{a}, O(alkylène en C₁₋₄)C(=O)OR^{a}, (alkylène en C₁₋₄)O(alkylène en C₁₋₄)C(=O)OR^{a}, C(=O)NR^{a}SO₂R^{a}, (alkylène en C₁₋₄)N(R^{a})₂, (alcénylène en C₂₋₆)N(R^{a})₂, C(=O)-NR^{a} (alkylène en C₁₋₄)OR^{a}, C(=O)NR^{a} (alkylène en C₁₋₄)Het, 0 (alkylène en C₂₋₄)N(R^{a})₂, O(alkylène en C₁₋₄)CH(OR^{b})CH₂N(R^{a})₂, O(alkylène en C₁₋₄)Het, 0 (alkylène en C₂₋₄) OR^{a}, 0 (alkylène en C₂₋₄)NR^{a}C(=O)OR^{a}, NR^{a} (alkylène en C₁₋₄)N(R^{a})₂, NR^{a}C(=O)R^{a}, NR^{a}C(=O)N(R^{a})₂, N(SO₂(alkyle en C₁₋₄))₂, NR^{a}(SO₂(alkyle en C₁₋₄)), SO₂N(R^{a})₂, OSO₂CF₃, (alkylène en C₁₋₃) aryle, alkylène en C₁₋₄) Het, (alkylène en C₁₋₆)OR^{b}, (alkylène en C₁₋₃)N(R^{a})₂, C(=O)N(R^{a})₂, NHC(=O) (alkylène en C₁₋₃) aryle, cycloalkyle en C₃₋₈, hétérocycloalkyle en C₃₋₈, aryl-OC(=O)R^{b}, NHC (=0) (alkylène en C₁₋₃) (hétérocycloalkyle en C₃₋₈), NHC(=O)(alkylène en C₁₋₃)Het, 0 (alkylène en C₁₋₄)O(alkylène en C₁₋₄)C(=O)OR^{b}, C(=O)(alkylène en C₁₋₄)Het, et NHC (=0) halogéno (alkyle en C₁₋₆);
ou R¹ et R² sont pris ensemble pour former un composant chaîne alkylène ou alcénylène à 3 ou 4 chaînons d'un cycle à 5 ou 6 chaînons, en option contenant au moins un hétéroatome ;
R³ est un atome d'hydrogène ou est choisi dans le groupe consistant en les groupes alkyle en C₁₋₆, cycloalkyle en C₃₋₈, hétérocycloalkyle en C₃₋₈, (alkylène en C₁₋₄)cycloalkyle, alcényle en C₂₋₆, (alkylène en C₁₋₃)aryle, aryl (alkyle en C₁₋₃), C(=O)R^{a}, aryle, hétéroaryle, C(=O)OR^{a}, C(=O)N(R^{a})₂, C(=S)N(R^{a})₂, SO₂R^{a}, SO₂N(R^{a})₂, S(=O)R^{a}, S(=O)N(R^{a})₂, C(=O)NR^{a}(alkylène en C₁₋₄)OR^{a}, C(=O)NR^{a} (alkylène en C₁₋₄)Het, C(=O)(alkylène en C₁₋₄) aryle, C(=O) (alkylène en C₁₋₄)hétéroaryle, (alkylène en C₁₋₄)hétéroaryle, (alkylène en C₁₋₄)Het, (alkylène en C₁₋₄)C(=O)(alkylène en C₁₋₄)aryle, (alkylène en C₁₋₄)C(=O)(alkylène en C₁₋₄)hétéroaryle, (alkylène en C₁₋₄)C(=O)Het, (alkylène en C₁₋₄)C(=O)N(R^{a})₂, (alkylène en C₁₋₄)OR^{a}, (alkylène en C₁₋₄)NR^{a}C(=O)R^{a}, (alkylène en C₁₋₄)O(alkylène en C₁₋₄)OR^{a}, (alkylène en C₁₋₄)N(R^{a})₂, (alkylène en C₁₋₄)C(=O)OR^{a}, et (alkylène en C₁₋₄)O(alkylène en C₁₋₄)C(=O)OR^{a}, dont chacun est en option substitué par un substituant choisi dans le groupe consistant en les substituants halogéno, OR^{a}, alkyle en C₁₋₆, aryle, hétéroaryle, NO₂, N(R^{a})₂, NR^{a}SO₂CF₃, NR^{a}C(=O)R^{a}, C(=O)OR^{a}, SO₂N(R^{a})₂, CN, C(=O)R^{a}, (alkylène en C₁₋₄)N(R^{a})₂, O(alkylène en C₁₋₄)N(R^{a})₂ et N(R^{a})(alkylène en C₁₋₄)N(R^{a})₂; ou
R³ est un groupe (alkylène en C₁₋₄)aryle en option substitué par un ou plusieurs substituants halogéno, SO₂N(R^{a})₂, N(R^{a})₂, C(=O)OR^{a}, NR^{a}SO₂CF₃, CN, NO₂, C(=O)R^{a}, OR^{a}, (alkylène en C₁₋₄)N(R^{a})₂ et 0 (alkylène en C₁₋₄)N(R^{a})₂*;*
R^{a} est choisi dans le groupe consistant en l'atome d'hydrogène, les groupes alkyle en C₁₋₆, cycloalkyle en C₃₋₈, hétérocycloalkyle en C₃₋₈, (alkylène en C₁₋₃)N(R^{a})₂, aryle, aryl (alkyle en C₁₋₃), (alkylène en C₁₋₃)aryle, hétéroaryle, hétéroaryl(alkyle en C₁₋₃) et (alkylène en C₁₋₃)hétéroaryle ;
ou deux groupes R^{a} sont pris ensemble pour former un cycle à 5 ou 6 chaînons, contenant en option au moins un hétéroatome ;
R^{b} est choisi dans le groupe consistant en l'atome d'hydrogène, les groupes alkyle en C₁₋₆, aryle, hétéroaryle, aryl(alkyle en C₁₋₃), hétéroaryl(alkyle en C₁₋₃), (alkylène en C₁₋₃) aryle, et (alkylène en C₁₋₃)hétéroaryle ;
Het est un noyau hétérocyclique à 5 ou 6 chaînons, saturé ou partiellement ou entièrement insaturé, contenant au moins un hétéroatome choisi dans le groupe consistant en l'oxygène, l'azote et le soufre, et en option substitué par un ou des substituants alkyle en C₁₋₄ ou C(=O)OR^{a} ;
et ses sels et solvates pharmaceutiquement acceptables, où "aryle" est défini comme un groupe aromatique monocyclique ou polycyclique qui peut être non substitué, ou substitué par un ou plusieurs, en particulier un à trois, substituants halogéno, alkyle, phényle, hydroxyalkyle, alcoxy, alcoxyalkyle, halogènalkyle, nitro, amino, alkylamino, acylamino, alkylthio, alkylsulfinyle et alkylsulfonyle, et où "hétéroaryle" est défini comme un système cyclique monocyclique ou bicyclique contenant un ou deux noyaux aromatiques et contenant au moins un atome d'azote, d'oxygène ou de soufre dans le noyau aromatique, qui peut être non substitué ou substitué par un ou plusieurs, en particulier un à trois substituants halogéno, alkyle, hydroxy, hydroxyalkyle, alcoxy, alcoxyalkyle, halogènalkyle, nitro, amino, alkylamino, acylamino, alkylthio, alkylsulfinyle et alkylsulfonyle.

2. Composé selon la revendication 1, dans lequel X est choisi dans le groupe consistant en CH₂, CH₂CH₂, CH=CH, CH(CH₃), CH₂CH(CH₃) et C(CH₃)₂ ; ou sel ou solvate pharmaceutiquement acceptable de ce composé.

3. Composé selon la revendication 1, dans lequel Y est choisi dans le groupe consistant en rien et NH ; ou un sel ou solvate pharmaceutiquement acceptable de ce composé.

4. Composé selon la revendication 1, dans lequel le système cyclique A est non substitué ; ou sel ou solvate pharmaceutiquement acceptable de ce composé.

5. Composé selon la revendication 1, dans lequel le système cyclique A est choisi dans le groupe consistant en 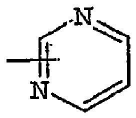 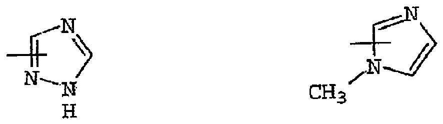 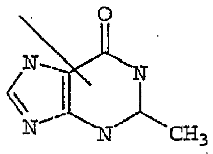 et 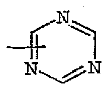 ou sel ou solvate pharmaceutiquement acceptable de ce composé.

6. Composé selon la revendication 1, dans lequel le substituant cyclique A est substitué par un ou trois substituants choisis dans le groupe consistant en les substituants N(R^{a})₂, halogéno, alkyle en C₁₋₃, S (alkyle en C₁₋₃), OR^{a}, halogéno, et 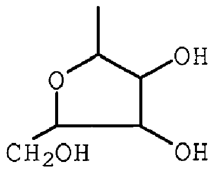 ou sel ou solvate pharmaceutiquement acceptable de ce composé.

7. Composé selon la revendication 1, dans lequel le système cyclique A est substitué par un à trois substituants choisis dans le groupe consistant en les substituants NH₂, NH(CH₃), N(CH₃)₂, NHCH₂C₆H₅, NH(C₂H₅), Cl, F, CH₃, SCH₃, OH, et 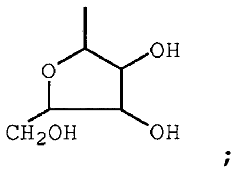 sel ou solvate pharmaceutiquement acceptable de ce composé.

8. Composé selon la revendication 1, dans lequel R¹ et R² sont indépendamment l'un de l'autre choisis dans le groupe consistant en l'atome d'hydrogène, les groupes OR^{a}, halogéno, alkyle en C₁₋₆, CF₃, NO₂, N(R^{a})₂, NR^{a}(alkylène en C₁₋₃)N(R^{a})₂ et 0 (alkylène en C₁₋₃)OR^{a} ; ou sel ou solvate pharmaceutiquement acceptable de ce composé.

9. Composé selon la revendication 1, dans lequel R¹ et R² sont choisis chacun indépendamment de l'autre parmi H, OCH₃, Cl, Br, F, CH₃, CF₃, NO₂, OH, N(CH₃)₂, 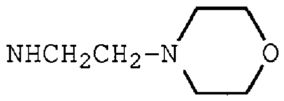 et O(CH₂)₂OCH₂C₆H₅ ; ou sel ou solvate pharmaceutiquement acceptable de ce composé.

10. Composé selon la revendication 1, dans lequel R¹ et R² sont pris ensemble pour former un cycle à cinq ou six chaînons ; ou sel ou solvate pharmaceutiquement acceptable de ce composé.

11. Composé selon la revendication 1, dans lequel R³ est choisi dans le groupe consistant en les groupes alkyle en C₁₋₆, aryle, hétéroaryle, cycloalkyle en C₃₋₈, hétérocycloalkyle en C₃₋₈, C(=O)OR^{a}, (alkylène en C₁₋₄)Het, (alkylène en C₁₋₄)cycloalkyle, (alkylène en C₁₋₄) aryle, (alkylène en C₁₋₄)C(=O)(alkylène en C₁₋₄) aryle, (alkylène en C₁₋₄)C(=O)OR^{a}, (alkylène en C₁₋₄)C(=O)N(R^{a})**₂,** (alkylène en C₁₋₄)C(=O)Het, (alkylène en C₁₋₄)N(R^{a})₂ et (alkylène en C₁₋₄)NR^{a}C(=O)R^{a} ; ou sel ou solvate pharmaceutiquement acceptable de ce composé.

12. Composé selon la revendication 1, dans lequel R³ est choisi dans le groupe consistant en les groupes OR^{a}, alkyle en C₁₋₆, aryle, hétéroaryle, NO₂, N(R^{a})₂, NR^{a}C(=O)R^{a}, C=(O)OC₂H₅, CH₂CH(CH₃)₂ 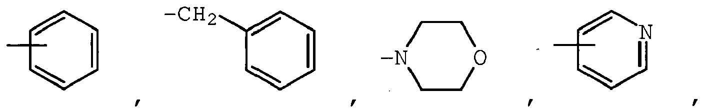 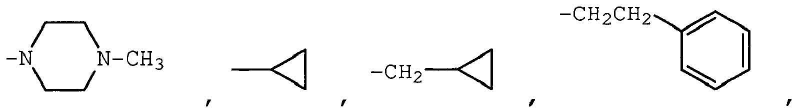 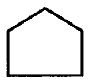 et 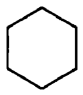 ou sel ou solvate pharmaceutiquement acceptable de ce composé.

13. Composé selon la revendication 1, dans lequel R³ est substitué par un substituant choisi dans le groupe consistant en les substituants halogéno, OR^{a}, alkyle en C₁₋₆, aryle, hétéroaryle, NO₂, N(R^{a})₂, NR^{a}SO₂CF₃, NR^{a}C(=O)R^{a}, C(=O)OR^{a}, SO₂N(R^{a})₂, CN, C(=O)R^{a}, (alkylène en C₁₋₄)N(R^{a})₂, O(alkylène en C₁₋₄)N(R^{a})₂, et N(R^{a}) (alkylène en C₁₋₄)N(R^{a})₂; ou sel ou solvate pharmaceutiquement acceptable de ce composé.

14. Composé selon la revendication 5, dans lequel R³ est substitué par un substituant choisi dans le groupe consistant en les substituants Cl, F, CH₃, CH(CH₃)₂, OCH₃, C₆H₅, NO₂, NH₂, NHC(=O)CH₃, CO₂H et N(CH₃)CH₂CH₂N(CH₃)₂ ; ou sel ou solvate pharmaceutiquement acceptable de ce composé.

15. Composition pharmaceutique comprenant un composé selon l'une quelconque des revendications précédentes en même temps qu'un support pharmaceutiquement acceptable.

16. Composé selon l'une quelconque des revendications 1 à 14 ou composition selon la revendication 15 pour utilisation en thérapie.

17. Composé ou composition selon la revendication 16 pour utilisation dans un procédé d'amélioration d'un trouble de la résorption osseuse chez un animal.

18. Composé ou composition selon la revendication 17, ledit trouble de la résorption osseuse étant l'ostéoporose.

19. Composé ou composition selon la revendication 16, pour utilisation dans un procédé d'inhibition de la croissance ou de la prolifération de cellules de leucémie myélogène chronique.

20. Composé ou composition selon la revendication 16 pour le traitement d'une maladie choisie parmi :
i) l'ischémie, résultant de préférence d'un arrêt cardiaque, d'un infarctus du myocarde, de l'obstruction d'une artère coronaire, d'une occlusion thromboembolytique d'un vaisseau cérébral, d'une lésion traumatique de la tête, d'un oedème et d'une tumeur cérébrale,
ii) la lésion de reperfusion, de préférence associée à un accident vasculaire cérébral, un choc hémorragique, une ischémie myocardique ou un infarctus, une transplantation d'organes et un vasospasme cérébral,
iii) les maladies osseuses, de préférence choisies parmi l'ostéoporose, les maladies de Paget et les troubles associés de la résorption osseuse,
iv) les cancers hématopoïétiques, de préférence :
a) les lymphomes, de préférence choisis parmi le lymphome de Burkitt, le lymphome de Hodgkin, le lymphome non hodgkinien, les lymphomes lymphocytiques ;
b) les myélomes multiples,
c) les leucémies, de préférence choisies parmi les leucémies lymphocytiques, les leucémies myéloïdes (myélogènes) chroniques,
v) les maladies associées à la libération de l'histamine, de préférence choisies parmi la bronchopneumopathie chronique obstructive (BPCO), l'asthme, le SDRA et l'emphysème,
vi) les maladies arthritiques, de préférence choisies parmi la polyarthrite rhumatoïde, l'arthrite mono-articulaire, l'ostéoarthrite, l'arthrite goutteuse, la spondylite,
vii) la maladie de Behçet,
viii) la sepsie, le choc septique, le choc endotoxique, la sepsie Gram-négative, la sepsie Gram-positive et le syndrome de choc toxique,
ix) le syndrome de lésions multiples des organes, secondaire à une septicémie, un traumatisme ou une hémorragie,
x) les troubles ophtalmiques, de préférence choisis parmi la conjonctivite allergique, la conjonctivite vernale, l'uvéite et l'ophtalmopathie associée aux maladies thyroïdiennes,
xi) le granulome éosinophile,
xii) les troubles pulmonaires ou respiratoires, de préférence choisis parmi l'asthme, la bronchite chronique, la rhinite allergique, le SDRA, la maladie inflammatoire pulmonaire chronique, la bronchopneumopathie chronique obstructive, la silicose, la sarcoïdose pulmonaire, la pleurésie, l'alvéolite, la vasculite, l'emphysème, la pneumonie, la bronchectasie, et la toxicité pulmonaire de l'oxygène,
xiii) la fibrose, en particulier la mucoviscidose,
xiv) la formation de chéloïdes ou la formation d'un tissu cicatriciel,
xv) l'athérosclérose,
xvi) les maladies auto-immunes, de préférence choisies parmi le lupus érythémateux disséminé (LED), la thyroïdite auto-immune, la sclérose en plaques, certaines formes de diabète, et le syndrome de Reynaud,
xvii) les troubles de rejet des organes transplantés, de préférence choisis parmi la réaction du greffon contre l'hôte et le rejet des allogreffes,
xviii) la glomérulonéphrite chronique,
xix) les maladies intestinales inflammatoires, de préférence choisies parmi la maladie inflammatoire chronique de l'intestin (MICI), la maladie de Crohn, la recto-colite hémorragique et l'entérocolite nécrosante,
xx) les dermatoses inflammatoires, de préférence choisies parmi la dermatite de contact, la dermatite atopique, le psoriasis et l'urticaire,
xxi) la fièvre et les myalgies dues à une infection,
xxii) les troubles inflammatoires du système nerveux central et périphériques, de préférence choisis parmi la méningite, l'encéphalite et les lésions du cerveau ou de la moelle épinière dues à un traumatisme mineur,
xxiii) la maladie de Sjögren,
xxiv) les maladies impliquant une diapédèse leucocytaire,
xxv) l'hépatite alcoolique,
xxvi) la pneumonie bactérienne,
xxvii) les maladies médiées par un complexe antigène-anticorps,
xxviii) le choc hypovolémique,
xxix) le diabète sucré de type I,
xxx) hypersensibilité aiguë et retardée,
xxxi) les états pathologiques dus à une dyscrasie leucocytaire et à une métastase,
xxxii) la lésion thermique,
xxxiii) les syndromes associés à une transfusion de granulocytes, et
xxxiv) la toxicité induite par des cytokines.

21. Composé ou composition selon la revendication 16 pour le traitement d'une maladie choisie dans le groupe consistant en la leucémie, les lymphomes et le myélome multiple.

22. Composé ou composition selon la revendication 16 pour le traitement d'une maladie choisie dans le groupe consistant en le lymphome de Burkitt, le lymphome de Hodgkin, le lymphome non hodgkinien, le lymphome lymphocytique, le myélome multiple, la leucémie lymphocytique et la leucémie myéloïde (myélogène) chronique.

23. Composé ou composition selon la revendication 16 pour le traitement d'une maladie choisie dans le groupe consistant en la BPCO, l'asthme, la polyarthrite rhumatoïde, le diabète, la rhinite allergique et la sclérose en plaques.
